(19) Europäisches Patentamt
European Patent Office
Office européen des brevets

(11) Publication number: **0 067 105**
**B1**

(12) # EUROPEAN PATENT SPECIFICATION

(45) Date of publication of patent specification: **23.09.87**

(21) Application number: **82400991.4**

(22) Date of filing: **27.05.82**

(51) Int. Cl.$^4$: **C 07 C 93/14**, C 07 C 91/28, C 07 C 87/29, A 61 K 31/135, C 07 C 33/48, C 07 C 39/38, C 07 C 39/373, C 07 C 43/23

(54) **Allyl amine MAO inhibitors.**

(30) Priority: **01.06.81 US 268555**

(43) Date of publication of application:
**15.12.82 Bulletin 82/50**

(45) Publication of the grant of the patent:
**23.09.87 Bulletin 87/39**

(84) Designated Contracting States:
**AT BE CH DE FR IT LI LU NL SE**

(56) References cited:
**EP-A-0 011 608**
**DE-A-2 343 038**

**JOURNAL OF THE AMERICAN CHEMICAL SOCIETY, vol. 101, no. 11, 23 May 1979 S. MITRA et al. "Reagents for the cross-linking of proteins by equilibrium transfer alkylation" Pages 3097-3110**

**The file contains technical information submitted after the application was filed and not included in this specification**

(73) Proprietor: **MERRELL DOW FRANCE ET CIE**
**16 Rue d'Ankara**
**F-67084 Strasbourg (FR)**

(72) Inventor: **Bey, Philippe**
**8, Rue de Stockholm**
**F-67000 Strasbourg (FR)**

(74) Representative: **Sgarbi, Renato**
**Gruppo Lepetit S.p.A.**
**Patent & Trademark Department**
**34 Via Roberto Lepetit**
**I-21040 Gerenzano (Varese) (IT)**

(56) References cited:
**Chemical Abstracts vol. 83, no. 22, 1 December 1975 Columbus, Ohio, USA I.M. PRNOVA et al. "Preparation of alpha-chloromethylstyrene-based secondary and tertiary amines" page 1, column 2, abstract no. 179647e**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 067 105 B1**

**Description**

This invention relates to novel chemical compounds, to intermediates for their production, and to pharmaceutical compositions and methods of treatment employing said compounds.

The class of compounds known as monoamine oxidase inhibitors (MAO inhibitors) has been employed in psychiatry for over 20 years for the treatment of depression [See Goodman and Gilman, *The Pharmacological Basis of Therapeutics,* 6th Ed., McMillan Publishing Co., Inc., N.Y., 1980, pages 427—430]. MAO Inhibitors currently used in the USA for treating depression are tranylcypromine (PARNATE SKF), phenelzine (NARDIL, Parke-Davis), and isocarboxazid (MARPLAN, Roche). In addition, another MAO inhibitor, pargyline (EUTRON, Abbott), is available for the treatment of hypertension [See *Physicians' Desk Reference,* 34th Ed., Medical Economics Co., Oradell, N.J., 1980, pages 1327—1328 (phenelzine), pages 1466—1468 (isocarboxazid), pages 1628—1630 (tranylcypromine), and pages 521—522 (pargyline)]. In addition for being used in treating depression, MAO inhibitors can be employed to treat other psychiatric disorders, such as phobic anxiety states.

It is believed that the MAO inhibitors act to alleviate psychiatric disorders, such as depression, by increasing the concentration of one or more biogenic monoamines in the brain or sympathetic nervous system. The monoamine oxidase enzyme (MAO) plays an important role in the metabolic regulation of the monoamines since it catalyzes the biodegradation of the monoamines through oxidative deamination. By inhibiting MAO, the degradation of the monoamines is blocked, and the result is an increase in the availability of the monoamines for their physiological functions. Among the physiologically active monoamines which are known substrates for MAO are: (a) the so-called "neurotransmitter" monoamines, such as the catecholamines (e.g. dopamine, epinephrine, and norepinephrine) and the indoleamines (e.g. tryptamine and 5-hydroxytryptamine), (b) the so-called "trace" amines (e.g. *o*-tyramine, phenethylamine, *tele-N*-methylhistamine), and (c) tyramine.

The usefulness of the MAO inhibitors in treating depression is limited because the administration of such agents can potentiate the pharmacological actions of certain food substances or drugs leading to dangerrous and sometimes lethal effects. For example, persons receiving a MAO inhibitor must avoid the ingestion of foods which have a high tyramine content (such as cheese) because the MAO inhibitor will block the metabolic degradation of tyramine in the gut to produce high circulating levels of tyramine, consequent release of catecholamines in the periphery, and finally serious hypertension. The potentiation by a MAO inhibitor of the pressor effect of tyramine arising from the ingestion of cheese, and the hypertensive episode produced thereby, are commonly known as the "cheese reaction" or "cheese effect". Moreover, persons on conventional MAO therapy can not be given directly-acting sympathomimetic drugs (or precursors thereof) which are themselves substrates for MAO (e.g. dopamine, epinephrine, norepinephrine, or L—DOPA) and of indirectly-acting sympathomimetic drugs (e.g. amphetamines or cold, hay-fever, or weight control preparations that contain a vasoconstrictor). The potentiation of the pressor effect of indirectly-acting sympathomimetic drugs is especially profound. This is because such drugs act peripherally primarily by releasing catecholamines in nerve endings, and the concentration of the liberated catecholamines will be dangerously elevated if the metabolic degradation of the catecholamines via MAO is blocked. In addition, a MAO inhibitor should not be used in combination with another MAO inhibitor or with hypotensive agents, dibenzazepine antidepressants, meperidine, CNS depressants, and anticholinergic agents.

Biochemical and pharmacological studies indicate that the MAO enzyme exists in two forms known as "MAO Type A" (MAO—A) and "MAO type B" (MAO—B). The two forms differ in their distribution in body organs, in their substrate specificity, and in their sensitivity to inhibitors. In general, MAO—A selectivily oxidizes the so-called "neurotransmitter" monoamines (epinephrine, norepinephrine, and 5-hydroxy-tryptamine) while MAO—B selectively oxides the "trace" monoamines (*o*-tyramine, phenethylamine, and *tele-N*-methylhistamine). Both MAO—A and MAO—B oxidize tyramine, tryptamine, and dopamine. However, in man, dopamine has been shown to be a preferred substrate for MAO—B. The forms also differ in their sensitivity to inhibition, and thus they can be preferentially inhibited depending upon the chemical structure of the inhibitor and/or the relative concentrations of the inhibitor and the enzyme. The MAO inhibitors currently sold in the USA for the therapy of depression (tranylcypromine, phenelzine, and isocarboxazid) are not preferential in their action upon MAO. However, various chemical compounds are known in the art to be preferential inhibitors of MAO, the most important being clorgyline, pargyline, and L-deprenyl which are all reported to be clinically effective antidepressant agents. MAO—A is preferentially inhibited by clorgyline, while MAO—B is preferentially inhibited by pargyline and L-deprenyl. It should be observed that the "selectivity" of a MAO inhibitor arises because the inhibitor has a greater affinity for one form of the enzyme. thus, the selectivity of an inhibitor for MAO—A or MAO—B *in vivo* will be dose-dependent, selectivity being lost as the dosage is increased. Clorgyline, pargyline, and L-deprenyl are selective inhibitors at lower dosages, but are not selective inhibitors at higher dosages. The literature concerning MAO—A and MAO—B and the selective inhibition thereof is extensive [See, for example, Goodman and Gilman, *ibid,* pages 204—205; Neff *et al., Life Sciences, 14,* 2061 (1974); Murphy, *Biochemical Pharmacology, 27,* 1889 (1978); Knoll, Chapter 10, pages 151—171 and Sandler, Chapter 11, pages 173—181, in *Enzyme Inhibitors as Drugs,* M. Sandler, Ed., McMillan Press Ltd., London, 1980; Lipper *et al., Psychopharmacology, 62,* 123 (1979); Mann *et al., Life Sciences, 26,* 877 (1980); and various articles in

*Monoamines Oxidase: Structure Function, and Altered Functions,* T. Singer *et al.* Ed., Academic Press, N.Y., 1979/.

Of the selective inhibitors of MAO, L-deprenyl is of interest since the "cheese effect" is not observed at the low dosages where preferential inhibition of MAO—B occurs [See Knoll, *TINS,* pages 111—113, May 1979]. This observation is not unexpected since the intestinal mucosa contains predominantly MAO—A which, because it is not inhibited, permits oxidation and removal of the ingested tyramine. The selectivity of L-deprenyl for MAO—B may account for its ability to potentiate L—DOPA for the treatment of Parkinson's disease without producing peripheral side effects, such as hypertension due to potentiation of pressor catecholamines [See Lees *et al., Lancet,* Page 791—795, October 15, 1977 and Birkmeyer, *lancet,* pages 439—443, February 26, 1977]. α-(N-benzylamino)-methyl-4-nitrostyrene is described by Mitra et al. in J. Ann, Chem. Soc., *101,* 3097, (1979) in an article entitled "Reagents for cross-linking of proteins by equilibrium transfer alkylation".

In its first composition of matter aspect, this invention comprehends pharmacologically active compounds of the formula:

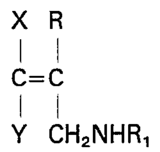 I or 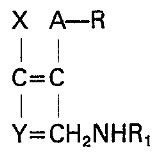 II

wherein:

R is 3,4-methylenedioxyphenyl; phenyl; phenyl monosubstituted, disubstituted, or trisubstituted by $(C_1—C_8)$alkyl, $(C_1—C_8)$alkoxy, $(C_1—C_6)$alkylcarbonyloxy, hydroxy, chlorine, bromine, iodine, fluorine, trifluoromethyl, $(C_1—C_6)$alkylcarbonyl, benzoyl, or phenyl; 1- or 2-naphthyl; 1-, 2-, or 3-indenyl; 1-, 2-, or 9-fluorenyl; 2-pyridinyl; 1-, 2-, or 3-piperidinyl; 2- or 3-pyrrolyl; 2- or 3-thienyl; 2- or 3-furanyl; 2- or 3-indolyl; 2- or 3-thianaphthenyl; or 2- or 3-benzofuranyl;

$R_1$ is hydrogen, $(C_1—C_8)$alkyl, benzyl, or phenethyl;

X and Y, independently, are hydrogen, fluorine, chlorine, or bromine; and

A is a divalent radical of the formula:

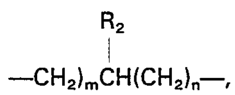

wherein $R_2$ is hydrogen, methyl, or ethyl, and m and n, independently, are an integer from 0 to 4, provided that m + n cannot be greater than 4;

$—CH_{2p}—D—(CH_2)_q—$, wherein D is oxygen or sulfur, p is an integer from 2 to 4, and q is an integer from 0 to 2 provided that p + q cannot be greater than 4; or

$—(CH_2)_rCH{=}CH(CH_2)_s—$, wherein r is an integer from 1 to 3 and s is an integer from 0 to 2, provided that r + s cannot be greater than 3;

or a non-toxic, pharmaceutically acceptable acid addition salt thereof; provided that when each of X and Y in Formula I is hydrogen, R cannot be phenyl.

The compounds of Formula I and II are pharmacologically active, being capable if inhibiting MAO *in vitro* and *in vivo.* The compounds of Formula I and II are useful for the treatment of psychiatric disorders, in particular depression, which are known to be responsive to MAO inhibitor therapy. For the treatment of depression, the compounds can be employed in a maner similar to that of the known clinically active MAO inhibitors, such as phenelzine and tranylcypromine.

Certain compounds of Formula I or II are capable of preferentially inhibiting the B form of MAO *in vitro* and, at suitable low dosages *in vivo,* such compounds will inhibit MAO—B without substantially inhibiting MAO—A. At dosage levels where such compounds exert a selective effect on MAO—B, the compounds will not produce a marked "cheese effect". Hence, as with L-deprenyl, a known selective inhibitor of MAO—B, such compounds can be employed at suitable dosages for the treatment of depression, or for the potentiation of L-DOPA in the treatment of Parkinsonism, with a significantly decreased risk of producing side effects, such as the "cheese effect". The preferred compounds of Formula I or II showing selective inhibition of MAO—B are (E)-2-(4'-methoxyphenyl)-3-fluoroallyl-amine and (E)-2-(3',4'-dimethoxyphenyl)-3-fluoro-allylamine. Both compounds, therefore, are the most preferred embodiments of Formula I or II.

In its second composition of matter aspect, the present invention comprehends chemical compounds of the Formula:

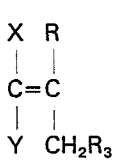 III or 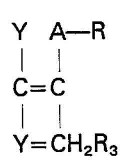 IV

wherein X, Y, R, and A have the meanings defined *supra* with respect to Formula I or II; except that R cannot be mono-, di-, or tri-hydroxyphenyl; and when Y is fluorine, chlorine, or bromine, X cannot be hydrogen; and $R_3$ is hydroxy or a leaving group. The compounds of Formula III or IV are intermediates for the preparation of the pharmacologically active compounds of Formula I and II, respectively. Preferred examples of leaving groups as defined by $R_3$ are: chlorine, bromine, tosyloxy, or mesyloxy. Other suitable leaving groups will be apparent to those skilled in the art of chemistry.

As employed herein, the term "alkyl" contemplates both straight- and branched-chain alkyl groups. Straight-chain alkyl groups are preferred. Illustrative examples of ($C_1$—$C_8$)alkyl groups are methyl, ethyl, *n*-propyl, *iso*-propyl, *n*-butyl, *iso*-butyl, *tert*-butyl, *n*-pentyl, *n*-hexyl, *n*-heptyl, and *n*-octyl. Methyl and ethyl are most preferred. The term "alkoxy" contemplates both straight- and branched-chain alkoxy groups. Straight-chain alkoxy groups are preferred. Illustrative examples of ($C_1$—$C_8$)alkoxy groups are methoxy, ethoxy, *n*-propoxy, *iso*-propoxy, *n*-butoxy, *iso*-butoxy, *tert*-butoxy, *n*-pentyloxy, *n*-hexyloxy, *n*-heptyloxy, and *n*-octyloxy. Methoxy and ethoxy are most preferred. The term "alkylcarbonyloxy" contemplates both straight- and branched alkylcarbonyloxy groups. Straight-chain groups are preferred. Illustrative ($C_1$—$C_6$)alkylcarbonyloxy groups are acetoxy, propionyloxy, *n*-butyroyloxy. Acetoxy is most preferred. The term "alkylcarbonyl" contemplates both straight- and branched-chain alkylcarbonyl groups. Straight-chain alkylcarbonyl groups are preferred. Illustrative ($C_1$—$C_6$)alkylcarbonyl groups are acetyl, propionyl, and *n*-butyryl. Acetyl is most preferred. The term "mono-substituted" as used herein in the definition of R in Formula I or II means that the phenyl ring is substituted by one substituent group which can be located at any of the available positions in the ring (i.e. in the *ortho, para,* or *meta* positions). The term "disubstituted" means that the phenyl ring is substituted by two substituents groups which may be located at any of the available positions in the ring or oriented in any manner with respect to each other. The term "trisubstituted" means that the phenyl ring is substituted by three substituents groups which may be located at any of the available positions in the ring or oriented in any manner with respect to ech other. When R in Formula I or II represents a di- or tri-substituted phenyl group, the groups substituted on the phenyl ring may be the same or they may be different.

Illustrative examples of divalent groups represented by A are $—CH_2—$, $—(CH_2)_2—$, $—(CH_2)_3—$, $—(CH_2)_4—$, $—(CH_2)_5—$, $—CH_2S(CH_2)_2—$, $—CH_2O(CH_2)_2—$, and $—CH{=}CH—CH_2—$. Methylene is preferred.

It will be apparent to those skilled in the art that, because the compounds of Formula I, II, III, or IV contain a double bond, depending on the meanings given to X and Y, geometric isomerism is possible. It should be understood, therefore, that, in Formula I or II, a group represented by Y is oriented in the position *trans* to the group represented by —R or —AR, while a group represented by X is oriented in the position *cis* to a group represented by —R or —AR. Thus, when X is fluorine, bromine, or chlorine, and Y is hydrogen, Formula I or II depicts compounds wherein the halogen is oriented in the *cis* position with respect to the group represented by —R or —AR, and, when Y is fluorine, bromine, or iodine, and X is hydrogen, Formula I or II depicts compounds wherein the halogen is oriented in the *trans* position with respect to the groups represented by —R or —AR. The compounds in which the halogen is oriented in the *cis* position with respect to —R or —AR are preferred. In naming the compounds of Formula I, II, III, or IV herein, the prefixes "(E)" and "(Z)" are used in the conventional manner to indicate the stereochemistry at the double bond. If no stereochemical designation is given, both the substantially pure isomers, or mixtures thereof, are meant. The compounds of Formula I or II can be isolated either in the form of the free base or in the form of a non-toxic pharmaceutically acceptable acid addition salt. Non-toxic pharmaceutically acceptable acids suitable for preparing the acid addition salts are known in the art. The free bases can be converted to the acid addition salts, or the acid addition salts can be converted to the free bases, by conventional chemical methods.

Preferred compounds of Formula I or II are those wherein Y is hydrogen and X is fluorine; $R_1$ is hydrogen, methyl, or ethyl; and R is phenyl, 1-naphthyl, 2-naphthyl, or phenyl mono-, di-, or tri-substituted by ($C_1$—$C_8$)alkyl, ($C_1$—$C_8$)alkylcarbonyloxy, ($C_1$—$C_6$)alkoxy, hydroxy, chlorine, bromine, iodine, fluorine, trifluoromethyl, ($C_1$—$C_6$)alkylcarbonyl, benzoyl, or phenyl. The most preferred compounds of Formula I are those wherein Y is hydrogen; X is fluorine; $R_1$ is hydrogen; and R is phenyl, ($C_1$—$C_8$)alkoxyphenyl, e.g. methoxyphenyl and ethoxyphenyl, di($C_1$—$C_8$)alkoxyphenyl, e.g. dimethoxyphenyl and diethoxyphenyl, methylphenyl, hydroxyphenyl, dihydroxyphenyl, chlorophenyl, trifluoromethylphenyl, 1-naphthyl, or 2-naphthyl. The most preferred compounds of Formula II are those wherein A is a divalent alkylene radical of the formula $—(CH_2)_t—$, wherein t is an integer from 1 to 5, e.g. methylene.

In its subgeneric aspects, the invention contemplates the following subclasses of compounds:

(i) A compound of Formula I wherein X is fluorine, Y is hydrogen, and $R_1$ is hydrogen.

(ii) A compound of Formula I wherein X is hydrogen, Y is fluorine, and $R_1$ is hydrogen.

(iii) A compound of Formula I wherein each of X and Y is fluorine, and $R_1$ is hydrogen.

(iv) A compound of Formula II wherein X is fluorine, Y is hydrogen, $R_1$ is hydrogen, and A is an alkylene radical of the formula $—(CH_2)_t—$ wherein t is an integer from 1 to 5.

(v) A compound of Formula II wherein X is hydrogen, Y is fluorine, $R_1$ is hydrogen, and A is an alkylene radical of the formula $—(CH_2)_t—$ wherein t is an integer from 1 to 5.

(vi) A compound of Formula II wherein each of X and Y is fluorine, $R_1$ is hydrogen, and A is an alkylene radical of the formula $—(CH_2)_t—$ wherein t is an integer from 1 to 5.

(vii) A compound as defined in paragraph (i), (ii), (iii), (iv), (v), or (vi) above wherein R is phenyl, 1-naphthyl, or 2-naphthyl.

(viii) A compound as defined in paragraph (i), (ii), (iii), (iv), (v), or (vi) above wherein R is phenyl mono-, di-, or tri-substituted by ($C_1$—$C_8$)alkyl, ($C_1$—$C_8$)alkoxy, ($C_1$—$C_6$)alkylcarbonyloxy, hydroxy, chlorine, bromine, iodine, fluorine, trifluoromethyl. ($C_1$—$C_6$)alkylcarbonyl, benzoyl, or phenyl.

(ix) A compound as defined in paragraph (i), (ii), (iii), (iv), (v), or (vi) above wherein R is phenyl monosubstituted by ($C_1$—$C_8$)alkoxy, ($C_1$—$C_8$)alkyl, hydroxy, chlorine, or trifluoromethylphenyl.

(x) A compound as defined in paragraph (i), (ii), (iii), (iv), (v), or (vi) above wherein R is phenyl di-substituted by ($C_1$—$C_8$)alkoxy, or hydroxy.

The most preferred compounds of Formula I or II, as defined in each of paragraphs (i) to (x), above, are those wherein $R_1$ is hydrogen, methyl, or ethyl and R is phenyl, ($C_1$—$C_8$)alkoxyphenyl, e.g. methoxyphenyl and ethoxyphenyl, di($C_1$—$C_8$)alkoxyphenyl, e.g. dimethoxyphenyl and diethoxyphenyl, methylphenyl, hydroxyphenyl, dihydroxyphenyl, chlorophenyl, trifluoromethylphenyl, 1-naphthyl, or 2-naphthyl. The most preferred compounds of Formula II, as defined in each of paragraphs (iv) to (ix), above, are those wherein A is methylene.

Illustrative examples of the compounds of Formula I are:

2-Phenyl-3-fluoroallylamine,
2-(2′-methoxy)phenyl-3-fluoroallylamine,
2-(3′-methoxy)phenyl-3-fluoroallylamine,
2-(4′-methoxy)phenyl-3-fluoroallylamine,
2-(2′,3′-dimethoxy)phenyl-3-fluoroallylamine,
2-(2′,4′-dimethoxy)phenyl-3-fluoroallylamine,
2-(2′,5′-dimethoxy)phenyl-3-fluoroallylamine,
2-(2′,6′-dimethoxy)phenyl-3-fluoroallylamine,
2-(3′,4′-dimethoxy)phenyl-3-fluoroallylamine,
2-(3′,5′-dimethoxy)phenyl-3-fluoroallylamine,
2-(3′,4′-methylenedioxy)phenyl-3-fluoroallylamine,
2-(2′-hydroxy)phenyl-3-fluoroallylamine,
2-(3′-hydroxy)phenyl-3-fluoroallylamine,
2-(4′-hydroxy)phenyl-3-fluoroallylamine,
2-(2′,3′-dihydroxy)phenyl-3-fluoroallylamine,
2-(2′,4′-dihydroxy)phenyl-3-fluoroallylamine,
2-(2′,5′-dihydroxy)phenyl-3-fluoroallylamine,
2-(2′,6′-dihydroxy)phenyl-3-fluoroallylamine,
2-(3′,4′-dihydroxy)phenyl-3-fluoroallylamine,
2-(3′,5′-dihydroxy)phenyl-3-fluoroallylamine,
2-(2′-methyl)phenyl-3-fluoroallylamine,
2-(3′-methyl)phenyl-3-fluoroallylamine,
2-(4′-methyl)phenyl-3-fluoroallylamine,
2-(2′-chloro)phenyl-3-fluoroallylamine,
2-(3′-chloro)phenyl-3-fluoroallylamine,
2-(4′-chloro)phenyl-3-fluoroallylamine,
2-(2′-trifluoromethyl)phenyl-3-fluoroallylamine,
2-(3′-trifluoromethyl)phenyl-3-fluoroallylamine,
2-(4′-trifluoromethyl)phenyl-3-fluoroallylamine,
2-benzyl-3-fluoroallylamine,
2-(2′-methoxy)benzyl-3-fluoroallylamine,
2-(3′-methoxy)benzyl-3-fluoroallylamine,
2-(4′-methoxy)benzyl-3-fluoroallylamine,
2-(2′,3′-dimethoxy)benzyl-3-fluoroallylamine,
2-(2′,4′-dimethoxy)benzyl-3-fluoroallylamine,
2-(2′,5′-dimethoxy)benzyl-3-fluoroallylamine,
2-(2′,6′-dimethoxy)benzyl-3-fluoroallylamine,
2-(3′,4′-dimethoxy)benzyl-3-fluoroallylamine,
2-(3′,5′-dimethoxy)benzyl-3-fluoroallylamine,
2-(2′-hydroxy)benzyl-3-fluoroallylamine,
2-(3′-hydroxy)benzyl-3-fluoroallylamine,
2-(4′-hydroxy)benzyl-3-fluoroallylamine,
2-(2′,3′-dihydroxy)benzyl-3-fluoroallylamine,
2-(2′,4′-dihydroxy)benzyl-3-fluoroallylamine,
2-(2′,5′-dihydroxy)benzyl-3-fluoroallylamine,
2-(2′,6′-dihydroxy)benzyl-3-fluoroallylamine,
2-(3′,4′-dihydroxy)benzyl-3-fluoroallylamine,
2-(3′,5′-dihydroxy)benzyl-3-fluoroallylamine,
2-(3′,4′-methylenedioxy)-3-fluoroallylamine,

2-(2'-methyl)benzyl-3-fluoroallylamine,
2-(3'-methyl)benzyl-3-fluoroallylamine,
2-(4'-methyl)benzyl-3-fluoroallylamine,
2-(2'-chloro)benzyl-3-fluoroallylamine,
2-(3'-chloro)benzyl-3-fluoroallylamine,
2-(4'-chloro)benzyl-3-fluoroallylamine,
2-(2'-trifluoromethyl)benzyl-3-fluoroallylamine,
2-(3'-trifluoromethyl)benzyl-3-fluoroallylamine,
2-(4'-trifluoromethyl)benzyl-3-fluoroallylamine,
2-(1-naphthyl)-3-fluoroallylamine,
2-(2-naphthyl)-3-fluoroallylamine.

Preferred compounds of Formula III or IV are those wherein X is fluorine and Y is hydrogen; each of X and Y is fluorine; R is phenyl or phenyl mono-, di, or tri-substituted by ($C_1$—$C_8$)alkyl, ($C_1$—$C_8$)alkoxy, ($C_1$—$C_6$)alkylcarbonyloxy, chlorine, bromine, iodine, fluorine, trifluoromethyl, ($C_1$—$C_6$)alkylcarbonyl, benzoyl, or phenyl. Most preferred compounds of Formula III or IV are those wherein R is phenyl, ($C_1$—$C_8$)alkoxyphenyl, e.g. methoxyphenyl or ethoxyphenyl, di($C_1$—$C_8$)alkoxyphenyl, e.g. dimethoxyphenyl or diethoxyphenyl, methylphenyl, chlorophenyl, trifluoromethylphenyl, 1-naphthyl, or 2-naphthyl. Most preferred compounds of Formula IV are those wherein A is methylene.

Illustrative embodiments of Formula III or IV are:

2-phenyl-3-fluoroallyl alcohol,
2-benzyl-3-fluoroallyl alcohol,
2-(2'-methoxy)phenyl-3-fluoroallyl alcohol,
2-(3'-methoxy)phenyl-3-fluoroallyl alcohol,
2-(4'-methoxy)phenyl-3-fluoroallyl alcohol,
2-(2',3'-dimethoxy)phenyl-3-fluoroallyl alcohol,
2-(2',4'-dimethoxy)phenyl-3-fluoroallyl alcohol,
2-(2',5'-dimethoxy)phenyl-3-fluoroallyl alcohol,
2-(2',6'-dimethoxy)phenyl-3-fluoroallyl alcohol,
2-(3',4'-dimethoxy)phenyl-3-fluoroallyl alcohol,
2-(3',5'-dimethoxy)phenyl-3-fluoroallyl alcohol,
2-(2'-methoxy)benzyl-3-fluoroallyl alcohol,
2-(3'-methoxy)benzyl-3-fluoroallyl alcohol,
2-(4'-methoxy)benzyl-3-fluoroallyl alcohol,
2-(2',3'-dimethoxy)benzyl-3-fluoroallyl alcohol,
2-(2',4'-dimethoxy)benzyl-3-fluoroallyl alcohol,
2-(2',5'-dimethoxy)benzyl-3-fluoroallyl alcohol,
2-(2',6'-dimethoxy)benzyl-3-fluoroallyl alcohol,
2-(3',4'-dimethoxy)benzyl-3-fluoroallyl alcohol,
2-(3',5'-dimethoxy)benzyl-3-fluoroallyl alcohol,
2-(1-naphthyl)-3-fluoroallyl alcohol,
2-(2-naphthyl)-3-fluoroallyl alcohol,
2-(2'-methyl)phenyl-3-fluoroallyl alcohol,
2-(3'-methyl)phenyl-3-fluoroallyl alcohol,
2-(4'-methyl)phenyl-3-fluoroallyl alcohol,
2-(2'-chloro)phenyl-3-fluoroallyl alcohol,
2-(3'-chloro)phenyl-3-fluoroallyl alcohol,
2-(4'-chloro)phenyl-3-fluoroallyl alcohol,
2-(2'-trifluoromethyl)phenyl-3-fluoroallyl alcohol,
2-(3'-trifluoromethyl)phenyl-3-fluoroallyl alcohol,
2-(4'-trifluoromethyl)phenyl-3-fluoroallyl alcohol,
2-(2'-methyl)benzyl-3-fluoroallyl alcohol,
2-(3'-methyl)benzyl-3-fluoroallyl alcohol,
2-(4'-methyl)benzyl-3-fluoroallyl alcohol,
2-(2'-chloro)benzyl-3-fluoroallyl alcohol,
2-(3'-chloro)benzyl-3-fluoroallyl alcohol,
2-(4'-chloro)benzyl-3-fluoroallyl alcohol,
2-(2'-trifluoromethyl)benzyl-3-fluoroallyl alcohol,
2-(3'-trifluoromethyl)benzyl-3-fluoroallyl alcohol,
2-(4'-trifluoromethyl)benzyl-3-fluoroallyl alcohol.

In its method of use aspect, the present invention provides a method for treating depression which comprises administering to a depressed patient an effective amount of a compound of the formula

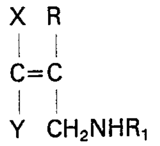 I or 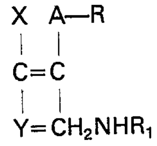 II

wherein:

R, $R_1$, X, Y and A have the meanings set forth hereinabove.

For pharmacological use, the compounds of Formula I or II may be administered in the form of an acid addition salt of a non-toxic organic or inorganic acid. Appropriate salts are those formed, for example, from the following acids: hydrochloric, hydrobromic, sulfonic, sulfuric, phosphoric, nitric, maleic, fumaric, benzoic, ascorbic, pamoic, succinic, methanesulfonic, acetic, propionic, tartaric, citric, lactic, malic, mandelic, cinnamic, palmitic, itaconic, and benzenesulfonic.

When employed to treat depression, the effective dosage of the compounds of Formula I or II will vary according to the particular compound being employed, the severity and nature of the depression and the particular subject being treated. In general, effective results can be achieved by administering a compound at a dosage level of from about 5 mg to about 100 mg per day, given systemically. Therapy should be initiated at lower dosages, the dosage thereafter being increased until the desired effect is obtained.

At the dosage levels set forth above, the compounds of Formula I or II, in general, will inhibit both forms of MAO. At lower dosage levels, certain compounds of Formula I or II may preferentially inhibit MAO—B and may have a decreased risk of producing the "cheese effect". For example, (E)-2-(4'-methoxy)-phenyl-3-fluoroallylamine or (E)-2-(3',4'-dimethoxy)phenyl-3-fluoroallylamine will selectively inhibit MAO—B at a systemic dosage range of about 0.1 mg to about 5 mg per day, and, at this dosage range, the risk of adverse reaction from the "cheese effect" will be substantially reduced or eliminated.

The compounds of this invention can be administered in various manners to achieve the desired effect. The compounds can be administered alone or in combination with pharmaceutically acceptable cariers or diluents, the proportion and nature of which are determined by the solubility and chemical properties of the compound selected, the chosen route of administration, and standard pharmaceutical practice. The compounds may be administered orally in solid dosage forms, e.g. capsules, tablets, powders, or in liquid forms, e.g. solutions or suspensions. The compounds may also be injected parenterally in the form of sterile solutions or suspensions. Solid oral forms may contain conventional excipients, for instance: lactose, succrose, magnesium stearate, resins, and like materials. Liquid oral forms may contain various flavoring, coloring, preserving, stabilizing, solubilizing, or suspending agents. Parenteral preparations are sterile aqueous or nonaqueous solutions or suspensions which may contain certain various preserving, stabilizing, buffering, solubilizing, or suspending agents. If desired, additives, such as saline or glucose may be added to make the solutions isotonic.

The amount of active compound administered will vary and can be any effective amount. Unit doses of these compounds can contain, for example, from about 5 mg to about 100 mg of the compounds and may be administered, for example, one or more times daily, as needed.

The term "unit dosage form" is used herein to mean a single or multiple dose form containing a quantity of the active ingredient in admixture with or otherwise in association with the diluent or carrier, said quantity being such that one or more predetermined units are normally required for a single therapeutic administration. In the case of multiple dose forms such as liquids or scored tablets, said predetermined unit will be one fraction such as 5 ml (teaspoon) quantity of a liquid or a half or quarter of a scored tablet, of the multiple dose form.

In the composition aspect of the invention, there are provided pharmaceutical formulations in which form the active compounds of the invention will normally be utilized. Such formulations are prepared in a manner well known *per se* in the pharmaceutical art and usually comprise at least one active compound of the invention in admixture or otherwise in association with a pharmaceutically acceptable carrier or diluent therefor. A carrier or diluent may be solid, semi-solid, or liquid material which serves as a vehicle, excipient, or medium for the active ingredient. Suitable diluents or carriers are well known *per se*. The pharmaceutical formulations may be adapted for enteral or parenteral use and may be administered to the patient in the form of tablets, capsules, suppositories, solutions, suspensions, or the like.

In the specific examples included herein-below, illustrative examples of suitable pharmaceutical formulations are described.

The manner and processes for preparing the compounds of Formula I or II will now be discussed with reference to the DRAWINGS. The compounds of Formula I and II wherein: (a) X and Y are each hydrogen; (b) X is fluorine, chlorine, or bromine and Y is hydrogen; and (c) X and Y, independently, are fluorine, chlorine, or bromine; can be prepared in general by the process steps depicted in *SCHEME I* of the DRAWINGS. In *SCHEME I*, the symbols $R_a$, $R_b$, $R_c$, $R_d$, $R_e$, B, Q, X, Y, W, and Z in the various formula have the following meanings:

$R_a$ is R— or R—A— wherein R and A have the meanings defined with respect to Formula I and II;

$R_b$ is *tert*-butyl, benzyl, diphenylmethyl, or triphenylmethyl;

$R_c$ is $(C_1—C_4)$straight-chain alkyl, *tert*-butyl, benzyl, diphenylmethyl, or triphenylmethyl;

$R_d$ is hydrogen or straight-chain $(C_1—C_4)$alkyl;

Z is a halomethyl group of the formula: $—CHFX_a$, $—CF_2X_a$, $—CH_2X_a$, $—CHClX_b$, $—CCl_2X_b$, $—CHBrX_c$, or $CBr_2X_c$, wherrein $X_a$ is fluorine, chlorine, bromine, or iodine; $X_b$ is chlorine, bromine, or iodine; and $X_c$ is bromine or iodine;

X and Y, independently, are hydrogen, fluorine, chlorine, or bromine, provided that when Y is fluorine, chlorine, or bromine, X cannot be hydrogen;

Q is chlorine, bromine, iodine, benzenesulfonyloxy, *p*-toluenesulfonyloxy (tosyloxy), methylsulfonyloxy (mesyloxy), or other leaving group;

B is the hexamethylenetetrammonium group, or the phthalimido, succinimido, maleimido group, or a group of the formula $—NHCO_2R_e$ wherein $R_e$ is $(C_1—C_4)$alkyl; or other group capable of generating a primary amino group; and

W is

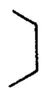 , 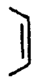 , or 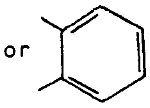

The process depicted in *SCHEME I* comprises the following steps:

(1) Halomethylating a malonic acid diester of Formula (1) to form the halomethyl diester of Formula (2) [Step A].

(2) Hydrolysing the halomethyl diester under acidic conditions or catalytically hydrogenating the diester to cleave one or both of the ester groups and then treating the intermediate so-produced with a base, whereby the intermediate undergoes decarboxylation and elimination of a halide ion to form the acrylic acid or acrylate ester of Formula (3) [Step B].

(3) Reducing the acrylic acid or acrylate ester to form the allyl alcohol of Formula (4) [Step C].

(4) Replacing the hydroxy group of the allyl alcohol of Formula (4) with a primary amino group to form the allyl primary amine of Formula (8), via formation of intermediates of Formula (5), Formula (6), or Formula (7) [Steps D—J].

The individual process steps shown in *SCHEME I*, and described in general above, can be carried out in a manner known *per se* in the art of chemistry. Examples of the methods that can be employed for carrying out the particular transformations depicted in *SCHEME I* are described as follows:

In *Step A*, a diester of Formula (1) is halomethylated in manner known *per se* by first treating the diester with a strong base to produce the corresponding carbanion and then contacting the carbanion with a suitable halomethylating agent. The strong base must be non-nucleophilic and be of sufficient strength to remove a proton from the carbon atom adjacent to the carboxy groups of the starting diester. Suitable such bases are known in the art. Examples are: (a) an alkyl lithium (e.g. *n*-butyllithium), (b) an aryl lithium (e.g. phenyllithium), (c) a lithium dialkylamide (e.g. lithium diisopropylamide), (d) sodium or lithium amide, (e) a metal hydride (e.g. sodium or potassium hydride), (f) metal alcoholate (e.g. sodium or potassium *tert*-butoxide), or (g) lithium or dilithium acetylide. The reaction between the diester and the base can be performed in an aprotic organic solvent [such as tetrahydrofuran (THF), diethyl ether, dimethylformamide (DMF), dimethyl sulfoxide (DMSO), dimethoxyethane, or dioxane, or mixtures thereof], using ambient temperature and a reaction time ranging from about 5 minutes to about 2 hours. Preferred bases for forming the carbanion are sodium hydride in dimethoxyethane, potassium *tert*-butoxide/*n*-butyllithium in THF, or sodium *tert*-butoxide in THF.

Suitable halomethylating agents are, for example, the polyhalomethanes of the formula:

$CH_2ClX_a$, $CHCl(X_a)_2$, $CCl_2(X_b)_2$, $CH_2BrX_b$, $CHBr(x_b)_2$, $CF(X_c)_3$, $CBr_2I_2$, $CClBr_3$, or $CH_2I_2$, wherein $X_a$, $X_b$ and $X_c$ are defined as above.

The selection of the particular polyhalomethane to be employed for the introduction of the halomethyl group desired in the compounds of Formula (2) will be apparent to those skilled in the art. Preferred polyhalomethanes are: $CHClF_2$ and $CHBrF_2$ for introducing the $—CHF_2$ group; $CHCl_2F$ for introducing the $—CHClF$ group; $—CHBrCl_2$ and $CHCl_3$ for introducing the $—CHCl_2$ group; $—CH_2BrCl$ for introducing the $—CH_2Cl$ group; $CHBr_2Cl$ for introducing the $—CHClBr$ group; $CHBr_3$ for introducing the $—CHBr_2$ group; and $CBr_2F_2$ for introducing the $—CBrF_2$ group.

The halomethylation of the carbanion can be carried out *in situ* by adding the appropriate polyhalomethane at a temperature range of about ambient to 60°C and allowing the reaction to proceed for about 15 minutes to 16 hours. Depending upon the reactivity of the reactants, the polyhalomethane can be introduced at a higher temperature (about 40°C), and the reaction mixture can be allowed to cool to room temperature to complete the reaction, or the polyhalomethane can be introduced at room temperature.

A preferred procedure for carrying out *Step A* employs reacting the diester with sodium *tert*-butoxide in THF at ambient temperature for about 30 minutes to form the carbanion, treating the carbanion *in situ* with the halomethylating agent at 45°C for about 5 minutes, and allowing the reaction mixture to stand for about 1 hour at room temperature.

*Step B* can be carried out in manner known *per se* in two stages. In the first stage, the halomethylmalonic acid diester of Formula (2) is cleaved by acid hydrolysis or by catalytic hydrogenation to convert either one or both of the ester groups ($—COOR_b$ or $—COOR_c$) to a free carboxylic acid group. Whether cleavage of one or both ester groups occurs will depend upon the nature of each ester group and the

conditions employed for the cleavage reaction. In order to effect cleavage of only one ester group, it is preferred that the diester be mixed, the groups defined by $R_b$ and $R_c$ being chosen so that the ester group —$COOR_b$ can be selectively cleaved without cleaving the ester group —$COOR_c$. The selection of particular ester groups which can be selectively cleaved and methods for performing the selective cleavage will be apparent to those skilled in the art. To accomplish selective cleavage of the diester, it is preferred to employ a halomethyl mixed diester of Formula (2) wherein $R_b$ is *tert*-butyl, benzyl, diphenylmethyl, or triphenylmethyl and $R_c$ is a straight-chain ($C_1$—$C_4$)alkyl group (such as methyl, ethyl, propyl, or *n*-butyl).

The ester group defined by —$COOR_b$ can be selectively hydrolyzed by treatment with an organic or inorganic acid, either with or without an added solvent, using a temperature range of about 0 to 25°C and a reaction time of about 1 to 10 hours. Ambient temperature is preferred. The choice of the acid for the hydrolysis is not critical, except that the acid should be chosen so that it can be easily removed after the hydrolysis stage. Trifluoroacetic acid is preferred since its low boiling point permits it to be easily removed from the hydrolysis product. When $R_b$ is benzyl, diphenylmethyl, or triphenylmethyl and $R_c$ is a straight-chain ($C_1$—$C_4$)alkyl group, the ester group —$COOR_b$ can also be selectively cleaved by subjecting the mixed diester of Formula (2) to catalytic hydrogenolysis using conventional procedures: for example, by treatment under a hydrogen atmosphere in the presence of a catalyst (e.g. Pd/C) at ambient temperature for 1 to 48 hours. As will be apparent to those skilled in the art, the ester groups can be chosen so that both groups can be cleaved simultaneously by acid hydrolysis or catalytic hydrogenolysis. Thus, when it is desired to cleave both ester groups simultaneously, each of $R_b$ and $R_c$ should be a *tert*-butyl, benzyl, diphenyl, or triphenylmethyl group. In the second stage of *Step B,* the acid obtained by cleavage of the diester (either a diacid or a mixed acid-ester) is treated with a base whereby the acid undergoes decarboxylation and elimination of halide ion to afford the acrylic acid or the acrylate ester of Formula (3). Whether the product is an ester [$R_c$ is a straight-chain ($C_1$—$C_4$)alkyl group] or an acid ($R_c$ is hydrogen) depends upon whether the cleavage reaction in the first stage was performed selectively or non-selectively. The reaction can be performed using an aqueous or non-aqueous solvent. Strong bases, such as sodium hydroxide and the like, or weak bases, such as triethylamine or sodium bicarbonate, can be used. However, with strong bases, care must be taken to avoid using an excess of base to avoid interaction with the double bond. Weak bases (which do not interact with the double bond) can be used in excess. The choice of a particular base, the reaction solvent, and reaction conditions will be apparent to those skilled in the art. A preferred procedure is to employ aquoeus sodium hydroxide in THF at ambient temperature. In general, a temperature range of about 0 to 25°C and reaction time of 15 minutes to 2 hours can be used.

In *Step C,* the acrylic acid or acrylate ester of Formula (3) is reduced in manner known *per se* to yield the allyl alcohol of Formula (4). The reducing agent employed for this transformation can be any reagent which is known in the art to be capable of selectively reducing an ester function or carboxylic acid function to the corresponding carbinol in the presence of a double bond. A preferred reducing agent is diisobutylaluminum hydride (DIBAL—H®) in hexane, THF, diethyl ether, or dichloromethane, or mixtures thereof. In a preferred procedure, a solution of the acrylate methyl ester in THF is cooled to about 0 to −78°C (preferably 0 to −10°C), the DIBAL—H dissolved in hexane is added, and the temperature of the mixture is allowed to rise to ambiance. The reaction time can be about 30 minutes to about 5 hours (preferably 1 hour). Acidic work-up of the product is desired.

The allyl alcohol of Formula (4) can be converted to the desired allyl primary amine in manner known *per se* to be useful for replacing an allylic hydroxyl group by an allylic primary amino group. A preferred method is shown by *Step D* and *Step E.* This involves the direct formation in manner known *per se* of an imido derivative of Formula (6), preferably the phthalimide and subsequent cleavage in manner known *per se* of the imido group to generate the primary amino group. In *Step D,* the imido derivative of Formula (6) can be prepared conveniently by treating the allyl alcohol of Formula (4) with the appropriate imide (i.e. phthalimide, succinimide, or maleimide) in the presence of a triarylphosphine (e.g. triphenylphosphine) or a trialkylphosphine and diethyl azodicarboxylate in an aprotic organic solvent (e.g. THF or dioxane). The reaction can be performed using a temperature range of about 0 to about 70°C and a reaction time of about 1 to 24 hours. Ambient temperature is preferred. In *Step E,* the imido derivative of Formula (6) can be cleaved, preferably by reaction with hydrazine in an organic solvent, such as an alkanol (e.g. ethanol), at reflux temperature (50 to 100°C) and a reaction time of about 30 minutes to 10 hours. It is preferable to add an acid (e.g. hydrochloric acid) after the hydrazine treatment to convert the product to the acid addition salt. Other reagents can be used to cleave the imido function. For example, the imide can be heated with a strong mineral acid (e.g. hydrochloric or sulfuric acid) or a mixture of hydrochloric acid and acetic acid. Acids, such as hydrobromic acid, which are reactive towards olefins usually cannot be used. The final products of Formula (8) can be conveniently purified and isolated as the acid addition salt using conventional purification methods.

The allyl alcohol of Formula (4) can also be converted in manner known *per se* to the allyl primary amine via formation (*Step F*) of the reactive intermediate of Formula (5), in which the —OH group is replaced by a leaving group (Q). Suitable leaving groups are known in the art. For example, chlorine, bromine, iodine, tosyloxy, or mesyloxy can be employed. Methods for replacing the hydroxy group by the leaving group are known *per se.* For example, the allyl alcohol of Formula (4) can be treated with a phosphorus trihalide (e.g. $PCl_3$ or $PBr_3$) in an organic solvent, such as toluene or benzene, to introduce halogen (e.g. chlorine or bromine). A preferred procedure employs phosphorus tribromide in toluene at a

temperature ranging from about 0 to about 25°C (preferably 5 to 10°C) and a reaction time ranging from about 30 minutes to about 5 hours (preferably 1 to 5 hours). The allyl alcohol can also be treated with a tosyl halide or mesyl halide (e.g. tosyl chloride or mesyl chloride) in the presence of a base (e.g. pyridine) to introduce the tosyloxy or mesyloxy group. The reactive intermediate of Formula (5) can be converted to the allyl primary amine of Formula (8) in manner known *per se* by displacement of the leaving group (Q) either in *Step J* directly by ammonia or in *Step G* by a nucleophilic group (B) which can then be cleaved (*Step H*) to generate the primary amino group. Examples of groups defined by B in Formula (7) which can be used to generate a primary amino group are the hexamethylenetetrammonium group, an imido group (e.g. phthalimido, succinimido, or maleimido group) or an alkylcarboxyamino group of the formula: $—NHCO_2R_e$ wherein $R_e$ is $(C_1—C_4)$alkyl. The hexamethylenetetrammonium group can be introduced by treating the reactive intermediate of Formula (5) with hexamethylenetetramine in an organic solvent [e.g. a $(C_1—C_4)$alkanol or chloroform] using ambient temperature and a reaction time of about 30 minutes to 24 hours. The hexamethylenetetrammonium group can be cleaved to generate the primary amino group by treatment by heating with an aqueous strong acid (e.g. hydrochloric acid), preferably under reflux. Acids which are reactive to the double bond cannot be used. The imido group can be introduced by treating the reactive intermediate of Formula (5) with the appropriate alkali metal imide (e.g. sodium or potassium phthalimide, succinimide, or maleimide) in an organic solvent, such as THF, TMF, DMF, DMSO, or dioxane using a temperature range of about 40 to about 100°C (preferably about 60 to about 70°C) and a reaction time of about 2 to about 16 hours (preferably 3 hours). A preferred method employs potassium phthalimide in DMF at a temperature of about 65°C and a reaction time of about 4 hours. The imido group can be cleaved to generate the primary amino group using the methods described *supra* with respect to *Step E* of *SCHEME I.* The alkylcarboxyamino group $—NHCO_2R_e$ can be introduced by treating the reactive intermediate of Formula (5) with an alkali metal cyanate (e.g. sodium or potassium cyanate) and a $(C_1—C_4)$alkanol using a temperature range of about 70 to 150°C, preferably 100°C, and a reaction time of about 1 to 6 hours, preferably 2 hours. The alkylcarboxyamino group can be cleaved to generate the primary amino group by treatment with iodotrimethylsilane followed by hydrolysis. The reaction with iodotrimethylsilane is performed in an organic solvent (e.g. chloroform) using a temperature range of about 0 to about 100°C, preferably 50°C, and a reaction time of about 1 to 24 hours, preferably 1 to 2 hours.

It should be observed that, in *Step B* of the method depicted in *SCHEME I,* when Z is a dihalomethyl group, elimination of the halide ion gives the geometric isomer in which the remaining halogen located on the double bond is oriented *cis* to the group represented by R [i.e. the product is a compound of Formula (3) wherein X is fluorine, chlorine, or bromine and Y is hydrogen].

The compounds of Formula I or II wherein X and Y, independently, are hydrogen, chlorine, or bromine can be made by the process depicted in the DRAWINGS in *SCHEME II.* In *SCHEME II,* the symbols $R_a$, W, and X have the following meanings:

$R_a$ is the group R— or RA— wherein R— and RA— have the meanings set forth with respect to Formula I and Formula II, respectively,

W is

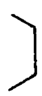 ,  , or 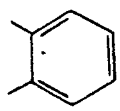

and

X is chlorine or bromine.

In *Step K,* an allyl imido derivative of Formula (9) is chlorinated or brominated, using procedures known *per se* to be useful for adding chlorine or bromine to a double bond. The product formed is the dihalo imido derivative of Formula (10). The bromination or chlorination reaction can be performed by treating the allyl imido derivative with chlorine or bromine in a suitable solvent, such as carbon tetrachloride, chloroform, or methylene chloride, in the absence of light using a temperature range of about −10°C to ambient temperature, preferably 0 to 5°C, and a reaction time of about 1 to 6 hours, preferably 3 hours. The olefinic amido compounds of Formula (11a) and (11b) are prepared from the dihalo imido derivative by treatment with a conventional dehydrohalogenating agent. A preferred dehydrohalogenating agent is 1,5-diazabicyclo[5.4.0]undec-5-ene (DBU). The reaction is carried out in DMSO using a temperature range of from ambiance to 120°C, preferably 85—95°C, and a reaction time of about 4 to 24 hours. The product of the dehydrohalogenation reaction is a mixture of the monochloro or monobromo allyl imido derivatives shown in Formula (11a) and (11b), which compounds are isomeric and differ with respect to the orientation of the halogen atom of the double bond. The isomers can be separated by conventional methods, such as column chromatography. The individual imido derivatives of Formula (11a) and (11b), or a mixture thereof, can be cleaved in *Step M* in known manner using the methods described *supra* with respect to *Step E* in *SCHEME I* to yield the monohalo allyl primary amines shown in Formula (12a) or Formula (12b). The dihalo allyl primary amines of Formula (15) can be prepared in *Steps N, O, and P* by chlorinating or brominating the double bond of a monohalo allyl imido derivative of Formula (11a) or (11b), or a mixture thereof, to form the trihaloallyl imido derivative of Formula (13), and treating the trihalo derivative with a dehydrohalogenating reagent, and cleaving the imido function. Methods for chlorinating

or brominating the double bond (*Step N*) and for the dehydrohalogenation reaction (*Step O*) are described *supra* with respect to *Steps K and L*, respectively. In *Step P*, the imido moiety of the dihalo allyl imido derivative of Formula (14) is cleaved to give the dihalo allyl primary amine of Formula (15) using known methods as described *supra* with respect to *Step E* of *SCHEME I*.

The compounds of Formula I wherein (a) X is fluorine and Y is chlorine or bromine, (b) Y is fluorine and X is chlorine or bromine, can also be prepared from a compound of the Formula (11a) or (11b) wherein $X_d$ is fluorine, via *Steps N, O, and P* using methods described *supra*. Chlorination or bromination as described in *Step N* followed by dehydrohalogenation as described in *Step O* will provide the diahlo allyl imide derivative of the formula:

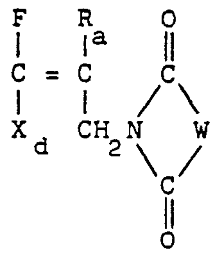
or
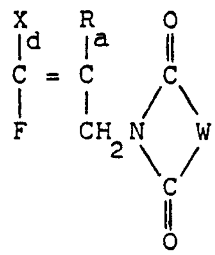

wherein $R_a$ is the group R— or RA— wherein R— and RA— have the meanings set forth with respect to Formula I and II, respectively, $X_d$ is chlorine or bromine, and W is

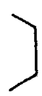 , 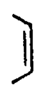 , or 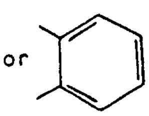

The imido groups can be cleaved by methods hereinbefore discussed with respect to *Steps E, M, and P*.

The compounds of Formula I or II wherein X is hydrogen and Y is fluorine, chlorine, or bromine can be prepared from an appropriate imido derivative of Formula (6) in *SCHEME I*, wherein Y is fluorine, chlorine, or bromine and X is hydrogen, by a process which includes halogenation of the double bond, dehalogenation to re-introduce the double bond, and cleavage of the imido function to generate the primary amine. For example, (E)-1-fluoro-2-phenyl-3-phthalimidopropene is brominated in methylene chloride in the absence of light, the 1,2-dibromo product is then debrominated using potassium iodide in acetone, and finally the phthalimido group is cleaved using hydrazine in ethanol. The major product is (*Z*)-2-phenyl-3-fluoroallylamine.

In the procedures of *SCHEME I and II*, the product is the primary allyl amine. The secondary allyl amines of Formula I or II can be made by conventional *N*-alkylation methods. For example, the *N*-ethyl derivatives can be made by treating the primary amine with benzaldehyde in a lower alcohol (e.g. ethanol) to form the Schiff base, treating the Schiff base with triethyloxonium tetrafluoroborate, and hydrolyzing the intermediate thus formed.

The compounds of Formula I or II wherein R is a mono-, di-, or tri-hydroxyphenyl group can be prepared using as the starting compound in *SCHEME I or II* a compound wherein $R_a$ is R, wherein R is a mono-, di-, or tri-alkoxy phenyl group. The processes depicted in *SCHEME I* or *SCHEME II* can be carried out unchanged up to the preparation of the allyl imido derivative of Formula (6), (11a and b), or (14). At this point, the aromatic alkoxy group is cleaved to the hydroxy group in manner known *per se*. A preferred procedure employs boron tribromide in dichloromethane at a temperature ranging from about −78°C to ambient temperature (preferably −78°C to 0°C) and a reaction time of about 10 minutes to about 1 hour (preferably 30 minutes). The imide function is then cleaved, as discussed *supra*, to generate the primary amino group. The product is conveniently isolated via the *N-tert*-butyloxycarbonyl derivative.

When it is desired to prepare a compound of Formula I or II wherein R is a mono-, di-, or tri-($C_1$—$C_6$)-alkylcarbonyloxy group, the corresponding compound of Formula I or II can be acylated in manner known *per se*, provided that the free primary amino group is protected in known manner, preferably with a t-butyloxycarbonyl (BOC) or benzyloxycarbonyl group. Methods for protecting and removing the primary amino groups are known in the art. The acylation reaction can be carried out using, for example, an acid halide or anhydride in an inert solvent.

The malonic acid diester of Formula (1) used as the starting compounds in the process depicted in *SCHEME I* are either known compounds or they can be prepared from known compounds using known methods or obvious modifications thereof. In particular, the diesters of Formula (1) can be made by acylating an appropriate carboxylic acid ester of Formula (16a) or (16b), shown below:

$$R_aCH_2CO_2R_b \qquad (16a)$$

$$R_aCH_2CO_2R_c \qquad (16b)$$

In Formula (16a) or (16b), $R_a$ is R— or RA— wherein R and A have the meanings defined with respect to Formula I and II; $R_b$ is *tert*-butyl, benzyl, diphenylmethyl, or triphenyl and $R_c$ is ($C_1$—$C_4$)-(straight-chain)-alkyl, *tert*-butyl, benzyl, diphenylmethyl, or triphenylmethyl. Methods of acylating the ester of Formula (16a) or (16b) are known in the art. One method is to treat the ester with a non-nucleophilic strong base to produce the carbanion, and then to treat the carbanion with a suitable acylating agent. Suitable strong bases are known in the art, and are discussed with respect to *Step A* of *SCHEME I.* A preferred base is lithium diisopropylamide. Any conventional acylating agent can be employed. A preferred acylating agent is a reactive halide of a formic acid alkyl ester, as shown in Formula (17a) or (17b):

$$\text{Hal-}CO_2R_b \qquad (17a)$$

$$\text{Hal-}CO_2R_c \qquad (17b)$$

wherein $R_b$ and $R_c$ are as defined *supra* with respect to Formula (16a) or (16b) and Hal is chlorine or bromine. In a preferred acylation procedure, an ester of Formula (16a) or (16b) is treated with a base (e.g. lithium diisopropylamide) in an organic solvent (e.g. THF, diethyl ether, acetonitrile, DMF, DMSO, or dioxane) at a low temperature (e.g. about −30 to about −78°C, preferably −65 to −78°C). The reaction can be allowed to proceed for a period of from 5 minutes to 2 hours, preferably about 1 hour. The acylation reaction can be performed by adding the haloformate ester to the cooled reaction mixture containing the carbanion and allowing the mixture to warm to room temperature. The acylation is allowed to continue for a period of about 4 to 24 hours, preferably 16 hours.

The diesters of Formula (1) in *SCHEME I* wherein $R_a$ is RA—, as defined with respect to Formula II *supra,* can be made by an alternative method. In this method, a malonic acid diester of Formula (18):

$$R_bO_2C—CH_2—CO_2R_c; \qquad (18)$$

wherein $R_b$ and $R_c$ have the meanings given with respect to Formula (17a) and (17b), *supra,* is alkylated using an alkylating agent of Formula (19):

$$R—A—Q; \qquad (19)$$

wherein RA— has the meaning given with respect to Formula II, *supra,* and Q is a leaving group, such as chlorine, bromine, iodine, tosyloxy, or mesyloxy. The alkylation is performed in two stages, the first being treatment with a strong base to form the carbanion and the second being treatment of the carbanion with the alkylating agent. Methods for carrying out the malonic acid ester alkylation are discussed *supra* and are well known in the art.

In the compounds of Formula II and IV, the group "A" is defined as a divalent radical which is a "bridging" group inserted between the double bond and the group defined by "R". It will be apparent to those skilled in the art that the divalent radical defined by "A" can be either symmetrical or unsymmetrical depending upon the particular radical employed. When "A" is an unsymmetrical divalent radical, it will be understood that the divalent radical must be attached to the double bond by means of the terminal carbon shown on the *left* side of the divalent formula as written herein, and the divalent radical must be attached to the group defined by "R" by means of the terminal carbon shown on the *right* side of the divalent formula as written herein.

In process aspects, the invention contemplates the methods of preparation as described below:

(1) A method of preparing a compound of Formula I or Formula II which comprises treating a compound of the formula:

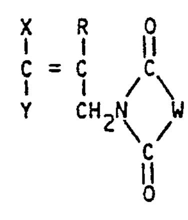

or

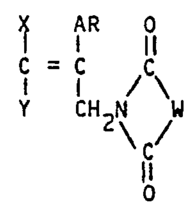

wherein X, Y, R, and A are as defined with respect to Formula I or Formula II and W is

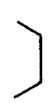 , 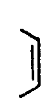 , or 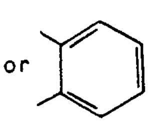

in manner known *per se* to convert the succinimido, maleimido, or phthalimido group to the primary amino group.

(2) A method as defined in (1) above wherein W is

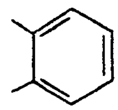

and the treatment comprises: reaction with hydrazine, or hydrolytic cleavage using a strong mineral acid.

(3) A method for preparing a compound of Formula I or Formula II which comprises treating an alcohol of the formula:

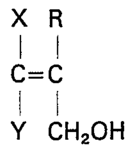

or

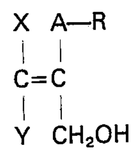

wherein X, Y, A, and R are as defined with respect to Formula I or Formula II, provided that when Y is fluorine, chlorine, or bromine, X cannot be hydrogen, in manner known *per se* to convert the hydroxy group into the primary amino group.

(4) A method as defined in (3) above wherein said alcohol is converted in manner known *per se* into a derivative of the formula:

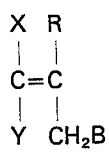

or

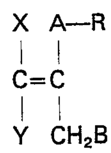

wherein X, Y, and R are as defined with respect to Formula I or Formula II, provided that when Y is fluorine, chlorine, or bromine, X cannot be hydrogen, and B is the succinimido, phthalimido, or maleimido group, the hexamethylenetetrammonium group, or a ($C_1$—$C_4$)alkylcarboxyamino group; and said derivative is subsequently treated in manner known *per se* to convert the succinimido, phthalimido, or maleimido group, the hexamethylenetetrammonium group, or a ($C_1$—$C_4$)alkylcarboxyamino group to a primary amino group.

(5) A method as defined in (4) above wherein:

(a) B is the succinimido, phthalimido, or maleimido group and the conversion of said group to amino comprises reaction with hydrazine or hydrolytic cleavage using a strong acid.

(b) B is the hexamethylenetetrammonium group and the conversion of said group to amino comprises treatment by heating with a strong acid; or

(c) B is a ($C_1$—$C_4$)alkylcarboxyamino group and the conversion of said group to amino comprises hydrolysis with a strong mineral acid.

(6) A method for preparing a compound of Formula III or Formula IV which comprises reducing in manner known *per se* a compound of the formula:

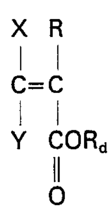

or

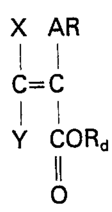

wherein X, Y, R, and A are as defined with respect to Formula II or Formula III and $R_d$ is hydrogen or ($C_1$—$C_4$)alkyl.

(7) A method as defined in (6) above wherein the reduction is carried out using diisobutylaluminum hydride.

The following examples (1 to 27) further illustrate the manner and processes for making the compounds of Formula I or II. In the Examples, all temperatures are in degree Centigrade.

Example 1

Ethyl 2-carbo-*tert*-butoxy(3',4'-dimethoxy)phenylacetate

A solution of lithium diisopropylamide in THF is prepared at 5°C by the addition of *n*-butyllithium (200 ml, 1.4 M) to diisopropylamide (41.2 ml) in THF (500 ml). The temperature is lowered to about −65°C and a solution of *tert*-butyl 3,4-dimethoxyphenylacetate (65 g) in THF (100 ml) is added. After 1 hour at this temperature, the reaction mixture is treated with a solution of ethyl chloroformate (33.02 g) in THF (100 ml). Cooling is removed and the solution is stirred overnight at room temperature. The solvent is evaporated

and the residue mixed with ether, is washed consecutively with N HCl, water, and brine. The ether solution is dried and the solvent removed by evaporation. The product (91.86 g, yellowish oil) is purified by chromatography on silica gel (1 kg) using as eluant 20% ether/80% light petroleum to give ethyl 2-carbo-*tert*-butoxy(3',4'-dimethoxy)phenylacetate (61.79 g):

NMR ($CCl_4$): δ 1.25 t (J=7Hz), 3H; 1.43, s, 9H; 3.75, 3.78, two s, 6H; 4.12, q (J=7Hz), 2H; 4.25, s, 1H; 6.72, s, 2H; 6.85, s, 1H.

Analysis for $C_{17}H_{24}O_6$:

| | | |
|---|---|---|
| Found: | C, 62.96; | H, 7.26% |
| Requires: | C, 62.95; | H, 7.46% |

## Example 2

Ethyl 2-carbo-*tert*-butoxy-(4'-methoxy)phenylacetate

A solution of lithium diisopropylamide in THF (500 ml) is prepared at 5° by the addition of *n*-butyllithium (427 ml of 1.6 M solution) to diisopropylamine (89.5 ml; 64.64 g) in THF (500 ml). The temperature is lowered to about −65° and a solution of *tert*-butyl 4-methoxyphenylacetate(70.47 g) in THF (100 ml) is added over about 5 minutes. After 1 hour at this temperature, the reaction mixture is treated with a solution of ethyl chloroformate (34.6 g) in THF (100 ml). Cooling is removed and the solution is stirred overnight at room temperature. 6 N HCl (53 ml) is added slowly so that the temperature does not rise above 20°. The THF is evaporated and the residue, dissolved in ether, is washed consecutively with water, 1 N HCl, and water (× 4). The ether solution is dried and the solvent is removed by evaporation to give ethyl 2-carbo-*tert*-butoxy(4'-methoxy)phenylacetate (93.76 g): orange oil, b.p. 124—125°/0.05 mm:

NMR ($CDCl_3$): δ 1.17, t (J=7Hz), 3H; 1.38, s, 9H; 3.67, s, 3H; 4.10, q (J=7Hz), 2H; 4.37, s, 1H; centred at 6.96, $A_2B_2$($J_{AB}$=9Hz), 4H.

## Example 3

Repeating the procedure of Example 1, but using the appropriate starting materials in place of *tert*-butyl 3,4-dimethoxyphenylacetate the following compounds are obtained:

(a) Ethyl 2-carbo-*tert*-butoxyphenylacetate: b.p. 90°/0.06 mm:

NMR ($CCl_4$): δ 1.22, t (J=7Hz), 3H; 1.38, s, 9H; 4.07, q (J=7Hz), 2H; 4.32, s, 1H; 7.22, s, 5H.

(b) Ethyl 2-carbo-*tert*-butoxy(3'-methoxy)phenylacetate: b.p. 132—133°/0.04 mm:

NMR ($CCl_4$): δ 1.20, t (J=7Hz), 3H; 1.37, s, 9H; 3.70, s, 3H; 4.07, q (J=Hz), 2H; 4.23, s, 1H; 6.52 to 7.20, m, 4H.

(c) Ethyl 2-carbo-*tert*-butoxyphenylpropionate: b.p. 95°/0.05 mm (oven):

NMR ($CDCl_3$): δ 1.20, t (J=7Hz), 3H; 1.40, s, 9H; 3.17 and 3.55, $AB_2$ system, 3H; 4.17, q (J=7Hz), 2H; 7.22, s, 5H.

## Example 4

Repeating the procedure of Example 2, but using the appropriate starting materials in place of *tert*-butyl 4-methoxyphenylacetate, the following compounds are obtained:

(a) Ethyl 2-carbo-*tert*-butoxy(2'-methoxy)phenylacetate:

NMR ($CCl_4$): δ 1.23, t (J=7Hz), 3H; 1.45, s, 9H; 3.77, s, 3H; 4.13, q (J=7Hz), 2H; 4.87, s, 1H; 6.67 to 7.43, m, 4H.

Analysis for $C_{16}H_{22}O_5$:

| | | |
|---|---|---|
| Found: | C, 65.04; | H, 7.26% |
| Requires: | C, 65.29; | H, 7.53% |

(b) Ethyl 2-carbo-*tert*-butoxy(4'-chloro)phenylacetate: m.p. 56—57°:

NMR (CDCl): δ 1.27, t (J=7Hz), 3H; 1.47, s, 9H; 4.19, q, (J=7Hz), 2H; 4.52, s, 1H; 7.35, s, 1H.

Analysis for $C_{15}H_{19}ClO_4$:

| | | |
|---|---|---|
| Found: | C, 60.32; | H, 6.28% |
| Requires: | C, 60.30; | H, 6.41% |

(c) Ethyl 2-carbo-*tert*-butoxy(3'-trifluoromethyl)phenylacetate:

NMR (CCl): δ 1.23, t (J=7Hz), 3H; 1.43, s, 9H; 4.13, q (J=7Hz), 2H; 4.43, s, 1H; 7.37 to 7.70, m; 4H.

Analysis for $C_{16}H_{19}F_3O_4$:

| | | |
|---|---|---|
| Found: | C, 57.97; | H 5.69% |
| Requires: | C, 57.83; | H, 5.76% |

(d) Ethyl 2-carbo-*tert*-butoxy(4'-methoxy)phenylpropionate:

NMR ($CDCl_3$): δ 1.20, t (J=7Hz), 3H; 1.38, s, 9H; 3.12 and 3.50, $AB_2$ system, 3H; 3.75, s, 3H; 4.15, q (J=7Hz), 2H; centred at 6.97, $A_2B_2$ system (J=9Hz), 4H.

Analysis for $C_{17}H_{24}O_5$:

| | | |
|---|---|---|
| Found: | C, 66.34; | H, 7.94% |
| Requires: | C, 66.21; | H, 7.84% |

Example 5

Ethyl 2-difluoromethyl-2-carbo-*tert*-butoxy(3',4'-dimethoxy)phenylacetate

A solution of ethyl 2-carbo-*tert*-butoxy(3',4'-dimethoxy)phenylacetate (9.72 g) in dimethoxyethane (DME, 80 ml) is added to sodium hydride (1.58 g as a 50—55% dispersion in oil which was previously washed free of oil with light petroleum). When anion formation is complete the reaction mixture is heated to about 40° and a stream of chlorodifluoromethane (Freon 22) is bubbled through the mixture for a few minutes. A balloon is attached to the reaction vessel and the Freon 22 is added until the balloon is full. The heating bath is then removed and the mixture is stirred for about 16 hours. The DME is partially evaporated and the residue is mixed with water and extracted with ether. The ether solution is washed with brine and dried ($MgSO_4$). Evaporation of the solvent gives crude ethyl 2-difluoromethyl-2-carbo-*tert*-butoxy(3',4'-dimethoxy)phenylacetate (9.93 g): pale-orange oil:

NMR ($CCl_4$): δ 1.25, t (J=7Hz), 3H; 1.42, s, 9H; 3.73, s, 6H; 4.20, q (J=7Hz), 2H; 6.25, t (J=56Hz), 1H; 6.68, s, 2H; 6.78, s (broad), 1H.

Example 6

Repeating the procedure of Example 5 but substituting the appropriate starting material in place of ethyl 2-carbo-*tert*-butoxy(3',4'-dimethoxy)phenylacetate, the following compounds are obtained:

(a) Ethyl 2-difluoromethyl-2-carbo-*tert*-butoxyphenylacetate:

NMR ($CCl_4$): δ 1.27, t (J=7Hz), 3H; 1.47, s, 9H; 4.18, q (J=7Hz), 2H; 6.30, t (J=55Hz), 1H; 7.30, s, 5H.

Analysis for $C_{16}H_{20}F_2O_4$:

| | | |
|---|---|---|
| Found: | C, 61.49; | H, 6.48% |
| Requires: | C, 61.14; | H, 6.41% |

(b) Ethyl 2-difluoromethyl-2-carbo-*tert*-butoxy(4'-methoxy)phenylacetate: b.p. 118—119°/0.05 mm:

NMR ($CDCl_3$): δ 1.23, t (J=7Hz), 3H; 1.42, s, 9H; 3.67, s, 3H; 4.20, q (J=7Hz), 2H; 6.30, t (J=57Hz), 1H; centred at 6.97, $A_2B_2$ ($J_{AB}$=9Hz), 4H.

(c) Ethyl 2-difluoromethyl-2-carbo-*tert*-butoxy(3'-methoxy)phenylacetate: b.p. 101—108°/0.05 mm:

NMR ($CDCl_3$): δ 1.16, t (J=7Hz), 3H; 1.37, s, 9H; 3.63, s, 3H; 4.05, q (J=7Hz), 2H; 6.38, t (J=54Hz), 1H; 6.63 to 7.28, m, 4H.

Analysis for $C_{17}H_{22}F_2O_5$:

| | | |
|---|---|---|
| Found: | C, 59.16; | H, 6.41% |
| Requires: | C, 59.29; | H, 6.44% |

(d) Ethyl 2-difluoromethyl-2-carbo-*tert*-butoxy(4'-chloro)phenylacetate:

NMR ($CCl_4$): δ 1.28, t (J=7Hz), 3H; 1.48, s, 9H; 4.27, q (J=7Hz), 2H; 6.38, t (J=55Hz), 1H; 7.28, s, 4H.

(e) Ethyl 2-difluoromethyl-2-carbo-*tert*-butoxyphenylpropionate:

NMR ($CDCl_3$): δ 1.25, t (J=7Hz), 3H; 1.43, s, 9H; 3.38, s, 2H; 4.20, q (J=7Hz), 2H; 6.03, t (J=55Hz), 1H; 7.23, s, 5H.

Analysis for $C_{17}H_{22}F_2O_4$:

| | | |
|---|---|---|
| Found: | C, 62.56; | H, 6.80% |
| Requires: | C, 62.18; | H, 6.75% |

Example 7

Repeating the procedure of Example 5 but substituting the appropriate starting materials in place of ethyl 2-carbo-*tert*-butoxy(3',4'-dimethoxy)phenylacetate, and potassium-*tert*-butoxide/*n*-butyllithium in THF in place of sodium hydride in DME, the following compounds are obtained:

(a) Ethyl 2-difluoromethyl-2-carbo-*tert*-butoxy(2'-methoxy)phenylacetate:
NMR ($CCl_4$): δ 1.25, t (J=7Hz), 3H; 1.47, s, 9H; 3.73, s, 3H; 4.22, q (J=7Hz), 2H; 6.53, t (J=56Hz), 1H; 6.67 to 7.50, m, 4H.

Analysis for $C_{17}H_{22}F_2O_5$:
Found: C, 59.24; H, 6.45%
Requires: C, 59.29; H, 6.44%

(b) Ethyl 2-difluoromethyl-2-carbo-*tert*-butoxy(3'-trifluoromethyl)phenylacetate:
NMR ($CCl_4$): δ 1.30, t (J=7Hz), 3H; 1.50, s, 9H; 4.35, q (J=7Hz), 2H; 4.87, t (J=55Hz), 1H; 7.60, m; 4H.

(c) Ethyl 2-difluoromethyl-2-carbo-*tert*-butoxy(4'-methoxy)phenylpropionate:
NMR ($CDCl_3$): δ 1.25, t (J=7Hz), 3H; 1.42, s, 9H; 3.33, s, 2H; 3.73, s, 3H; 4.18, q (J=7Hz), 2H; 6.00, t (J=54Hz), 1H; centre at 6.92, $A_2B_2$ ($J_{AB}$=9Hz), 4H.

Analysis for $C_{18}H_{24}F_2O_5$:
Found: C, 60.43; H, 5.71%
Requires: C, 60.32; H, 6.75%

## Example 8

Ethyl (*E*)-2-(3',4'-dimethoxy)phenyl-3-fluoroacrylate

A solution of ethyl 2-difluoromethyl-2-carbo-*tert*-butoxy(3',4'-dimethoxy)phenylacetate (61.70 g) in trifluoroacetic acid (152 ml) is stirred at room temperature for 1 hour whereupon the mixture is evaporated to dryness. The residue is dissolved in tetrahydrofuran (THF, 70 ml) and treated with aqueous sodium hydroxide (2M, 83 ml) at room temperature for 15 minutes. The reaction mixture is diluted with water and extracted with ether. The ether solution is washed with brine, dried ($MsSO_4$), and evaporated to yield an orange oil (44.05 g). Chromatography on silica gel (200 g) using 20% ethyl acetate in light petroleum as eluant affords an oil (39.70 g) which slowly crystallizes. Purification by recrystallization from *n*-pentane gives ethyl (*E*)-2-(3',4'-dimethoxy)phenyl-3-fluoroacrylate: colorless plates, m.p. 71—72°:
NMR ($CCl_4$): δ 1.27, t (J=7Hz), 3H, 3.75, s, 6H; 4.15, q (J=7Hz), 2H; 6.72, s, 3H; 7.53, d (J=42Hz), 1H.

Analysis for $C_{13}H_{15}FO_4$:
Found: C, 61.49; H, 5.94%
Requires: C, 61.41; H, 5.95%

## Example 9

Repeating the procedure of Example 8 but substituting the appropriate starting materials for 2-difluoromethyl-2-carbo-*tert*-butoxy(3',4'-dimethoxy)phenylacetate, the following compounds are obtained:

(a) Ethyl (*E*)-2-phenyl-3-fluoroacrylate: b.p. 81°/0.4 mm:
NMR ($CCl_4$): δ 1.25, t (J=7Hz), 3H; 4.18, q (J=7Hz), 2H; 7.27, s, 5H; 7.80, d (J=81Hz), 1H.

(b) Ethyl (*E*)-2-(3'-methoxy)phenyl-3-fluoroacrylate: b.p. 74—75°/0.05 mm:
NMR ($CDCl_3$): δ 1.35 t (J=7Hz), 3H; 3.80, s, 3H; 4.25, q (J=7Hz), 2H; 6.77 to 7.47, m, 4H; 7.72, d (J=82Hz), 1H.

Analysis for $C_{12}H_{13}FO_3$:
Found: C, 63.93; H, 5.89%
Requires: C, 64.28; H, 5.84%

(c) Ethyl (*E*)-2-(2'-methoxy)phenyl-3-fluoroacrylate: b.p. 88°/0.05 mm:
NMR ($CCl_4$): δ 1.20, t (J=7Hz), 3H; 3.72, s, 3H; 4.13, q (J=7Hz), 2H; 6.67 to 7.42, m, 4H; 7.57, d (J=82Hz), 1H.

Analysis for $C_{12}H_{13}FO_3$:
Found: C, 64.45; H, 5.82%
Requires: C, 64.28; H, 5.84%

(d) Ethyl (*E*)-2-(3'-trifluoromethyl)phenyl-3-fluoroacrylate:
NMR ($CDCl_3$): δ 1.30, t (J=7Hz), 3H; 4.28, q (J=7Hz), 2H; 7.55, m 4H; 7.80, d (J=80Hz), 1H.

(e) Ethyl (*E*)-2-(4'-chloro)phenyl-3-fluoroacrylate:
NMR ($CCl_4$): δ 1.27, t (J=7Hz), 3H; 4.22, q (J=7Hz), 2H; 7.27, s, 4H; 7.67, d (J=81Hz), 1H.

(f) Ethyl (*E*)-2-(4'-methoxy)benzyl-3-fluoroacrylate: b.p.104°/0.04 mm:
NMR ($CDCl_3$): δ 1.18, t (J=7Hz), 3H; 3.53, d (J=3Hz), 2H; 3.70, s, 3H; 4.12, q (J=7Hz), 2H; centred at 6.93, $A_2B_2$ ($J_{AB}$=9Hz), 4H; 7.55, d (J=83Hz), 1H.

Analysis for $C_{13}H_{15}FO_3$:
Found: C, 65.50; H, 6.49%
Requires: C, 65.53; H, 6.34%

(g) Ethyl (*E*)-2-benzyl-3-fluoroacrylate: b.p. 75° (oven)/0.05 mm:
NMR ($CDCl_3$): δ 1.18, t (J=7Hz), 3H; 3.60, d (J=3Hz), 2H; 4.12, q (J=7Hz), 2H; 7.18, s, 5H; 7.60, d (J=83Hz), 1H.

(h) Ethyl (*E*)-2-(4'-methoxy)phenyl-3-fluoroacrylate: b.p. 89—90°/0.04 mm:
NMR ($CDCl_3$): δ 1.42, t (J=7Hz), 3H; 3.90, s, 3H; 4.37, q (J=7Hz), 2H; centred at 7.17, $A_2B_2$ ($J_{AB}$=9Hz), 4H; 7.77, d (J=80Hz), 1H.

Analysis for $C_{12}H_{13}FO_3$:
Found: C, 63.80; H, 5.83%
Requires: C, 64.28; H, 5.84%

Example 10

(*E*)-2-(3',4'-Dimethoxy)phenyl-3-fluoroallyl alcohol

A solution of ethyl (*E*)-2-(3',4'-dimethoxy)-3-fluoroacrylate (35 g) in THF (650 ml) is cooled to about −78° and treated with a solution of diisobutylaluminum hydride (690 ml) in hexane (1 M solution). The cooling bath is removed and the temperature is allowed to rise to room temperature over about 4½ hours. The solution is again cooled (*ca* 5°) and is then cautiously treated with methanol (140 ml) followed by 10% aqueous KOH (70 ml). The mixture is dried by the addition of $MgSO_4$ and filtered. The solids are washed thoroughly with methanol. Solvent is removed by evaporation to leave an almost colorless, crystalline mass (25.48 g). Usually, this product is used directly in the next reaction step without purification. If necessary, the product can be recrystallized from *n*-hexane which gives (*E*)-2-(3',4'-dimethoxy)phenyl-3-fluoroallyl alcohol: colorless plates, m.p. 56—57°:

NMR ($CDCl_3$): δ 2.60, s (broad), 1H; 3.83, s, 6H; 4.25, d (J=5Hz), 2H; 6.78, d (J=83Hz), 1H; 6.68 to 7.20, m; 3H.

Analysis for $C_{11}H_{13}FO_3$:
Found: C, 62.21; H, 6.32%
Requires: C, 62.26; H, 6.17%

Example 11

Repeating the procedure of Example 10 but substituting the appropriate starting materials for ethyl (*E*)-2-(3,4-dimethoxy)phenyl-3-fluoroacrylate, the following compounds are obtained:

(a) (*E*)-2-Phenyl-3-fluoroallyl alcohol:
NMR ($CDCl_3$): δ 1.68, s, 1H; 4.33, d (broadened, J=5Hz), 2H; 6.17, s, ½H; 7.20 to 7.63, m, 5½H.

(b) (*E*)-2-(3'-Methoxy)phenyl-3-fluoroallyl alcohol: m.p. 47—48°:
NMR ($CDCl_3$): δ 2.13, s, 1H; 3.85, s, 3H; 4.37, m, 2H; 6.92, d (broad, J=82Hz), 1H; 7.13 to 7.53, m, 4H.

(c) (*E*)-2-(2'-Methoxy)phenyl-3-fluoroallyl alcohol:
NMR ($CCl_4$): δ 3.27, s, 1H; 3.67, s, 3H; 4.08, d.d (J=5Hz, 1.5Hz), 2H; 6.60 to 7.40, m, 4H; 6.45, d (J=82Hz), 1H.

(d) (*E*)-2-(3'-Trifluoromethyl)phenyl-3-fluoroallyl alcohol.

(e) (*E*)-2-(4'-Chloro)phenyl-3-fluoroallyl alcohol.

(f) (*E*)-2-(4'-Methoxy)benzyl-3-fluoroallyl alcohol:
NMR ($CDCl_3$): δ 2.48, s (broad), 1H; 3.30, d (J=2Hz), 2H; 3.60, s, 3H; 3.70, d (J=4Hz), 2H; 6.52, d (J=84Hz), 1H; centred at 6.82, $A_2B_2$ ($J_{AB}$=9Hz), 4H.

(g) (*E*)-2-Benzyl-3-fluoroallyl alcohol:
NMR ($CCl_4$): δ 3.43, m, 3H; 3.72, d (broad, J=4Hz), 2H; 6.53, d (J=85Hz), 1H; 7.17, m, 5H.

(h) (*E*)-2-(4'-Methoxy)phenyl-3-fluoroallyl alcohol: m.p. 43—44°:
NMR ($CDCl_3$): δ 1.93, s, 1H; 3.80, s, 3H; 4.33, d (broad, J=4.5Hz), 2H; 6.82, d (J=82Hz), 1H; centred at 7.20, $A_2B_2$ ($J_{AB}$=9Hz).

Example 12

($E$)-1-Fluoro-2-(3',4'-dimethoxy)phenyl-3-phthalimidopropene

A solution of ($E$)-2-(3',4'-dimethoxy)phenyl-3-fluoroallyl alcohol (25 g), triphenylphosphine (31.23 g), and phthalimide (17.52 g) in THF (450 ml) is treated with a solution of diethyl azodicarboxylate (20.74 g) in THF (100 ml). The mixture is then stirred for about 16 hours. The THF is evaporated, and the by-products are largely removed by recrystallization from toluene, and then from ether. The solvent is evaporated and the residue is purified by chromatography on silica gel (1 kg) using 20% ethyl acetate in light petroleum. The major fraction (20.48 g) is recrystallized from dichloromethane/$n$-hexane to give ($E$)-1-fluoro-2-(3',4'-dimethoxy)phenyl-3-phthalimidopropene (16.50 g): colorless plates, m.p. 102—103°:

NMR ($CDCl_3$): δ 3.80, 3.85, two overlapping singlets, 6H; 4.52, m, 2H; 6.32, s, ½H, 6.68 to 7.28, m, 3H; 7.52 to 7.88, m, 4½H.

Analysis for $C_{19}H_{16}FNO_4$:

| | | | |
|---|---|---|---|
| Found: | C, 66.76; | H, 4.89; | N, 4.34% |
| Requires: | C, 66.86; | H, 4.72; | N, 4.10% |

Example 13

Repeating the procedure of Example 12 but substituting the appropriate starting materials for ($E$)-2-(3',4'-dimethoxy)phenyl-3-fluoroallyl alcohol, the following compounds are obtained:

(a) ($E$)-1-Fluoro-2-phenyl-3-phthalimidopropene: m.p. 98—99°:

NMR ($CDCl_3$): δ 4.55, m, 2H; 6.32, s (broad), ½H; 7.13 to 7.93, m, 9½H.

Analysis for $C_{17}H_{12}FNO_2$:

| | | | |
|---|---|---|---|
| Found: | C, 72.63; | H, 4.49; | N, 4.47% |
| Requires: | C, 72.59; | H, 4.30; | N, 4.98% |

(b) ($E$)-1-Fluoro-2-(3'-methoxy)phenyl-3-phthalimidopropene: m.p. 85—86°:

NMR ($CDCl_3$): δ 3.80, s, 3H; 4.57,m, 2H; 6.30, s (broad), ½H; 6.63 to 7.43, m, 4H; 7.50 to 7.96, m, 4½H.

·(c) ($E$)-1-Fluoro-2-(2'-methoxy)phenyl-3-phthalimidopropene: m.p. 128—129°:

NMR ($CDCl_3$): δ 3.68, s, 3H; 4.50, m, 2H; 6.20, s, ½H; 6.53 to 7.40, m, 4H; 7.60, m, 4½H.

Analysis for $C_{18}H_{14}FNO_3$:

| | | | |
|---|---|---|---|
| Found: | C, 69.43; | H, 4.69; | N, 4.48% |
| Requires: | C, 69.45; | H, 4.53; | N, 4.50% |

(d) ($E$)-1-Fluoro-2-(3'-trifluoromethyl)phenyl-3-phthalimidopropene:

NMR ($CDCl_3$): δ 4.57, d (J=4H, broad), 2H; 3.82, s, ½H; 4.38 to 7.83, m, 8½H.

(e) ($E$)-1-Fluoro-2-(4'-chloro)phenyl-3-phthalimidopropene: m.p. 118—119°:

NMR ($CDCl_3$): δ 4.50, m, 2H; 6.32, s (broad), ½H; 7.07 to 7.83, m, 8½H.

Analysis for $C_{17}H_{11}ClFNO_2$:

| | | | |
|---|---|---|---|
| Found: | C, 64.57; | H, 3.67; | N, 4.32% |
| Requires: | C, 64.67; | H, 3.51; | N, 4.44% |

(f) ($E$)-1-Fluoro-2-(4'-methoxy)benzyl-3-phthalimidopropene: m.p. 138—139°:

NMR ($CDCl_3$): δ 3.33, d (J=2.5Hz), 2H; 3.58, s, 3H; 4.07, d (J=3Hz), 2H; centred at 6.83, $A_2B_2$ ($J_{AB}$=9Hz), 4H; 6.88, d (J=86Hz), 1H; 7.68, s (broad), 4H.

(g) ($E$)-1-Fluoro-2-benzyl-3-phthalimidopropene: m.p. 114—115°:

NMR ($CDCl_3$): δ 3.45, d (J=2.5Hz), 2H; 4.13, d.d (J=3Hz, 1Hz), 2H; 6.21, s (broad), ½H; 7.20 to 7.30, m, 5H; 7.67, m, 5½H.

Analysis for $C_{18}H_{14}FNO_2$:

| | | | |
|---|---|---|---|
| Found: | C, 73.22; | H, 5.16; | N, 4.63% |
| Requires: | C, 73.21; | H, 4.78; | N, 4.74% |

(h) ($E$)-1-Fluoro-2-(4'-methoxy)phenyl-3-phthalimidopropene: m.p. 169—170°:

NMR ($CDCl_3$): δ 3.78, s, 3H; 4.55, d.d (J=3.5Hz, 1.5Hz), 2H; 7.00, m, ½H; centred at 7.50, $A_2B_2$ ($J_{AB}$=9Hz), 4H; 7.63 to 7.90, m, 4½H.

Analysis for $C_{18}H_{14}FNO_3$:

| | | | |
|---|---|---|---|
| Found: | C, 69.42; | H, 4.51; | N, 4.40% |
| Requires: | C, 69.45; | H, 4.53; | N, 4.50% |

Example 14

(*E*)-2-(3′,4′-Dimethoxy)phenyl-3-fluoroallylamine

A mixture of (*E*)-1-fluoro-2-(3′,4′-dimethoxy)phenyl-3-phthalimdopropene (6.82 g) and hydrazine hydrate (1.10 g) in methanol (45 ml) is refluxed for 3 hours. To the reaction mixture, is added 18% aqueous hydrochloric acid (12 ml). Refluxing is continued for another 30 minutes. The mixture is cooled and filtered. Solvent is removed by evaporation to give a residue which is triturated several times with methanol. Crystallization of the solid residue from ethanol/diethyl ether gives (*E*)-2-(3′,4′-dimethoxy)phenyl-3-fluoroallylamine, as the hydrochloride (2.56 g): colorless plates: m.p. 216—217°:

NMR ($D_2O$): δ 3.87, s, 6H overlapping 4.00, d (broad, J=4Hz), 2H; 7.10, s (broad), 3H; 7.17, d (J=82Hz), 1H.

Analysis for $C_{11}H_{15}ClFNO_2$:

| | | | |
|---|---|---|---|
| Found: | C, 53.38; | H, 6.02; | N, 5.60% |
| Requires: | C, 53.34; | H, 6.10; | N, 5.65% |

Example 15

Repeating the procedure of Example 14, but substituting the appropriate starting materials for (*E*)-fluoro-2-(3′,4′-dimethoxy)phenyl-3-phthalimidopropene, the following compounds are obtained:

(a) (*E*)-2-Phenyl-3-fluoroallylamine, as the hydrochloride: m.p. 195—196°:
NMR ($D_2O$): δ 3.98, d (J=3Hz), 2H; 7.13, d (J=8Hz), 1H; 7.50, s, 5H.

Analysis for $C_9H_{11}ClFN$:

| | | | |
|---|---|---|---|
| Found: | C, 57.53; | H, 5.93; | N, 7.52% |
| Requires: | C, 57.61; | H, 5.91; | N, 7.46% |

(b) (*E*)-2-(3′-Methoxy)phenyl-3-fluoroallylamine, as the hydrochloride: m.p. 146—147°:
NMR ($D_2O$): δ 3.87, s, 3H; 4.00, d (J=3.5Hz), 2H; 7.18, d (J=80Hz), 1H; 6.91 to 7.67, m, 4H.

Analysis for $C_{10}H_{13}ClFNO$:

| | | | |
|---|---|---|---|
| Found: | C, 55.25; | H, 5.81; | N, 6.41% |
| Requires: | C, 55.18; | H, 6.02; | N, 6.43% |

(c) (*E*)-2-(2′-Methoxy)phenyl-3-fluoroallylamine, as hydrochloride: m.p. 224—225°:

Analysis for $C_{10}H_{13}ClFNO$:

| | | | |
|---|---|---|---|
| Found: | C, 55.10; | H, 5.89; | N, 6.41% |
| Requires: | C, 55.18; | H, 6.02; | N, 6.43% |

(d) (*E*)-2-(4′-Chloro)phenyl-3-fluoroallylamine, as the hydrochloride: m.p. 190°:
NMR ($CD_3OD$): δ 3.97, d (broad, J=4Hz), 2H; 7.27, d (J=81Hz), 1H; 7.53, s, 4H.

Analysis for $C_9H_{10}Cl_2FN$:

| | | | |
|---|---|---|---|
| Found: | C, 48.47; | H, 4.44; | N, 6.26% |
| Requires: | C, 48.67; | H, 4.54; | N, 6.31% |

(e) (*E*)-2-(4′-Methoxy)benzyl-3-fluoroallylamine, as the hydrochloride: m.p. 185—186°:

Analysis for $C_{11}H_{15}ClFNO$:

| | | | |
|---|---|---|---|
| Found: | C, 57.09; | H, 6.49; | N, 6.00% |
| Requires: | C, 57.02; | H, 6.53; | N, 6.05% |

(f) (*E*)-2-Benzyl-3-fluoroallylamine, as the hydrochloride: m.p. 179°:
NMR ($D_2O$): δ 3.50, d.d (J=3Hz, 1Hz) 2H; 3.63, d (J=2.5Hz), 2H; 7.05, d.m (J=82Hz), 1H; 7.37, s, 5H.

Analysis for $C_{10}H_{13}ClFN$:

| | | | |
|---|---|---|---|
| Found: | C, 59.30; | H, 6.43; | N, 6.91% |
| Requires: | C, 59.56; | H, 6.50; | N, 6.95% |

(g) (*E*)-2-(4′-Methoxy)phenyl-3-fluoroallylamine, as the hydrochloride: m.p. 87°:
NMR ($D_2O$): δ 3.88, s, 3H; 3.97, d (broad, J=3.5Hz), 2H; 7.12, d (J=82Hz), 1H; centred at 7.30, $A_2B_2$ ($J_{AB}$=9Hz), 4H.

Analysis for $C_{10}H_{13}ClFNO$:

| | | | |
|---|---|---|---|
| Found: | C, 54.84; | H, 5.90; | N, 6.24% |
| Requires: | C, 55.18; | H, 6.03; | N, 6.43% |

Example 16

(*E*)-*N*-Methoxycarbonyl 2-phenyl-3-fluoroallylamine

A solution of phosphorus tribromide (227 mg) in toluene (2 ml) is slowly added to a solution of (*E*)-2-phenyl-3-fluoroallyl alcohol (305 mg) in toluene at about −5° so that the temperature does not rise above 0°. The cooling bath is removed and stirring is continued for 3 hours. The reaction mixture is then poured into saturated aqueous potassium carbonate (20 ml). The mixture is extracted with ether and the ether solution is washed with water and dried ($MgSO_4$). Evaporation of solvent gives (*E*)-2-phenyl-3-fluoroallyl bromide (318 mg): colorless oil:

NMR ($CDCl_3$): δ 4.13, d (J=4Hz), 2H; 6.85, d (J=80Hz), 1H; 7.10 to 7.50, m, 5H.

Without purification, a portion of this bromide (185 mg) is heated at 100° for 3 hours in dimethylformamide (DMF, 5 ml) containing methanol (65 mg) and potassium cyanate (60 mg). The mixture is cooled and filtered. The filtrate is then diluted with water and extracted with ether. The ether extract is washed with water, dried ($MgSO_4$), and evaporated to give yellowish solid (135 mg). Recrystallization from diethyl ether/light petroleum gives (*E*)-*N*-methoxycarbonyl 2-phenyl-3-fluoroallylamine (123 mg): colorless needles, m.p. 73—74°:

NMR ($CDCl_3$): δ 3.49, s, 3H; 3.90, m, 2H; 5.23, S (broad), 1H; 6.00, s (broad), ½H; 7.07 to 7.50, m, 5½H.

Example 17

(*E*)-*N*-*tert*-Butoxycarbonyl-2-(4′-chloro)phenyl-3-fluoroallylamine

A mixture of the hydrochloride salt of (*E*)-2-(4′-chloro)phenyl-3-fluoroallylamine (1.23 g), sodium bicarbonate (0.48 g), sodium chloride (1.1 g), and di-*tert*-butyl dicarbonate (1.22 g) in chloroform (50 ml) and water (9 ml) is refluxed for 2 hours. The reaction mixture is cooled and extracted with chloroform. The chloroform soluble material is recrystallized from hexane/light petroleum to give the title compound (0.98 g) as cream needles: m.p. 50—51°.

Example 18

2-Phenyl-3-bromoallylamine

(A) 2-Phenyl-3-phthalimidopropene

A mixture of 2-phenyl-3-bromo-1-propene (75% of the mixture) and 1-bromo-2-phenyl-1-propene (25%) is prepared by the method of S. Reed, *J. Org. Chem., 30,* 3258 (1965).

A portion (30.13 g) of this mixture is treated with potassium phthalimide (21.20 g) in DFM (100 ml) at 90° for 3 hours. The mixture is then cooled in an ice-bath, quenched with cold water, and extracted with chloroform. The chloroform extract is washed with brine, dried ($MgSO_4$), and evaporated to give a partially crystalline mass. Trituration with methanol followed by recrystallization from chloroform/light petroleum affords 2-phenyl-3-phthalimidopropene (18.6 g): colorless needles, m.p. 122—124°:

NMR ($CDCl_3$): δ 4.63, m, 2H; 5.00, s (broad), 1H; 5.33, s, 1H; 6.97 to 7.90, m, 9H.

2-Phenyl-3-phthalimidopropene is a known compound. See Lattrell, R. and Lohaus, G., *Liebigs Ann Chem.,* 870 (1974).

(B) 1,2-Dibromo-2-phenyl-3-phthalimidopropane

To a stirred solution of 2-phenyl-3-phthalimidopropene (2.63 g) in carbon tetrachloride (50 ml) at about 5° in the absence of light is added dropwise a solution of bromine (1.76 g) in carbon tetrachloride (40 ml). The mixture is subsequently stirred for 3 hours, water (50 ml) is added, and the two phases are decolorized with an aliquot of aqueous sodium sulfite. Evaporation of the organic layer gives a colorless mass (4.25 g) which crystallizes from *n*-hexane/chloroform to give 1,2-dibromo-2-phenyl-3-phthalimidopropane (3.85 g): colorless needles, m.p. 162—163°:

NMR ($CDCl_3$): δ 4.42, q AB ($v_A$=4.60, $v_B$=4.23, J=12Hz), 2H; 4.65, m, 2H; 7.38 to 8.17, m, 9H.

Analysis for $C_{17}H_{13}Br_2NO_2$:

| | | | |
|---|---|---|---|
| Found: | C, 48.02; | H, 3.09; | N, 3.14% |
| Requires: | C, 48.26; | H, 3.10; | N, 3.31% |

(C) 1-Bromo-2-phenyl-3-phthalimidopropene

A solution of 1,2-dibromo-2-phenyl-3-phthalimidopropane (1.27 g) and 1,8-diazabicyclo[5.4.0]undec-7-ene (DBU, 0.51 g) in dimethyl sulfoxide (DMSO, 50 ml) is heated at 90° for 16 hours. The solution is cooled, diluted with ice-water, and exhaustively extracted with ether. The orange oil (0.98 g) is chromatographed on silica gel (75 g) with 20% ethyl acetate in light petroleum as eluant to give a colorless mass which is crystallized from *n*-hexane/dichloromethane to give 1-bromo-2-phenyl-3-phthalimidopropene (0.66 g): colorless plates, m.p. 128—129°:

NMR ($CDCl_3$): δ 4.58, d (J=1.5Hz), 2H; 6.48, t (J=1.5Hz), 1H; 7.28, s, 5H; 7.53 to 7.90, m, 4H.

Analysis for $C_{17}H_{12}BrNO_2$:

| | | | |
|---|---|---|---|
| Found: | C, 59.90; | H, 3.58; | N, 4.12% |
| Requires: | C, 59.67; | H, 3.53; | N, 4.09% |

(D) 2-Phenyl-3-bromoallylamine

A mixture of 1-bromo-2-phenyl-3-phthalimidopropene (0.34 g) and hydrazine hydrate (0.06 g) in methanol (5 ml) is refluxed for 2½ hours after which 50% aqueous hydrochloride acid (4 ml) is added. Heating is continued for another hour. The mixture is cooled and filtered. Evaporation of solvent gives a solid residue which is triturated several times with methanol. Recrystallization from ethanol/diethyl ether affords 2-phenyl-3-bromoallylamine as the hydrochloride (0.17 g): colorless needles, m.p. 190—191°:

NMR ($D_2O$): δ 4.03, s (broad), 2H; 6.93, s (broad), 1H; 7.48, s, 5H.

Analysis for $C_9H_{11}BrClN$:

| | | | |
|---|---|---|---|
| Found: | C, 43.48; | H, 4.37; | N, 5.62% |
| Requires: | C, 43.49; | H, 4.46; | N, 5.64% |

## Example 19

2-Phenyl-3,3-dibromoallylamine

(A) 1,1,2-Tribromo-2-phenyl-3-phthalimidopropane

Bromination of 1-bromo-2-phenyl-3-phthalimidopropene (1.03 g) using the procedure of Example 18 (B) gives a colorless solid (1.42 g). Recrystallization from *n*-hexane/dichloroform gives 1,1,2-tribromo-2-phenyl-3-phthalimidopropane (1.22 g): colorless needles, m.p. 175—176°:

NMR ($CDCl_3$): δ 4.67, m, 2H; 6.55, s, 1H; 7.08 to 7.83, m, 9H.

Analysis for $C_{17}H_{12}Br_3NO_2$:

| | | | |
|---|---|---|---|
| Found: | C, 40.81; | H, 2.48; | N, 2.89% |
| Requires: | C, 40.67; | H, 2.41; | N, 2.79% |

(B) 1,1-Dibromo-2-phenyl-3-phthalimidopropene

A solution of 1,1,2-tribromo-2-phenyl-3-phthalimidopropane (1.22 g) and DBU (0.50 g) in DMSO (50 ml) is heated at 85° for 4 hours. Work-up as described in Example 18 (C) gives 1,1-dibromo-2-phenyl-3-phthalimidopropene (0.71 g): colorless needles, m.p. 154—155°:

NMR ($CDCl_3$): δ 4.78, s, 2H; 7.13, s, 5H; 7.65, s, 4H.

Analysis for $C_{17}H_{12}Br_2NO_2$:

| | | | |
|---|---|---|---|
| Found: | C, 48.57; | H, 2.67; | N, 3.43% |
| Requires: | C, 48.45; | H, 2.63; | N, 3.33% |

(C) 2-Phenyl-3,3-dibromoallylamine hydrochloride

Treatment of 1,1-dibromo-2-phenyl-3-phthalimidopropene (421 mg) with hydrazine hydrate (55 mg) and methanol (6 ml) followed by 50% aqueous hydrochloric acid as described in Example 18 (D) gives 2-phenyl-3,3-dibromoallylamine hydrochloride (175 mg): colorless needles, m.p. 243—244°:

NMR ($CD_3OD$): δ 4.12, s (broad), 2H; 7.38, s, 5H.

Analysis for $C_9H_{12}Br_2ClN$

| | | | |
|---|---|---|---|
| Found: | C, 32.81; | H, 3.09; | N, 4.36% |
| Requires: | C, 33.02; | H, 3.08; | N, 4.28% |

## Example 20

(*Z*)- and (*E*)-2-Phenyl-3-chloroallylamine

(A) 1,2-Dichloro-2-phenyl-3-phthalimidopropane

A solution of 2-phenyl-3-phthalimidopropane (7.9 g) in dichloromethane (100 ml) is cooled in an ice-bath and protected from light. Chlorine gas is then bubbled through the solution for 5 minutes. After 10 minutes, the mixture is poured into brine (200 ml). The resulting mixture is then extracted with pentane. The pentane extract is washed successively with water, 2% aqueous sodium bicarbonate, and water. The extract is dried and the solvent removed by evaporation. The resulting colorless residue (9.30 g) is crystallized from *n*-hexane/dichloromethane to give 1,2-dichloro-2-phenyl-3-phthalimidopropane (6.70 g): colorless needles, m.p. 114—115°:

NMR ($CDCl_3$): δ 4.22, q AB ($v_A$=4.50, $v_B$=3.94, J=12.5Hz), 2H; 4.32, s, 2H; 7.17 to 7.97, m, 5H.

Analysis for $C_{17}H_{13}ClNO_2$:

| | | | |
|---|---|---|---|
| Found: | C, 61.40; | H, 3.94; | N, 4.18% |
| Requires: | C, 61.10; | H, 3.92; | N, 4.19% |

(B) (*Z*)- and (*E*)-1-Chloro-2-phenyl-3-phthalimidopropene

A solution of 1,2-dichloro-2-phenyl-3-phthalimidopropane (4.00 g) and DBU (2.74 g) in DMSO (200 ml) is heated at 95° for 12 hours. The reaction mixture is cooled, diluted with ice-water (300 ml), and extracted

with ether. The ether solution is washed with water and dried. Evaporation of solvent affords a brown oil (3.98 g). Chromatography on silica gel (170 g) using 10% ethyl acetate in light petroleum as eluant, gives two majors products. These are:

1. (*E*)-1-Chloro-2-phenyl-3-phthalimidopropene (0.91 g) which crystallizes from *n*-hexane/dichloromethane as colorless needles: m.p. 106—108°:

NMR ($CDCl_3$): δ 4.58, d (J=1Hz); 2H, 6.38, t (distorted), 1H; 7.30, s, 5H; 7.55 to 7.88, m, 4H.

Analysis for $C_{17}H_{12}ClNO_2$:

| | | | |
|---|---|---|---|
| Found: | C, 68.65; | H, 4.18; | N, 4.48% |
| Requires: | C, 68.58; | H, 4.06; | N, 4.70% |

2. (*Z*)-1-Chloro-2-phenyl-3-phthalimidopropene (0.93 g) which crystallizes from *n*-hexane/dichloromethane as colorless needles: m.p. 133—134°:

NMR ($CDCl_3$): δ 4.95, d (J=1.8Hz), 2H; 6.42, t (distorted), 1H; 7.32, s, 5H; 7.57 to 7.90, m, 4H.

Analysis for $C_{17}H_{12}ClNO_2$:

| | | | |
|---|---|---|---|
| Found: | C, 68.86; | H, 4.08; | N, 4.61% |
| Requires: | C, 68.58; | H, 4.06; | N, 4.70% |

(C) (*Z*)-2-Phenyl-3-chloroallylamine

(*Z*)-1-Chloro-2-phenyl-3-phthalimidopropene (450 mg) is treated as described in Example 14 with hydrazine hydrate (85 mg) in methanol (6 ml). Hydrolysis with 50% aqueous hydrochloric acid (4 ml) and recrystallization of the product from ethanol/diethyl ether gives (*Z*)-2-phenyl-3-chloroallylamine as the hydrochloride (142 mg): colorless needles, m.p. 156—157°:

NMR ($D_2O/DCl$): δ 4.32, s, 2H; 6.75, s, 1H; 7.40, s, 5H.

Analysis for $C_9H_{11}Cl_2N$:

| | | | |
|---|---|---|---|
| Found: | C, 53.02; | H, 5.57; | N, 6.83% |
| Requires: | C, 52.97; | H, 5.43; | N, 6.86% |

(D) (*E*)-2-Phenyl-3-chloroallylamine

(*E*)-1-Chloro-2-phenyl-3-phthalimidopropene (445 mg) is treated as described for the *Z*-isomer with hydrazine hydrate (85 mg) in methanol (6 ml). Hydrolysis with 50% aqueous hydrochloric acid gives (*E*)-2-phenyl-3-chloroallylamine hydrochloride (152 mg): colorless needles, m.p. 185—186°:

NMR ($D_2O/DCl$): δ 4.07, s (broad), 2H; 6.78, m, 1H; 7.50, s, 5H.

Analysis for $C_9H_{11}Cl_2N$:

| | | | |
|---|---|---|---|
| Found: | C, 52.86; | H, 5.45; | N, 6.75% |
| Requires: | C, 52.97; | H, 5.43; | N, 6.86% |

## Example 21

(A) (*E*)-*N*-Ethyl 2-(3'-methoxy)phenyl-3-fluoroallylamine.

(*E*)-2-(3'-Methoxy)phenyl-3-fluoroallylamine hydrochloride is treated with 10% aqueous sodium hydroxide, and the mixture is extracted with ether. The ether solution is washed with water and dried. Evaporation of the solvent yields the free amine as an oil.

A mixture of (*E*)-2-(3'-methoxy)phenyl-3-fluoroallylamine (220 mg) and freshly-distilled benzaldehyde (150 mg) in ethanol (1 ml) is refluxed for 45 minutes. Solvent is evaporated to give a residue (315 mg) which is dissolved in dichloromethane (3 ml) and treated with triethyloxonium tetrafluoroborate (230 mg) at room temperature for about 16 hours. The solvent is evaporated and the residue is refluxed for 30 minutes with water (2 ml) and ethanol (7 ml). After evaporation of solvent, the solution is diluted with water, washed with ether, and made alkaline with 10% aqueous sodium hydroxide. The alkaline solution is then extracted with ether. The ether extract is washed with water, dried, and evaporated to leave an orange oil (205 mg). This product is dissolved in ethanol and treated with a saturated solution of hydrogen chloride in ether. The resulting precipitate is crystallized from ethanol/diethyl ether to give (*E*)-*N*-ethyl 2-(3'-methoxy)phenyl-3-fluoroallylamine hydrochloride (181 mg): colorless needles, m.p. 166—167°:

NMR ($D_2O$): δ 1.25, t (J=7.5Hz), 3H; 3.10, q (J=7.5Hz), 2H; 3.87, s, 3H; 4.05, d (J=3Hz), 2H; 7.20, d (J=80Hz), 1H; 6.93 to 7.67, m, 4H.

Analysis for $C_{12}H_{17}ClFNO$:

| | | | |
|---|---|---|---|
| Found: | C, 58.79; | H, 6.80; | N, 5.55% |
| Requires: | C, 58.66; | H, 6.97; | N, 5.70% |

(B) (*E*)-*N*-Ethyl 2-(3',4'-dimethoxy)phenyl-3-fluoroallylamine.

Repeating the procedure of part (A), but substituting (*E*)-2-(3',4'-dimethoxy)phenyl-3-fluoroallylamine hydrochloride for (*E*)-2-(3'-methoxy)phenyl-3-fluoroallylamine hydrochloride, there is obtained: (*E*)-*N*-ethyl 2-(3',4'-dimethoxy)phenyl-3-fluoroallylamine, as the hydrochloride: m.p. 145°:

NMR ($D_2O$): δ 1.28, t (J=8Hz), 3H, 3.13, q (J=8Hz), 2H; 3.90, s, 6H; 4.03, d (broad, J=3Hz), 2H; 7.13, s (broad), 3H; 7.17, d (J=82Hz), 1H.

Analysis for $C_{13}H_{19}$ $ClFNO_2$:

| | | | |
|---|---|---|---|
| Found: | C, 56.67; | H, 6.97; | N, 5.04% |
| Requires: | C, 56.62; | H, 6.95; | N, 5.08% |

Example 22

2-Phenyl-3,3-difluoroallylamine

(A) Ethyl 2-bromodifluoromethyl-2-carbo-*tert*-butoxyphenylacetate.

A solution of ethyl 2-carbo-*tert*-butoxyphenylacetate (15.84 g, 60 mmol) in tetrahydrofuran (THF, 200 ml) is added to sodium hydride (5.76 g, *ca* 120 mmol, 50—55% dispersion in oil which was washed with dry light petroleum to remove the oil). When anion formation is complete, the bath temperature is raised to about 40° and a solution of dibromodifluoromethane (63 g, 300 ml) in THF (100 ml) is added. The mixture is stirred at this temperature for 30 minutes, and then is allowed to cool to room temperature over 3½ hours. The solvent is evaporated and the residue is treated with water. The water solution is then extracted with ether. The ether extract is washed with water, dried ($MgSO_4$), and evaporated to yield a yellow oil (21.21 g). Chromatography on silica gel (200 g) using an eluant of 3% ethyl acetate in light petroleum affords a colorless oil (19.69 g) of ethyl 2-bromodifluoromethyl-2-carbo-*tert*-butoxyphenylacetate:

NMR ($CCl_4$): δ 1.28, t (J=7Hz), 3H; 1.52, s, 9H; 4.25, q (J=7Hz), 2H; 7.13 to 7.55, m, 5H.

The product is contaminated with ethyl 2-difluoromethyl-2-carbo-*tert*-butoxyphenylacetate and possibly with ethyl 2-dibromofluoromethyl-2-carbo-*tert*-butoxyphenylacetate.

(B) Ethyl 2-phenyl-3,3-difluoroacrylate

A solution of impure ethyl 2-bromodifluoro-2-carbo-*tert*-butoxyphenylacetate (20.95 g) in trifluoroacetic acid (44 ml) is stirred at room temperature for 1 hour. The solvent is removed by evaporation to give a pale-brown oil (17.27 g) which is then dissolved in THF (350 ml) and treated with vigorous stirring with 2 M aqueous sodium hydroxide (25.7 ml, 1 equivalent) for 15 minutes. The solution is then diluted with water and extracted with ether. The ether extract is washed with water, dried ($MgSO_4$), and evaporated. The residual yellow oil (10.80 g) is distilled to afford ethyl 2-phenyl-3,3-difluoroacrylate: colorless oil:

NMR ($CCl_4$): δ 1.25, t (J=7Hz), 3H; 4.15, q (J=7Hz), 2H; 7.18, s (broad), 5H.

The product may be contaminated with small amounts of ethyl 2-phenyl-3-fluoroacrylate and ethyl 2-phenyl-3-bromo-3-fluoroacrylate.

(C) 2-Phenyl-3,3-difluoroallyl alcohol

A solution of impure ethyl 2-phenyl-3,3-difluoroacrylate (7.13 g, 33.6 mmol) in THF (180 ml) is cooled to about −78° and treated with a solution of diisobutylaluminum hydride (136 mmol) in hexane (1 M solution). The cooling bath is removed and the temperature is allowed to rise to room temperature over about 45 minutes. The solution is again cooled (*ca* 5°) and methanol (50 ml) and then 10% aqueous potassium hydroxide (13.5 ml) are cautiously added. The mixture is then dried ($MgSO_4$) and filtered. Removal of solvent yields a yellow oil (4.60 g). Chromatography on silica gel (200 g) using 20% ethyl acetate in light petroleum gives two major products. The first-eluted compound is 2-phenyl-3,3-difluoroallyl alcohol (1.94 g), an almost colorless oil:

NMR ($CCl_4$): δ 2.70, s, 1H; 4.13 to 4.43, m, 2H; 6.98 to 7.35, m, 5H.

The oil is used without purification in the following step. The second-eluted compound is 2-phenyl-3-fluoroallyl alcohol (1.42 g). In addition to the above fraction, additional material (1.00 g), which is a mixture of the two compounds, is obtained.

(D) 1,1-Dibromo-2-phenyl-3-phthalimidopropene

A solution of 2-phenyl-3,3-difluoroallyl alcohol (1.94 g), triphenylphosphine (2.99 g), and phthalimide (1.68 g) in THF (80 ml) is treated with a solution of diethyl azodicarboxylate (1.99 g) in THF (20 ml) at room temperature. The reaction is allowed to proceed about 16 hours. The THF is evaporated. Much of the byproduct can be removed by its recrystallization from toluene and then from ether. The ether-soluble material (3.22 g) is purified by chromatography on silica gel (200 g) using 10% ethyl acetate in light petroleum. Recrystallization of the major portion (2.12 g) from hexane affords 1,1-difluoro-2-phenyl-3-phthalimidopropene: colorless needles, m.p. 102—103°:

NMR ($CCl_4$): δ 4.67, m, 2H; 7.28, s (broad), 5H; 7.57 to 7.88, m, 4H.

Analysis for $C_{17}H_{11}F_2NO_2$:

| | | | |
|---|---|---|---|
| Found: | C, 68.40; | H, 3.78; | N, 4.68% |
| Requires: | C, 68.22; | H, 3.70; | N, 4.68% |

(E) 3-Phenyl-3,3-difluoroallylamine

A mixture of 1,1-difluoro-2-phenyl-3-phthalimidopropane (0.60 g) and hydrazine hydrate (0.11 g) in ethanol (4 ml) is vigorously stirred and refluxed for 1 hour. Water (4 ml) and concentrated hydrochloric acid (4 ml) are added, and the mixture is refluxed for another hour. The filtrate is washed with ether and evaporated to dryness to leave an almost colorless residue (0.41 g). The crude amine is purified *via* its *N-tert*-butoxycarbonyl derivative: colorless needles, m.p. 44—45°:

NMR ($CCl_4$): δ 1.33, s, 9H; 3.93 to 4.27, m, 2H; 4.60, s (broad), 1H; 7.27, s, 5H.

Analysis for $C_{14}H_{17}F_2NO_2$:

| | | | |
|---|---|---|---|
| Found: | C, 62.19; | H, 6.28; | N, 4.92% |
| Requires: | C, 62.44; | H, 6.36; | N, 5.20% |

The *N-tert*-butoxycarbonyl derivative (0.14 g) is treated for about 16 hours at room temperature with a saturated solution of hydrogen chloride gas in dry ether (20 ml). After removal of solvent, the residue (0.18 g) is recrystallized from ethanol/diethyl ether to give 2-phenyl-3,3-difluoroallylamine, as the hydrochloride (0.07 g): colorless needles, m.p. 139—140°:

NMR ($D_2O$): δ 4.10, s (broad), 2H; 7.43, s, 5H.

Analysis for $C_9H_{17}ClF_2N$:

| | | | |
|---|---|---|---|
| Found: | C, 52.53; | H, 5.00; | N, 6.74% |
| Requires: | C, 52.57; | H, 4.90; | N, 6.81% |

Example 23

2-(3′-Hydroxy)phenylallylamine

(A) 2-(3′-Methoxy)phenyl-3-bromopropene

A mixture of 2-(3′-methoxy)phenylpropene (3.00 g) and *N*-bromosuccinimide (3.60 g) in $CCl_4$ (1 ml) is heated at 180° (bath temperature) until a vigorous reaction occurs. The mixture is allowed to cool over 2 hours, after which it is mixed with more solvent. After filtration and evaporation of solvent, there is obtained a residual oil (4.18 g):

NMR ($CDCl_3$): δ 2.13, d (J=1.5Hz); 3.70 s; 4.22, s; 5.42, d (J=3Hz); 6.35, m; 6.57 to 7.37, m.

The oil is a mixture of 2-(3′-methoxy)phenyl-1-bromopropene (25%) and 2-(3′-methoxy)phenyl-3-bromopropene (75%).

(B) 2-(3′-Methoxy)phenyl-3-phthalimidopropene

A mixture of (0.43 g) containing 2-(3′-methoxy)phenyl-3-bromopropene (75%) and 2-(3′-methoxy)phenyl-1-bromopropene (25%) is treated with potassium phthalimide (2.77 g) in dimethyl formamide (35 ml) at 90° for 3 hours. Upon reaching room temperature, the reaction mixture is treated with ice-water, and the resulting mixture is extracted with $CHCl_3$. The organic layer is washed consecutatively with 10% aqueous KOH, water, and brine. Upon drying and removal of solvent by evaporation, there is obtained a colorless mass (3.62 g). Crystallization from *n*-hexane/$CH_2Cl_2$ affords 2-(3′-methoxy)phenyl-3-phthalimidopropene (3.11 g): colorless prisms, m.p. 118—119°:

NMR ($CDCl_3$): δ 3.73, s, 3H; 4.60, s (broad), 2H; 5.10, s (broad), 1H; 5.40, s (broad), 1H; 6.60 to 7.30, m, 4H; 7.40 to 7.87, m, 4H.

Analysis for $C_{18}H_{15}NO_3$:

| | | | |
|---|---|---|---|
| Found: | C, 73.81; | H, 5.13; | N, 4.65% |
| Requires: | C, 73.71; | H, 5.15; | N, 4.78% |

(C) 2-(3′-Hydroxy)phenyl-3-phthalimidopropene

A solution of 2-(3′-methoxy)phenyl-3-phthalimidopropene (2.93 g) in $CH_2Cl_2$ (25 ml) at about −78° is treated with boron tribromide (2.76 g). The cooling bath is removed and the mixture is stirred for 1 hour. It is then poured into ice-water and the mixture is stirred for another 30 minutes. The mixture is saturated with salt and extracted with $CH_2Cl_2$. The $CH_2Cl_2$ extract is washed with water, dried, and evaporated to give an orange oil (2.68 g). Chromatography on silica gel (120 g) using $CH_2Cl_2$ as eluant affords essentially pure 2-(3′-hydroxy)phenyl-3-phthalimidopropene (1.62 g): m.p. 108—109°:

NMR ($CDCl_3$): δ 4.60, s (broad), 2H; 5.00, s (broad), 1H; 5.33, s (broad), 1H; 6.53 to 7.26, m, 4H; 7.50 to 7.93, m, 4H.

(D) 2-(3′-Hydroxy)phenylallylamine

2-(3′-Hydroxy)phenyl-3-phthalimidopropene (1.40 g) is treated with hydrazine hydrate (0.28 g) to give the crude amine, which is converted into the *tert*-butoxycarbonyl derivative. Essentially pure *N-tert*-butoxycarbonyl 2-(3′-hydroxy)phenylallylamine (0.63 g), m.p. 65—66°; is obtained after chromatography on silica gel (30 g) using light petroleum (70%)/ether (30%) as eluant. Treatment of this compound with dry

ether saturated with hydrogen chloride gives a precipitate of 3-(3'-hydroxy)phenylallylamine as the hydrochloride (0.50 g): colorless needles, m.p. 172—173°:

Analysis for $C_9H_{12}ClNO$:
Found: C, 58.04; H, 6.23; N, 7.48%
Requires: C, 58.23; H, 6.52; N, 7.54%

Example 24

(*Z*)-2-Phenyl-3-fluoroallylamine

(A) 1,2-Dibromo-1-fluoro-2-phenyl-3-phthalimidopropane

To a stirred solution of (*E*)-1-fluoro-2-phenyl-3-phthalimidopropene (0.56 g) in $CH_2Cl_2$ (30 ml) in the absence of light and at 0° is added a solution of bromine (0.35 g) in $CH_2Cl_2$ (5 ml). The mixture is stirred for 24 hours after which the solution is decolorised by shaking with aqueous sodium sulfite. The organic layer is separated, dried, and evaporated to leave a colorless solid (0.88 g). Recrystallization from *n*-hexane/$CH_2Cl_2$ gives 1,2-dibromo-1-fluoro-2-phenyl-3-phthalimidopropane (0.66 g): colorless needles, m.p. 163—164°:

NMR ($CDCl_3$): δ 4.50, m, 2H; 7.83, d (J=47Hz), 1H; 7.07 to 7.90, m, 9H.

(B) (*Z*)-1-Fluoro-2-phenyl-3-phthalimidopropene

To a stirred solution of 1,2-dibromo-1-fluoro-2-phenyl-3-phthalimidopropane (0.66 g) in acetone (100 ml) is added solid sodium iodide (15 g). The mixture is refluxed for 4 hours, cooled, and decolorised by the addition of sufficient sodium sulfite. The acetone is evaporated to give a residue which is extracted with ether. The ether extract is then washed with water, dried, and evaporated to leave a colorless mass (0.34 g). Chromatography on silica gel (25 g) using light petroleum/ethyl acetate as eluant affords two pure substances. The first eluted is (*Z*)-1-fluoro-2-phenyl-3-phthalimidopropene (0.25 g), m.p. 78—79°:

NMR ($CDCl_3$): δ 4.78, d.d (J=3Hz, 1.5Hz), 2H; 6.80, d.t. (J=82Hz, 1.5Hz), 1H; 7.07 to 7.87, m, 9H.

Analysis for $C_{17}H_{12}FNO_2$:
Found: C, 72.30; H, 4.63; N, 4.70%
Requires: C, 72.59; H, 4.30; N, 4.98%

The second product is (*E*)-1-fluoro-2-phenyl-3-phthalimidopropene (0.08 g).

(C) (*Z*)-2-Phenyl-3-fluoroallylamine

(*Z*)-1-Fluoro-2-phenyl-3-phthalimidopropene (0.25 g) is treated with hydrazine hydrate (50 mg) in ethanol (2 ml), and then concentrated hydrochloric acid (2 ml) and water (2 ml). Recrystallization of the resulting product (0.19 g) from ethanol/ether affords pure (*Z*)-2-phenyl-3-fluoroallylamine, as the hydrochloride: m.p. 145°:

NMR ($D_2O$): δ 4.20, d (broad, J=3Hz), 2H; 7.13, d (J=82Hz), 1H; 7.50, m, 5H.

Analysis for $C_9H_{11}ClFN$:
Found: C, 57.60: H, 5.91; N, 7.52%
Requires: C, 57.61; H, 5.91; N, 7.46%

Example 25

(*E*)-2-(4'-Methoxy)phenyl-3-chloroallylamine

(A) Ethyl 2-chloromethyl-2-carbo-*tert*-butoxy(4'-methoxy)phenylacetate.

A solution of ethyl 2-carbo-*tert*-butoxy(4'-methoxy)phenylacetate (11.80 g) in THF (120 ml) is cooled to about −70° and treated consecutatively with potassium *tert*-butoxide (4.93 g) and *n*-butyllithium (33 ml, 50 mmol). The solution is stirred for 30 minutes after which the cooling bath is removed and the temperature is allowed to rise over 1 hour. The solution is then heated to 45°. Chloroform (12 g) is added dropwise over 15 minutes and the mixture is refluxed for 2 hours, cooled, poured into water, and extracted with ether. The ether extract is washed with water, dried, and evaporated to leave a dark oil (15.65 g). A small portion is purified by silica gel chromatography to give ethyl 2-chloromethyl-2-carbo-*tert*-butoxy(4'-methoxy)-phenylacetate: colorless oil, b.p. 119—120°/0.05 mm:

NMR ($CDCl_3$): δ 1.30, t (J=7Hz), 3H; 1.48, s, 9H; 3.80, s, 3H; 4.28, q (J=7Hz), 2H; 6.60, s, 1H; centred at 7.17, $A_2B_2$ ($J_{AB}$=9Hz), 4H.

Analysis for $C_{17}H_{22}Cl_2O_5$:
Found: C, 54.16; H, 5.79%
Requires: C, 54.12; H, 5.87%

(B) Ethyl (*E*)-2-(4′-methoxy)phenyl-3-chloroacrylate

Ethyl 2-chloromethyl-2-carbo-*tert*-butoxy(4′-methoxy)phenylacetate is treated according to the procedure of Example 8 to give ethyl (*E*)-2-(4′-methoxy)phenyl-3-chloroacrylate: b.p. 94—95°/0.05 mm:

NMR ($CDCl_3$): δ 1.20, t (J=7Hz), 3H; 3.67, s, 3H; 4.13, q (J=7Hz), 2H; centred at 7.00, $A_2B_2$ (J=9Hz), 4H; 7.43, s, 1H.

Analysis for $C_{12}H_{13}ClO_3$:

| | | |
|---|---|---|
| Found: | C, 59.88; | H, 5.32% |
| Requires: | C, 59.88; | H, 5.44% |

(C) (*E*)-2-(4′-Methoxy)phenyl-3-chloroallyl alcohol

A solution of ethyl (*E*)-2-(4′-methoxy)phenyl-3-chloroacrylate (4.80 g) in hexane (50 ml) is added dropwise to a solution of diisobutylaluminum hydride (60 mmol) in hexane so that the temperature remains below 20°. The mixture is stirred for 2 hours, cooled, in an ice-bath, and treated consecutatively with methanol (8 ml) and 10% aqueous sulfuric acid (to give a pH of 4—5). The layers are separated and the hexane layer is washed with water, dried, and evaporated. The residue is crystallized from light petroleum/ether to obtain 2-(4′-methoxy)phenyl-3-chloroallyl alcohol (3.10 g): colorless needles, m.p. 58—59°:

NMR ($CDCl_3$): δ 3.10, s (broad), 1H; 3.73, s, 1H; 4.23, s (broad), 2H; 6.27, s (broad), 1H; centred at 7.07, $A_2B_2$ (J=9Hz), 4H.

(D) (*E*)-1-Chloro-2-(4′-methoxy)phenyl-3-phthalimidopropene

(*E*)-2-(4′-Methoxy)phenyl-3-chloroallyl alcohol is treated according to the procedure of Example 12 to give (*E*)-1-chloro-2-(4(methoxy)phenyl-3-phthalimidopropene: m.p. 154—155°:

NMR ($CDCl_3$): δ 3.70, s, 3H; 4.53, m, 2H; 6.32, m, 1H; centred at 7.07, $A_2B_2$ (J=9Hz), 4H; 7.43 to 7.83, m, 4H.

Analysis for $C_{18}H_{14}ClNO_2$:

| | | | |
|---|---|---|---|
| Found: | C, 65.91; | H, 4.51; | N, 4.20% |
| Requires: | C, 65.96; | H, 4.30; | N, 4.27% |

(E) 2-(4′-Methoxy)phenyl-3-chloroallylamine

(*E*)-1-Chloro-2-(4′-methoxy)phenyl-3-phthalimidopropene is treated according to the procedure of Example 14 to give 2-(4′-methoxy)phenyl-3-choroallylamine as the hydrochloride: m.p. 150—152°:

NMR ($D_2O$ + DCl): δ 3.87, s, 3H; 4.08, s, 2H; 6.78, s, 1H; centred at 7.27, $A_2B_2$ (J=9Hz), 4H.

Analysis for $C_{10}H_{13}Cl_2NO$:

| | | | |
|---|---|---|---|
| Found: | C, 51.05; | H, 5.39; | N, 5.86% |
| Requires: | C, 51.30; | H, 5.59; | N, 5.98% |

## Example 26

2-(3′-Methoxy)phenylallylamine hydrochloride

2-(3′-Methoxy)phenyl-3-phthalimidopropene is treated with hydrazine hydrate to give the crude amine which is purified by its *N*-protected derivative, *N-tert*-butoxycarbonyl 2-(3′-methoxy)phenylallylamine:

NMR ($CDCl_3$): δ 1.43, s, 9H; 4.07, d (J=6Hz), 2H; 4.83, t (J=6Hz), 1H; 5.13, s, 1H; 5.32, s, 1H; 6.63 to 7.30, m, 4H; 7.50, s, 1H.

Treatment of this derivative with ether/hydrogen chloride gives 2-(3′-methoxy)phenylallylamine, as the hydrochloride: colorless needles, m.p. 136°:

NMR ($D_2O$): δ 3.87, s, 3H; 4.13, s (broad), 2H; 5.50, t (J=1.5Hz), 1H; 5.70, s, 1H; 6.87 to 7.60, m, 4H.

Analysis for $C_{10}H_{14}ClNO$:

| | | | |
|---|---|---|---|
| Found: | C, 60.05; | H, 6.84; | N, 6.93% |
| Requires: | C, 60.15; | H, 7.07; | N, 7.01% |

## Example 27

(*E*)-1-Fluoro-2-(3′,4′-dihydroxyphenyl)-3-phthalimidopropene

A solution of (*E*)-1-fluoro-2-(3′,4′-dimethoxyphenyl)-3-phthalimidopropene (3.20 g) in dry methylene chloride (50 ml) is cooled to −78° and treated with a solution (molar) of boron tribromide in methylene chloride (31 ml; i.e. 3.3 equivalents). After 15 minutes, the cooling bath is removed and the stirring is continued for 30 minutes. The solution is then poured into a mixture of ice and water. The mixture is stirred for 30 minutes. The product is isolated by extraction with methylene chloride and recrystallized from ethyl

acetate/*n*-hexane. (*E*)-1-Fluoro-2-(3',4'-dihydroxy)phenyl-3-phthalimidopropene is obtained as cream needles (2.58 g): m.p. 184—185°:

NMR ($d_6$ acetone): δ 4.17, d.d (J=3Hz, 1.5 Hz), 2H; 6.10 to 6.33, m, 3H; 6.37, d (J=84Hz), 1H; 7.17, s, 4H; 7.37, s, 2H.

Analysis for $C_{17}H_{12}FNO_4$:

| | | | |
|---|---|---|---|
| Found: | C, 65.48; | H, 4.05; | N, 4.33% |
| Requires: | C, 65.18; | H, 3.86; | N, 4.47% |

Example 28

(*E*)-2-(3',4'-Dihydroxyphenyl)-3-fluoroallylamine

A mixture of (*E*)-1-fluoro-2-(3',4'-dihydroxyphenyl)-3-phthalimidopropene (0.62 g) and hydrazine hydrate (0.11 g) in ethanol (5 ml) is refluxed for 3 hours. The mixture is then cooled and filtered. Evaporation of the filtrate affords an orange foam (0.41 g), which is dissolved in THF (30 ml). Di-*tert*-butyl dicarbonate (0.44 g) is added and the solution is refluxed for 2 hours, cooled, filtered, and evaporated to give an orange oil (0.82 g). Chromatography on silica gel (100 g, 50% ethyl acetate in light petroleum) affords a crystalline substance which, upon recrystallization from chloroform, gives the BOC-derivative (0.63 g) as colorless plates: m.p. 145—146°. Deprotection is achieved by stirring a mixture of the BOC-derivative in ether (20 ml) saturated with dry hydrogen chloride for about 16 hours. Filtration affords (*E*)-2-(3',4'-dihydroxyphenyl)-3-fluoroallylamine as the hydrochloride (0.28 g): m.p. 193°:

NMR ($D_2O$): δ 3.92, d (J=3Hz), 2H; 6.97, m, 3H; 7.07, d (J=81Hz), 1H.

Analysis for $C_9H_{11}ClFNO_2$:

| | | | |
|---|---|---|---|
| Found: | C, 48.92; | H, 4.93; | N, 6.22% |
| Requires: | C, 49.22, | H, 5.05; | N, 6.38% |

Example 29

(*E*)-2-(3'-Hydroxy)phenyl-3-fluoroallylamine

Repeating the procedures of Examples 28 and 29 in sequence, but substituting the appropriate starting materials in each procedure, there is obtained (*E*)-2-(3'-hydroxy)phenyl-3-fluoroallylamine, as the hydrochloride: m.p. 143°:

NMR ($D_2O$): δ 4.00, d.d (J=3.5Hz, 1Hz), 2H; 6.83 to 7.57, m, 4H; 7.13, d (J=80Hz), 1H.

Analysis for $C_9H_{11}ClFNO$:

| | | | |
|---|---|---|---|
| Found: | C, 52.75; | H, 5.74; | N, 6.82% |
| Requires: | C, 53.08; | H, 5.44; | N, 6.88% |

Example 30

Ethyl 2-difluoromethyl-2-carbo-*tert*-butoxy(β)naphthylacetate

A solution of ethyl 2-carbo-*tert*-butoxy(β)naphthylacetate (1.54 g) in THF (10 ml) is added to a slurry of sodium *tert*-butoxide (0.94 g) in THF (10 ml). The mixture is stirred for 30 minutes at room temperature and the temperature is increased to 45°. A rapid stream of chlorodifluoromethane (Freon 22) is introduced for about 5 minutes, the heating bath is removed, and the mixture is stirred for 1 hour. Water is added and the product is isolated by ether extraction. Essentially pure ethyl 2-difluoromethyl-2-carbo-*tert*-butoxy(β)-naphthylacetate (1.68 g) is obtained as a pale orange oil. A small portion of the oil is purified by chromatography to give crystalline material: m.p. 70—71°:

NMR ($CDCl_3$): δ 1.28, t (J=7Hz), 3H; 1.50, s, 9H; 4.35, q (J=7Hz), 2H; 6.62, t (J=56Hz), 1H; 7.38 to 7.93, m, 7H.

Analysis for $C_{20}H_{22}F_2O_4$:

| | | |
|---|---|---|
| Found: | C, 65.81; | H, 6.18% |
| Requires: | C, 65.95; | H, 6.04% |

Example 31

(*E*)-2-(3',4'-Dimethoxy)phenyl-3-fluoroallyl alcohol

A solution of ethyl (*E*)-2-(3',4'-dimethoxy)-phenyl-3-fluoroacrylate (30 g) in a mixture of dry hexane (170 ml) and dry dichloromethane (40 ml) is cooled to 0°. To this is added a 1 M solution of diisobutylaluminum hydride (DIBAL—H) in hexane (170 ml) at such a rate that the temperature did not rise above 10°. After completion of the addition (about 20 minutes) the resulting solution is stirred at ambient temperature for 1 hour and is then cooled to about 5°. Methanol (130 ml) and 6 N hydrochloric acid (83 ml) are added. The temperature is kept below 10° with external cooling as before. The organic layer is separated and the aqueous layer is extracted several times with ether. The combined organic solutions are washed with water and dried. Evaporation of solvent affords (*E*)-2-(3',4'-dimethoxy)phenyl-3-fluoroallyl alcohol as a pale yellow solid mass (18.60 g).

Example 32

(*E*)-1-Fluoro-2-(3',4'-dimethoxyphenyl)-3-phthalimidopropene

A solution of 2-(3',4'-dimethoxyphenyl)-3-fluoroallyl alcohol (13.72 g) in dry toluene (200 ml) is cooled to about 10° and treated with a solution of phosphorous tribromide (7.59 g) in toluene (200 ml). The reaction is allowed to continue for 1½ hour without cooling during which time some tar forms. The supernatant is poured into saturated aqueous potassium carbonate. Ether extraction gives (*E*)-1-fluoro-2-(3',4'-dimethoxyphenyl)-3-bromopropene, brown crystals (16.0 g). This intermediate (16 g) and potassium phthalimide (11.32 g) are heated in dry DMF (130 ml) at 65° for 4 hours, after which the mixture is poured into water. Extraction with ether gives (*E*)-1-fluoro-2-(3',4'-dimethoxyphenyl)-3-phthalimidopropene which is recrystallized from *n*-hexane/dichloromethane to give almost colorless needles (15.95 g).

Example 33

Repeating the procedures of Examples 2, 30, 8 and 10 in sequence, but substituting the appropriate starting materials in each procedure, the following compounds are obtained:

(a) (*E*)-2-α-naphthyl-3-fluoroallyl alcohol:

NMR ($CDCl_3$): δ 2.26, s (broad), 1H; 4.13, d (J=4Hz), 2H; 6.13, s, ½H; 7.13 to 8.00, m, 7½H.

(b) (*E*)-2-β-naphthyl-3-fluoroallyl alcohol:

NMR ($CDCl_3$): δ 2.73, s (broad), 1H; 4.23, d (J=4.5Hz), 2H; 6.07, s, ½H; 7.27 to 8.00, m, 7½H.

(c) (*E*)-2-(4'-methyl)phenyl-3-fluoroallyl alcohol:

NMR ($CDCl_3$): δ 2.30, s, 3H; 2.57, s (broad), 1H; 4.17, d (J=5Hz), 2H; 6.70, d (J=82Hz); 1H; centred at 7.23, $A_2B_2$ ($J_{AB}$=8Hz), 4H.

Example 34

Repeating the procedures of Examples 12 and 14 in sequence, but substituting the appropriate starting materials in each procedure, the following compounds are obtained:

(a) (*E*)-2-(α)-naphthyl-3-fluoroallylamine, as the hydrochloride: m.p. 246°:

NMR ($D_2O$): δ 4.07, d.d (J=3Hz, 1Hz), 2H; 6.68, s, ½H; 7.53 to 8.10, m, 7½H.

Analysis for $C_{13}H_{13}ClFN$:

| | | | |
|---|---|---|---|
| Found: | C, 65.59; | H, 5.44; | N, 5.90% |
| Requires: | C, 65.69; | H, 5.51; | N, 5.89% |

(b) (*E*)-2-(β)-naphthyl-3-fluoroallylamine, as the hydrochloride: m.p. 205°:

NMR ($CD_3OD$): δ 4.05, d (J=3.5Hz), 2H; 6.60, s, ½H; 7.37 to 8.07, m, 7½H.

Analysis for $C_{13}H_{13}ClFN$:

| | | | |
|---|---|---|---|
| Found: | C, 65.51; | H, 5.41; | N, 5.77% |
| Requires: | C, 65.69; | H, 5.51; | N, 5.89% |

(c) (*E*)-2-(4'-methyl)phenyl-3-fluoroallylamine, as the hydrochloride:

Analysis for $C_{10}H_{13}ClFN$:

| | | | |
|---|---|---|---|
| Found: | C, 59.56; | H, 6.52; | N, 6.71% |
| Requires: | C, 59.59; | H, 6.45; | N, 6.95% |

Example 35

Repeating the procedures of Examples 2, 7, 8 and 31, in sequence, but substituting the appropriate starting materials in each procedure, there is obtained (*E*)-2-(4'-methoxy)phenyl-3-chloroallyl alcohol:

NMR ($CDCl_3$): δ 3.10, s (broad), 1H; 3.73, s, 3H; 4.23, s, 2H; 6.27, s (broad), 1H; centred at 7.00, $A_2B_2$ ($J_{AB}$=9Hz), 4H.

Example 36

Repeating the procedures of Examples 12 and 14, in sequence, but substituting the appropriate starting materials in each procedure, there is obtained (*E*)-2-(4'-methoxy)phenyl-3-chloroallylamine, as the hydrochloride: m.p. 143°:

NMR ($D_2O$ + DCl): δ 3.88, s, 3H; 4.08, s, 2H; 6.80, s, 1H; centred at 7.28, $A_2B_2$ ($J_{AB}$=9Hz), 4H.

Analysis for $C_{10}H_{13}Cl_2NO$:

| | | | |
|---|---|---|---|
| Found: | C, 51.05; | H, 5.39; | N, 5.86% |
| Requires: | C, 51.30; | H, 5.60; | N, 5.98% |

Example 37

Repeating the procedure of Example 17 but substituting the appropriate starting materials in place of (*E*)-2-(4'-chloro)phenyl-3-fluoroallylamine, the following compounds are obtained:

(a) (*E*)-*N*-*tert*-butoxycarbonyl-2-(2'-methoxy)phenyl-3-fluoroallylamine: colorless oil: NMR ($CDCl_3$): δ 1.37, s, 9H; 3.75, s, 3H; 3.92, m, 2H; 4.80, m, 1H; 6.67 to 7.28, m, 4H; 6.73, d (J=82Hz), 1H.

Analysis for $C_{15}H_{20}FNO_3$:

| | | | |
|---|---|---|---|
| Found: | C, 64.03; | H, 7.32; | N, 4.94% |
| Requires: | C, 64.04; | H, 7.16; | N, 4.98% |

(b) (*E*)-*N*-*tert*-butoxycarbonyl-2-(4'-chloro)phenyl-3-fluoroallylamine: m.p. 50—51°: NMR ($CDCl_3$): δ 1.42, s, 9H; 4.00, m, 2H; 4.48, m, 1H; 6.80, d (J=82Hz), 1H; 7.38, s, 4H.

Analysis for $C_{14}H_{17}ClFNO_2$:

| | | | |
|---|---|---|---|
| Found: | C, 58.98; | H, 6.08; | N, 4.94% |
| Requires: | C, 58.85; | H, 6.00; | N, 4.90% |

Example 38

*tert*-Butyl 3,4-dimethoxyphenyl acetate

A ten-liter reaction flask is charged with 3,4-dimethoxyphenylacetic acid (800 g), *tert*-butyl acetate (8 l), and perchloric acid (24 ml). The mixture is stirred overnight at room temperature. The solution is poured slowly into a mixture of sodium bicarbonate (3.9 kg) and water (6.9 l). When the effervescence ceases, the mixture is filtered and after decantation the organic phase is dried over sodium sulfate (0.7 kg). The solvent is evaporated under reduced pressure and the residue is stirred for two hours at 0°C with heptane (1.3 l). After filtration and drying, *tert*-butyl 3,4-dimethoxyphenylacetate (672 g) is obtained: m.p. 75.8°.

Example 39

Ethyl 2-carbo-*tert*-butoxy(3',4'-dimethoxy)phenylacetate

A one-hundred liter stainless reactor under nitrogen is charged with diisopropylamine (5.9 l) and tetrahydrofuran (24 l). The solution is cooled to −78° and butyllithium (15% in hexane, 17 kg) is added during 1 hour. After stiring for 15 minutes, a solution of *tert*-butyl 3,4-dimethoxyphenylacetate (4.9 kg) in tetrahydrofuran (18 l) is added at −78°, and the mixture is maintained at this temperature during 1 hour. Then, a solution of ethyl chloroformate (2 l) in tetrahydrofuran (9 l) is added at −78° during 80 minutes. After the addition, the mixture is allowed to reach room temperature and is stirred overnight. The mixture is cooled to 10° and HCl (5 N, 3.4 l) is added. The THF is removed under reduced pressure and the residue is taken with methylene chloride (34 l) and water (4 l). The organic phase is washed twice with a solution of sodium chloride (1.6 kg in 8 l of water) and dried over sodium sulfate (5 kg). After filtration, the solvent is evaporated under reduced pressure and ethyl 2-carbo-*tert*-butoxy(3,4-dimethoxy)phenylacetate (6.345 kg) is obtained as an oil which is used without purification in the next step.

Example 40

Ethyl 2-difluoromethyl-2-carbo-*t*-butoxy(3',4'-dimethoxyphenyl)acetate

A six-liter reaction flask under nitrogen is charged with sodium *tert*-butoxide (250 g) and tetrahydrofuran (3 l). The mixture is stirred at room temperature and a solution of ethyl *t*-butyl 3,4-dimethoxyphenylmalonate (750 g) in tetrahydrofuran (1 l) is added in half an hour. The temperature rises to 32° and is maintained at 30—32° for half an hour at the end of the addition. Then, a rapid stream of chlorodifluoromethane is bubbled through the solution. The temperature rises to 65°. The stirring is continued under chlorodifluoromethane for 10 minutes. The mixture is evaporated under reduced pressure and the residue is stirred with methylene chloride (4 l) and a solution of acetic acid (0.3 l) in water (1.5 l). After decantation, the organic phase is washed with water (1.5 l) and brine (1.5 l) and dried over sodium sulfate (500 g). The solvent is evaporated under reduced pressure and the residue obtained is stirred overnight at room temperature with hexane (2 l). After filtration and drying, ethyl 2-difluoromethyl-2-carbo-*t*-butoxy(3',4'-dimethoxyphenyl)acetate (672 g) is obtained as a white solid: m.p. 55.4°.

Example 41

Ethyl (*E*)-2-(3',4'-dimethoxy)phenyl-3-fluoroacrylate

A twenty-liter reaction flask is charged with ethyl 2-difluoromethyl-2-carbo-*t*-butoxy(3',4'-dimethoxyphenyl)acetate (1.4 kg) and trifluoroacetic acid (6 l). The solution is stirred at 25° for 16 hours. The mixture is evaporated under reduced pressure. The residue is dissolved in tetrahydrofuran (5 l) and the solution is cooled at 10—15°. Then, aqueous sodium hydroxide 2 M (2.9 l) is added and the mixture is stirred at 25° for 1 hour. Diisopropyl ether (5 l) is added and after decantation, the aqueous phase is extracted with diisopropyl ether (2 × 2 l). The organic phase is combined, washed with brine (2 l) and dried over sodium sulfate (0.5 kg). After filtration and evaporation under reduced pressure, the residue is stirred at 10° for 1

hour, with a mixture of ethyl acetate (0.2 l) and hexane (2 l). The crystals are filtered, dried, and ethyl (*E*)-2-(3',4'-dimethoxy)phenyl-3-fluoroacrylate (0.725 kg) is obtained: m.p. 72.6°.

Example 42

(*E*)-2-(3',4'-Dimethoxyphenyl)-3-fluoroallyl alcohol

A sixty-liter stainless reactor under nitrogen is charged with ethyl 2-(3',4'-dimethoxy)phenyl-3-fluoroacrylate (980 g), methylene chloride (dried over $CaCL_2$) (1.4 l), and hexane dried over molecular sieves (5.6 l). The solution is stirred and cooled to −10/−5°. Then, a solution of DIBAL (1 M in hexane) (8.5 l) is added during 1 hour between −10° and −5°. At the end of the addition, the mixture is stirred two hours at room temperature. The solution is cooled at 0° and methanol (0.7 l) is added. Then, a solution of hydrochloric acid (34%) (2 l) in water (4 l) is added. The solvents are removed under reduced pressure. Then, methylene chloride (6 l) is added to the residue. After decantation, the aqueous phase is extracted with methylene chloride (2 × 2 l). The organic layers are combined and dried over sodium sulfate (500 g). After filtration and evaporation of the solvent under reduced pressure, the residue is dissolved in toluene (1 l) and hexane (4 l) is added with good stirring. The product crystallizes. The mixture is stirred 2 hours at room temperature and after filtration and drying, 2-(3',4'-dimethoxy)-phenyl-3-fluoroallyl alcohol (776 g) is obtained: m.p. 59°.

Example 43

(*E*)-1-Fluoro-2-(3',4'-dimethoxy)phenyl-3-phthalimido-1-propene

A ten-liter reaction flask is charged with 2-(3',4'-dimethoxy)phenyl-3-fluoroallyl alcohol (615 g) and toluene (5 l). The mixture is cooled to −5°/0° and a solution of phosphorus tribromide (105 ml) in toluene (0.8 l) is added at this temperature during 1½ hour. The mixture is stirred at room temperature for 2 hours and a solution of potassium carbonate (300 g) in water (1 l) is added. The mixture is stirred half an hour. After decantation the organic phase is washed with a solution of potassium carbonate (300 g) in water (1 l) and brine (1 l). After drying over sodium sulfate (500 g), the solvent is evaporated under reduced pressure. The residue is poured into a ten-liter reaction flask with DMF dried over molecular sieves (6.6 l). Then, potassium phthalimide is added and the mixture is stirred at 70° for 4 hours. The DMF is evaporated under reduced pressure and methylene chloride (8 l) and water (1.8 l) are added to the mixture. The organic phase is washed with water (2 l) and brine (2 × 2 l) and dried over sodium sulfate (500 g). After filtration, the solvent is evaporated under reduced pressure and the residue obtained is stirred at room temperature for 2 hours with methanol (2.5 l). The crystals are filtered, dried, and (*E*)-1-fluoro-2-(3',4'-dimethoxy)phenyl-3-phthalimido-1-propene (602 g) is obtained: m.p. 107.8°.

Example 44

(*E*)-2-(3',4'-dimethoxy)phenyl-3-fluoroallylamine

A ten-liter reaction flask is charged with 1-fluoro-2-(3',4'-dimethoxy)phenyl-3-phthalimido-1-propene (631 g), methanol (6 l), and hydrazine hydrate (103 g) and the mixture is refluxed overnight. A solution of hydrochloric acid (34%, 0.45 l) in water (0.45 l) is added and the refluxing is continued for 1 hour. After cooling to room temperature, the mixture is filtered and the filtrate is evaporated under reduced pressure. The residue is stirred overnight at 0° with water (0.45 l) and acetone (5 l). After filtration and drying crude (*E*)-2-(3',4'-dimethoxy)phenyl-3-fluoroallylamine (420.2 g) is obtained. A ten-liter reaction flask is charged with the crude product (420.2 g), chloroform (4 l), water (0.4 l) and triethylamine (0.4 l). The mixture is stirred for 30 minutes. After decantation the organic phase is washed with water (0.4 l). The aqueous phases are combined and extracted with chloroform (0.8 l). The organic phase is dried over sodium sulfate (500 g), filtered, and poured into a ten-liter reaction flask. Then, a solution of hydrochloric acid (34%, 300 ml) in water (3 l) is added with good stirring. After decantation, the aqueous phase is washed with chloroform (1 l). The aqueous phase is heated to 60° for 1 hour with charcoal (5 g). After filtration, the water is evaporated under reduced pressure. When crystallization begins, acetone (4 l) is added and the mixture is stirred for 2 hours at room temperature. The white crystals are filtered, washed with chloroform (1 l) and dried at 60° under reduced pressure. (*E*)-2-(3',4'-dimethoxy)phenyl-3-fluoroallylamine (392 g) is obtained.

The following Examples illustrate and describe the testing of the compounds of this invention for their ability to inhibit MAO enzyme.

Example 45

Inhibition of MAO — *In vitro* testing

(A) The ability of a compound of Formula I or II to inhibit MAO can be determined *in vitro* by the method of A. Christmas *et al., Br. J. Pharmacol., 45,* 490 (1972) in partially purified mitochondria from rat brain using $^{14}C$ *p*-tyramine as the substrate. The MAO inhibitory activity of a compound is expressed as the "$IC_{50}$" value, which is the molar concentration required to produce 50% inhibition of the enzyme. The $IC_{50}$ values for certain compounds of Formula I or II were determined using the above-described method, and the results are set forth in Table I. For comparison, $IC_{50}$ values for clorgyline, L-deprenyl, and pargyline are also given. The data shown in Table I does not show selectivity of the compounds against MAO—A or MAO—B inhibitors, since $^{14}C$ *p*-tyramine is a substrate for both forms of the enzyme.

TABLE I

MAO Inhibitory Activity — In vitro

| Compound (a) | $IC_{50}$ (moles) |
|---|---|
| (*E*)-2-(4′-methoxy)phenyl-3-chloroallylamine | $1 \times 10^{-4}$ |
| clorgyline | $1 \times 10^{-7}$ |
| L-deprenyl | $1 \times 10^{-7}$ |
| pargyline | $2 \times 10^{-6}$ |

(a) Tested as hydrochloride salt.

The data shown in Table I demonstrate that the compounds tested are potent inhibitors of MAO.

(B) The compounds of Formula I or II can be tested to determine whether or not the MAO inhibition follows time-dependent kinetics by the procedure described below:

Mitochondria are prepared from rat brain by homogenation in phosphate buffer (0.1 M, pH 7.2) followed by differential centrifugation. The mitochondria are suspended in the same buffer, the test compound is added at the desired concentration, and the system is incubated. At different time intervals, aliquots are taken and MAO activity is measured using $^{14}C$ *p*-tyramine (a mixed substrate) as the substrate (See A. Christmas *et al., supra*). When the compounds shown in Table I were tested according to the above-described procedure, the MAO inhibitory activity increased as a function of time of incubation. The initial rate of decrease of activity increased with increasing concentration of inhibitor. (*Z*)-2-Phenyl-3-chloroallylamine did not show time-dependent inhibitory kinetics. The inhibition of MAO was shown to be irreversible since dialysis against phosphate buffer (24 hours) did not restore enzyme activity.

(C) The selectivity of a compound of Formula I or II with respect to inhibition of MAO—A and MAO—B can be determined by repeating the procedure of Part B and measuring the MAO activity using $^{14}C$ 5-hydroxy-tryptamine (a preferred substrate for MAO—A) and $^{14}C$ phenethylamine (a preferred substrate for MAO—B) as the substrate in place of $^{14}C$ *p*-tyramine (a mixed substrate). Certain compounds of Formula I or II were tested for MAO selectivity according to the above-described procedure and the results shown in Table II were obtained. Selectivity is expressed as the ratio of the inhibitory activity against MAO—B versus the inhibitory activity against MAO—A.

TABLE II

Selectivity of MAO Inhibitors — In vitro

| Compound | Selectivity (MAO—B/MAO—A) |
|---|---|
| (*E*)-2-(4′-methoxy)phenyl-3-fluoroallylamine | 1000 |
| (*E*)-2-(3′,4′-dimethoxy)phenyl-3-fluoroallylamine | 500 |
| (*E*)-*N*-ethyl 2-(3′,4′-dimethoxy)-phenyl-3-fluoroallylamine | 100 |
| (*E*)-*N*-ethyl 2-(3′-methoxy)phenyl-3-fluoroallylamine | 5 |
| (*E*)-2-(4′-methoxy)benzyl-3-fluoroallylamine | 500 — 1000 |
| (*E*)-2-phenyl-3-fluoroallylamine | 10 |
| (*Z*)-2-phenyl-3-fluoroallylamine | 20 |
| (*E*)-2-benzyl-3-fluoroallylamine | 200 |

TABLE II (continued)

Selectivity of MAO Inhibitors — In vitro

| Compound | Selectivity (MAO—B/MAO—A) |
|---|---|
| (*E*)-2-(3′-methoxy)phenyl-3-fluoroallylamine | 4 |
| (*E*)-2-(4′-chloro)phenyl-3-fluoroallylamine | 10 — 20 |
| (*E*)-2-(2′-methoxy)phenyl-3-fluoroallylamine | 2 |
| (*E*)-2-(4′-trifluoromethyl)-phenyl-3-fluoroallylamine | 1 |
| (*E*)-2-(4′-methyl)phenyl-3-fluoroallylamine | 5 — 10 |
| (*E*)-2-(3′-methoxy)phenylallylamine | 1 |
| (*E*)-2-(α)-naphthyl-3-fluoroallylamine | 2 |
| (*E*)-2-(β)-naphthyl-3-fluoroallylamine | 20 |
| (*E*)-2-(3′-hydroxy)phenylallylamine | 0.2 |
| (*E*)-2-(3′-hydroxy)phenyl-3-fluoroallylamine | 0.1 |
| (*E*)-2-(3′,4′-dihydroxyphenyl)-3-fluoroallylamine | 0.1 |
| clorgyline | 0.001 |
| L-deprenyl | 100 |
| pargyline (a) | 10 |

(a) literature figure.

The results in Table II indicate that (*E*)-2-(4′-methoxy)phenyl-3-fluoroallylamine, (*E*)-2-(3′,4′-dimethoxy)phenyl-3-fluoroallylamine, (*E*)-2-(4′-methoxy)phenyl-3-fluoroallylamine, and (*E*)-2-benzyl-3-fluoroallylamine are particularly selective for MAO—B as compared to MAO—A.

Example 46

Inhibition of MAO — *Ex vivo*

The ability of a compound of Formula I or II to inhibit MAO can be determined *ex vivo* by the following procedure:

The test compound is administered intraperitoneally (i.p.), intravenously (i.v.), or orally (p.o.) to rats or mice and the animals are killed at various times after treatment. The brain, heart, liver, or duodenum is removed and either a crude homogenate or a mitochondrial fraction, described in Example 45, Part (A), is prepared, MAO activity is determined in the homogenates using $^{14}C$ *p*-tyramine, as the substrate. Table III gives the results of the testing of certain compounds according to the above-described procedure. Selectivity can be determined by repeating the above-described test using either $^{14}C$ *p*-hydroxytryptamine (for MAO—A) or $^{14}C$ phenethylamine (for MAO—B) as the substrate for determining the % inhibition.

TABLE III

% Inhibition of MAO — *Ex vivo*

| Dose (mg/kg) | Animal | Time (hr) | % Inhibition (1) Brain | Heart | Liver | Duodenum |
|---|---|---|---|---|---|---|
| (*E*)-2-phenyl-3-fluoroallylamine | | | | | | |
| 0.1 (iv) | rat | 24 | — | 85 | 48 | 67 |
| 0.5 (ip) | mouse | 24 | 45 | 91 | — | — |
| 1.0 (ip) | mouse | 24 | 68 | 97 | — | — |
| (*E*)-2-(3′,4′-dimethoxy)phenyl-3-fluoroallylamine | | | | | | |
| 0.5 (po) | rat | 18 | 79 | — | — | — |
| 1.0 (iv) | rat | 2 | — | 17 | 58 | 40 |
| 2.5 (po) | rat | 18 | 88 | — | — | — |
| 10.0 (po) | rat | 18 | 93 | — | — | — |
| (*E*)-2-(4′-methoxy)phenyl-3-fluoroallylamine | | | | | | |
| 0.1 (iv) | rat | 2 | — | 50 | 30 | 80 |
| 0.25 (po) | rat | 18 | 75 | — | — | — |
| 1.0 (po) | rat | 18 | 86 | — | — | — |
| 5.0 (po) | rat | 18 | 97 | — | — | — |
| (*E*)-2-(3′-methoxy)phenyl-3-fluoroallylamine | | | | | | |
| 0.1 (iv) | rat | 2 | — | 98 | 70 | 90 |
| Clorgyline | | | | | | |
| 0.1 (iv) | rat | 2 | — | 84 | — | 87 |
| 0.25 (po) | rat | 18 | 1 | — | — | — |
| 1.0 (po) | rat | 18 | 20 | — | — | — |
| 5.0 (po) | rat | 18 | 64 | — | — | — |
| L-deprenyl | | | | | | |
| 0.5 (po) | rat | 18 | 16 | — | — | — |
| 1.0 (po) | rat | 2 | — | 48 | 91 | 62 |
| 5.0 (po) | rat | 18 | 39 | — | — | — |
| 10.0 (po) | rat | 18 | 88 | — | — | — |

TABLE III (continued)

% Inhibition of MAO — *Ex vivo*

| Dose (mg/kg) | Animal | Time (hr) | % Inhibition (1) Brain | Heart | Liver | Duodenum |
|---|---|---|---|---|---|---|
| (*E*)-2-(3′,4′-dihydroxy)phenyl-3-fluoroallylamine | | | | | | |
| 10.0 (ip) | mouse | 18 | 99 | 99 | 84 | — |
| 10.0 (po) | mouse | 18 | 61 | 72 | 54 | — |
| 50.0 (ip) | mouse | 18 | 100 | 100 | 98 | — |
| 50.0 (po) | mouse | 18 | 99 | 100 | 96 | — |
| (*E*)-*N*-ethyl 2-(3′,4′-dimethoxy)phenyl-3-fluoroallylamine | | | | | | |
| 5.0 (po) | rat | 18 | 40 | — | 45 | — |
| 20.0 (po) | rat | 18 | 60 | — | 70 | — |
| (*E*)-2-(4(-methoxy)benzyl-3-fluoroallylamine | | | | | | |
| 1.0 (po) | rat | 24 | 25 | 45 | — | — |
| 10.0 (po) | rat | 24 | 70 | 60 | — | — |
| 2-(3′-hydroxy)phenylallylamine | | | | | | |
| 250.0 (ip) | mouse | 4 | 43 | 38 | — | — |
| 250.0 (ip) | mouse | 24 | 61 | 67 | 31 | — |

(1) determined by using $^{14}C$ *p*-tyramine as substrate.

Example 47

Inhibition of MAO — *In vivo*

The ability of a compound of Formula I or II to inhibit MAO can be determined *in vivo* in mice according to the following procedure, wherein changes in concentration of the endogenous MAO substrates and the metabolites thereof are assayed.

Mice are given an intraperitoneal or oral injection of the test compound and 2 hours, 16 hours, or 18 hours later, the animals are killed. The levels of dopamine, dihydroxyphenylacetic acid (DOPAC), 5-hydroxy-tryptamine (5—HT), and 5-hydroxyindole-3-acetic (5—HIAA) in brain homogenates are determined by the method of J. Wagner *et al., J. Chromatog., 164,* 41 (1979) and P. Bey *et al., Br. J. Pharmac., 70,* 571 (1980). When certain compounds of Formula I or II are tested according to the above-described procedure, the results given in Table IV are obtained:

TABLE IV

| Dose (mg/kg) | Time (hr) | Dopamine | % Change (compared to control) DOPAC | 5—HT | 5—HIAA |
|---|---|---|---|---|---|
| (*E*)-2-phenyl-3-fluoroallylamine | | | | | |
| 1 (ip) | 2 | +15 | −67 | +41 | −35 |
| 10 (ip) | 2 | +32 | −92 | +90 | −90 |
| 100 (ip) | 2 | +19 | −95 | +96 | −91 |
| (*E*)-2-(3′,4′-dihydroxy)phenyl-3-fluoroallylamine* | | | | | |
| 10 (ip) | 18 | +62 | −62 | +170 | −76 |
| 10 (po) | 18 | + 6 | −15 | +40 | −12 |
| 50 (ip) | 18 | +72 | −67 | +398 | −79 |
| 50 (po) | 18 | +77 | −79 | +221 | −74 |
| (*E*)-2-(3′,4′-dimethoxy)phenyl-3-fluoroallylamine | | | | | |
| 10 (ip) | 16 | +18 | −62 | +31 | −8 |
| 50 (ip) | 16 | +22 | −85 | +122 | −32 |
| 100 (ip) | 16 | +25 | −85 | +150 | −36 |
| L-deprenyl | | | | | |
| 10 (ip) | 2 | +10 | −21 | +18 | +9 |

*Determined by the method of J. Wagner *et al., J. Neurochem., 38,* 1241 (1982).

Example 48

The following test procedures can be employed to assess the potential of a compound of Formula I or II for producing the "cheese effect":

(A) Rats are administered an intravenous injection of the test compound at various dosage levels, and 30 minutes later, the rats are challenged with several intravenous or intraduodenal doses of *p*-tyramine. The heart rate response is measured. Compounds having the "cheese effect" will potentiate the heart rate response to *p*-tyramine. The results of the testing of certain compounds of Formula I or II are shown below in Table V, where the values given represent the factors by which the heart rate response to *p*-tyramine is increased after administration of the test compound.

TABLE V
Potentiation of heart rate response to *p*-tyramine

| Dose (iv) | Route of administration of *p*-tyramine | Potentiation of heart rate response to *p*-tyramine |
|---|---|---|
| (*E*)-2-(4′-methoxy)phenyl-3-fluoroallylamine | | |
| 0.1 | i.v. | 1.4 fold |
| 1.0 | i.v. | 5.2 fold |
| 0.1 | i.d. | 2.0 fold |
| 1.0 | i.d. | 4.6 fold |

TABLE V (continued)

Potentiation of heart rate response to *p*-tyramine

| Dose (iv) | Route of administration of *p*-tyramine | Potentiation of heart rate response to *p*-tyramine |
|---|---|---|
| (*E*)-2-(3′,4′-dimethoxy)phenyl-3-fluoroallylamine | | |
| 1.0 | i.v. | 2.3 fold |
| 1.0 | i.d. | 2.5 fold |
| (*E*)-2-phenyl-3-fluoroallylamine | | – |
| 0.1 | i.v. | 8.4 fold |
| 0.1 | i.d. | 9.1 fold |
| L-deprenyl | | |
| 0.1 | i.v. | 1.3 fold |
| 1.0 | i.v. | 2.2 fold |
| 0.1 | i.d. | no effect |
| 1.0 | i.d. | 2.1 fold |
| clorgyline | | |
| 0.1 | i.v. | 5.2 fold |
| 0.1 | i.d. | 5.6 fold |

i.v.: tyramine administered intravenously
i.d.: tyramine administered intraduodenally

(B) The procedure of Part (A) is repeated except that the tyramine is administered at a single dosage level (25 µg/kg) 15—30 minutes after administration of the test compound at a dosage of 0.01, 0.1, 1.0, and 10.0 mg/kg (i.v.). The potentiation of tyramine (as compared to controls) by the test compounds is shown in Table VI.

TABLE VI

Potentiation of tyramine in rats (a)

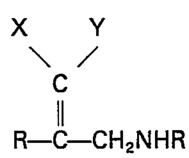

| Compound R | X | Y | R | Response (b) Dose (mg/kg, i.v.) 0.01 | 0.1 | 1.0 | 10.0 |
|---|---|---|---|---|---|---|---|
| 3-methoxyphenyl | F | H | H | 0 | *** | | |
| 4-chlorophenyl | F | H | H | 0 | ** | *** | |
| 3-hydroxyphenyl | F | H | H | * | *** | | |
| 3-methoxyphenyl | F | H | Ethyl | 0 | 0 | * | ** |

TABLE VI (continued)

| Compound R | X | Y | R | Response (b) Dose (mg/kg, i.v.) 0.01 | 0.1 | 1.0 | 10.0 |
|---|---|---|---|---|---|---|---|
| β-naphthyl | F | H | H | 0 | * | *** | |
| phenyl | F | F | H | 0 | 0 | * | *** |
| phenyl | Cl | H | H | 0 | 0 | 0 | * |
| phenyl | H | Cl | H | 0 | 0 | 0 | * |
| phenyl | Br | H | H | 0 | 0 | 0 | 0 |
| phenyl | Br | Br | H | 0 | 0 | 0 | 0 |
| 3,4-dimethoxy | F | H | Ethyl | 0 | 0 | 0 | 0 |

(a) Tyramine, 25 µg/kg, given i.v., 15—30 minutes after administration of the test compound at the indicated dose.

(b) 0: no effect on tyramine chronotropic response
*: marginal increase in tyramine chronotropic response
**: meaningful increase in tyramine chronotropic response
***: large increase in tyramine chronotropic response

In the following Examples relating to pharmaceutical compositions, the term "active compound" is used to indicate the compound (*E*)-2-(3′,4′-dimethoxy)phenyl-3-fluoroallylamine. This compound may be replaced in these compositions by any other compound of the invention. Adjustments in the amount of medicament may be necessary or desirable depending upon the degree of activity of the medicament as is well known in the art.

Example 49

An illustrative composition of hard gelatin capsules is as follows:

| | |
|---|---|
| (a) active compound | 5 mg |
| (b) talc | 5 mg |
| (c) lactose | 90 mg |

The formulation is prepared by passing the dry powders of (a) and (b) through a fine mesh screen and mixing them well. The powder is then filled into hard gelatine capsules at a net fill of 100 mg per capsule.

Example 50

An illustrative composition for tablets is as follows:

| | |
|---|---|
| (a) active compound | 5 mg |
| (b) starch | 45 mg |
| (c) lactose | 48 mg |
| (d) magnesium stearate | 2 mg |

The granulation obtained upon mixing the lactose with the compound (a) and the part of the starch and granulated with starch paste is dried, screened, and mixed with the magnesium stearate. The mixture is compressed into tablets weighing 100 mg each.

Example 51

An illustrative composition for an injectable suspension is the following 1 ml ampul for an intramuscular injection:

| | weight per cent |
|---|---|
| (a) active compound | 0.5 |
| (b) polyvinylpyrrolidone | 0.5 |
| (c) lecithin | 0.25 |
| (d) water for injection to make | 100.0 |

The materials (a)—(d) are mixed, homogenized, and filled into 1 ml ampuls which are sealed and autoclaved 20 minutes at 121°. Each ampul contains 5 mg per ml of the active compound.

**Claims for the Contracting States: BE CH DE FR IT LI LU NL SE**

1. A compound of the formula:

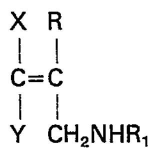 I or 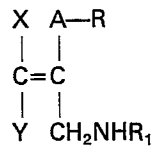 II

wherein:

R is 3,4-methylenedioxyphenyl; phenyl; phenyl monosubstituted, disubstituted, or trisubstituted by $(C_1—C_8)$alkyl, $(C_1—C_8)$alkoxy, $(C_1—C_8)$alkylcarbonyloxy, hydroxy, chlorine, bromine, iodine, fluorine, trifluoromethy, $(C_1—C_6)$alkylcarbonyl, benzoyl, or phenyl; 1- or 2-naphthyl; 1-, 2-, or 3-indenyl; 1-, 2-, or 9-fluorenyl; 2-pyridinyl; 1-, 2-, or 3-piperidinyl; 2- or 3-pyrrolyl; 2- or 3-thienyl; 2- or 3-furanyl; 2- or 3-indolyl; 2- or 3-thianaphthenyl; or 2- or 3-benzofuranyl;

$R_1$ is hydrogen, $(C_1—C_8)$alkyl, benzyl, or phenethyl;

X and Y, independently, are hydrogen, fluorine, chlorine, or bromine; and

A is a divalent radical of the formula:

$$—(CH_2)_m\overset{\displaystyle R_2}{\overset{|}{C}}H(CH_2)_n—,$$

wherein $R_2$ is hydrogen, methyl, or ethyl, and m and n, independently, are an integer from 0 to 4, provided that m + n cannot be greater than 4;

$—(CH_2)_p—D—(CH_2)_q—$, wherein D is oxygen or sulfur, p is an integer from 2 to 4, and q is an integer from 0 to 2 provided that p + q cannot be greater than 4; or

$—(CH_2)_rCH=CH(CH_2)_s—$, wherein r is an integer from 1 to 3 and s is an integer from 0 to 2, provided that r + s cannot be greater than 3;

or a non-toxic pharmaceutically acceptable acid addition salt thereof; provided that when each of X and Y in Formula I is hydrogen, R cannot be phenyl.

2. A compound of Formula I as claimed in Claim 1.

3. A compound of Formula II as claimed in Claim 1.

4. A compound of claimed in Claim 3 wherein A is $—CH_2—$.

5. A compound as claimed in any one of Claims 1 to 4 wherein $R_1$ is hydrogen.

6. A compound as claimed in any one of Claims 1 to 4 wherein $R_1$ is methyl or ethyl.

7. A compound as claimed in any one of Claims 1 to 6 wherein X is fluorine and Y is hydrogen.

8. A compound as claimed in any one of Claims 1 to 7 wherein R is 3,4-methylenedioxyphenyl; phenyl; phenyl monosubstituted, disubstituted, or trisubstituted by $(C_1—C_8)$alkyl, $(C_1—C_8)$alkoxy, $(C_1—C_6)$alkylcarbonyloxy, hydroxy, chlorine, bromine, iodine, fluorine, trifluoromethyl, $(C_1—C_6)$alkylcarbonyl, benzoyl, or phenyl; 1-naphthyl or 2-naphthyl.

9. A compound as claimed in Claim 8 wherein R is phenyl, $(C_1—C_8)$alkoxyphenyl, di$(C_1—C_8)$alkoxyphenyl, $(C_1—C_8)$alkylphenyl, hydroxyphenyl, dihydroxyphenyl, chlorophenyl, trifluoromethylphenyl, 1-naphthyl, or 2-naphthyl.

10. A compound as claimed in Claim 9 wherein R is methoxyphenyl, ethoxyphenyl, dimethoxyphenyl, or diethoxyphenyl.

11. A compound selected from:
(*E*)-2-phenyl-3-fluoroallylamine,
(*E*)-2-(2′-methoxyphenyl)-3-fluoroallylamine,
(*E*)-2-(3′-methoxyphenyl)-3-fluoroallylamine,
(*E*)-2-(4′-methoxyphenyl)-3-fluoroallylamine,
(*E*)-2-(3′,4′-dimethoxyphenyl)-3-fluoroallylamine,
(*E*)-*N*-ethyl-2-(3′-methoxyphenyl)-3-fluoroallylamine,
(*E*)-*N*-ethyl-2-(3′,4′-dimethoxyphenyl)-3-fluoroallylamine,
(*E*)-2-(3′-hydroxyphenyl)-3-fluoroallylamine,
(*E*)-2-(3′,4′-dihydroxyphenyl)-3-fluoroallylamine,
(*E*)-2-(3′-trifluoromethylphenyl)-3-fluoroallylamine,
(*E*)-2-(4′-methylphenyl)-3-fluoroallylamine,
(*E*)-2-(4′-chlorophenyl)-3-fluoroallylamine,
(*E*)-2-benzyl-3-fluoroallylamine,
(*E*)-2-(2′-methoxybenzyl)-3-fluoroallylamine,
(*E*)-2-(3′-methoxybenzyl)-3-fluoroallylamine,
(*E*)-2-(4′-methoxybenzyl)-3-fluoroallylamine,
(*E*)-2-(3′,4′-dimethoxybenzyl)-3-fluoroallylamine,
(*E*)-2-(3′-hydroxybenzyl)-3-fluoroallylamine,
(*E*)-2-(3′,4′-dihydroxybenzyl)-3-fluoroallylamine,
(*E*)-2-(2′-naphthyl)-3-fluoroallylamine, and
(*E*)-2-(1′-naphthyl)-3-fluoroallylamine,
or a pharmaceutically acceptable non-toxic acid addition salt thereof.

12. (*E*)-2-(3′,4′-dimethoxyphenyl)-3-fluoroallylamine or a pharmaceutically acceptable non-toxic acid addition salt thereof.

13. A compound as claimed in any one of the preceding claims for use in a method of treatment of the human body by therapy or of diagnosis practiced on the human or animal body.

14. A compound as claimed in any one of the preceding claims for use in the inhibition in the human body of monoamine oxidase enzyme.

15. A compound as claimed in any one of the preceding claims for use in the treatment of the human body for depression.

16. Pharmaceutical compositions comprising a compound as claimed in any one of the preceding claims in admixture or otherwise in association with a pharmaceutically acceptable carrier or diluent therefor.

17. Pharmaceutical compositions as claimed in Claim 15 in unit dosage form containing 5 to 100 mg of said compound per dose.

18. A compound of the formula:

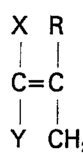
III

or

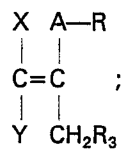
;
IV

wherein X, Y, R, and A are as defined in Claim 1; except that R cannot be mono-, di-, or tri-hydroxyphenyl; and when Y is fluorine, chlorine, or bromine, X cannot be hydrogen; and $R_3$ is hydroxy or a leaving group.

19. A compound as claimed in Claim 18 wherein the leaving group is chlorine, bromine, tosyloxy, or mesyloxy.

20. A compound of Formula III as claimed in any one of Claims 18 and 19.

21. A compound of Formula IV as claimed in any one of Claims 18 and 19.

22. A compound as claimed in Claim 21 wherein A is $-CH_2-$.

23. A compound as claimed in any one of Claims 18 or 20 to 22 wherein X is fluorine; Y is hydrogen and $R_3$ is hydroxy.

24. A compound as claimed in Claim 23 wherein R is methoxyphenyl, dimethoxyphenyl, ethoxyphenyl, or diethoxyphenyl.

25. (*E*)-2-(3′,4′-dimethoxy)phenyl-3-fluoroallyl alcohol.

26. A method of preparing a compound as claimed in Claim 1 wherein $R_1$ is hydrogen which comprises treating a compound of the formula:

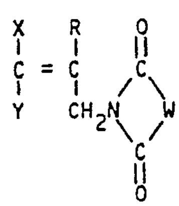

or

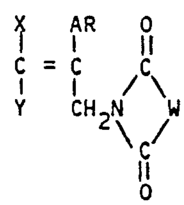

wherein X, Y, R, and A are as defined in Claim 1 and W is

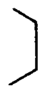 , 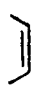 , or 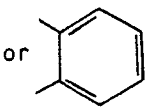

in manner known *per se* to convert the succinimido, maleimido, or phthalimido group to the primary amino group.

27. A method as claim ed in Claim 26 wherein W is

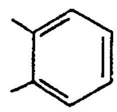

and the treatment comprises reaction with hydrazine or hydrolytic cleavage using a strong mineral acid.

28. A method for preparing a compound as claimed in Claim 1 wherein $R_1$ is hydrogen which comprises treating an alcohol of the formula:

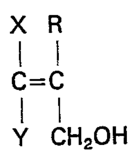

or

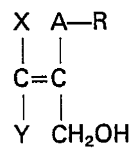

wherein X, Y, and R are as defined in Claim 1, provided that when Y is fluorine, chlorine, or bromine, X cannot be hydrogen, in manner known *per se*, to convert the hydroxy group into the primary amino group.

29. A method as claimed in Claim 28 wherein said alcohol is converted in manner known *per se* into a derivative of the formula

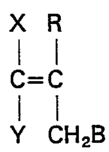

or

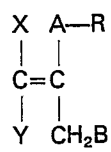

wherein X, Y, and R are as defined in Claim 1, provided that when Y is fluorine, chlorine, or bromine, X cannot be hydrogen, and B is the succinimido, phthalimido, or maleimido, the hexamethylenetetrammonium group, or a $(C_1—C_4)$alkylcarboxyamino group; and said derivative is subsequently treated in manner known *per se* to convert the succinimido, phthalimido, or maleimido group, the hexamethylenetetrammonium group, or a $(C_1—C_4)$alkylcarboxyamino group into a primary amino group.

30. A method as claimed in Claim 29 wherein:

(a) B is the succinimido, phthalimido, or maleimido group and the conversion of said group to amino comprises reaction with hydrazine or hydrolytic cleavage using a strong acid;

(b) B is the hexamethylenetetrammonium group and the conversion of said group to amino comprises treatment by heating with a strong acid; or

(c) B is $(C_1—C_4)$alkylcarboxyamino group and the conversion of said group to amino comprises hydrolysis with a strong mineral acid.

31. A method as claimed in any one of Claims 27 to 30 wherein X is fluorine, Y is hydrogen, R is 3,4-dimethoxyphenyl, and $R_1$ is hydrogen, and the product prepared is (*E*)-2-(3',4'-dimethoxyphenyl)-3-fluoroallylamine.

32. A method for preparing a compound as claimed in Claim 18 wherein $R_3$ is hydroxy which comprises reducing in manner known *per se* a compound of the formula:

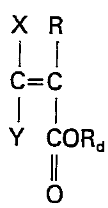

or

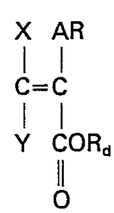

wherein X, Y, R, and A are as defined in Claim 1 and $R_d$ is hydrogen or $(C_1—C_4)$alkyl.

33. A method as claimed in Claim 32 wherein the reduction is carried out using diisobutylaluminum hydride.

34. A method as claimed in any one of Claims 32 and 33 wherein X is fluorine, Y is hydrogen, and R is 3,4-dimethoxyphenyl, and the product prepared is (*E*)-2-(3′,4′-dimethoxyphenyl)-3-fluoroallyl alcohol.

**Claims for the Contracting State: AT**

1. A process for preparing a compound of the formula

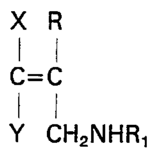 I or 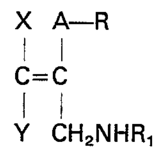 II

wherein:

R is 3,4-methylenedioxyphenyl; phenyl; phenyl monosubstituted, disubstituted, or trisubstituted by ($C_1$—$C_8$)alkyl, ($C_1$—$C_8$)alkoxy, ($C_1$—$C_8$)alkylcarbonyloxy, hydroxy, chlorine, bromine, iodine, fluorine, trifluoromethy, ($C_1$—$C_6$)alkylcarbonyl, benzoyl, or phenyl; 1- or 2-naphthyl; 1-, 2-, or 3-indenyl; 1-, 2-, or 9-fluorenyl; 2-pyridinyl; 1-, 2-, or 3-piperidinyl; 2- or 3-pyrrolyl; 2- or 3-thienyl; 2- or 3-furanyl; 2- or 3-indolyl; 2- or 3-thianaphthenyl; or 2- or 3-benzofuranyl;

$R_1$ is hydrogen, ($C_1$—$C_8$)alkyl, benzyl, or phenethyl;

X and Y, independently, are hydrogen, fluorine, chlorine, or bromine; and

A is a divalent radical of the formula:

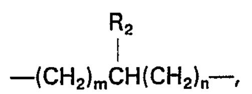

wherein $R_2$ is hydrogen, methyl, or ethyl, and m and n, independently, are an integer from 0 to 4, provided that m + n cannot be greater than 4;

$-(CH_2)_p-D-(CH_2)_q-$, wherein D is oxygen or sulfur, p is an integer from 2 to 4, and q is an integer from 0 to 2 provided that p + q cannot be greater than 4; or

$-(CH_2)_rCH{=}CH(CH_2)_s-$, wherein r is an integer from 1 to 3 and s is an integer from 0 to 2, provided that r + s cannot be greater than 3;

or a non-toxic pharmaceutically acceptable acid addition salt thereof; with the proviso that when each of X and Y in Formula I is hydrogen, R cannot be phenyl,

which comprises treating a compound of the formula

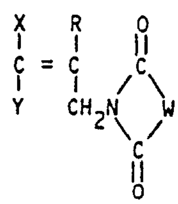 or 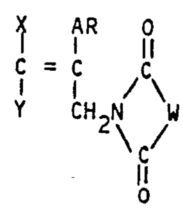

wherein X, Y, R, and A are as defined above and W is

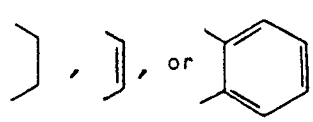

in manner known *per se* to convert the succinimido, maleimido, or phthalimido group to the primary amino group.

2. A process according to claim 1 for preparing a compound wherein $R_1$ is hydrogen, methyl or ethyl and R, X, Y and A are as therein defined.

3. A process as claimed in Claim 1 wherein W is

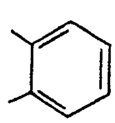

and the treatment comprises reaction with hydrazine or hydrolytic cleavage using a strong mineral acid.

4. A process for preparing a compound as claimed in Claim 1 which comprises treating an alcohol of the formula:

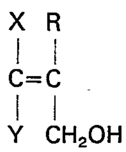

or

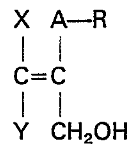

wherein X, Y, and R are as defined in Claim 1, provided that when Y is fluorine, chlorine, or bromine, X cannot be hydrogen, in manner known *per se*, to convert the hydroxy group into the primary amino group.

5. A process as claimed in Claim 4 wherein said alcohol is converted in manner known *per se* into a derivative of the formula

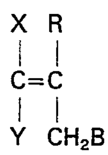

or

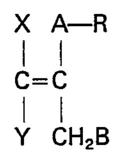

wherein X, Y, and R are as defined in Claim 1, provided that when Y is fluorine, chlorine, or bromine, X cannot be hydrogen, and B is the succinimido, phthalimido, or maleimido, the hexamethylenetetrammonium group, or a $(C_1$—$C_4)$alkylcarboxyamino group; and said derivative is subsequently treated in manner known *per se* to convert the succinimido, phthalimido, or maleimido group, the hexamethylenetetrammonium group, or a $(C_1$—$C_4)$alkylcarboxyamino group into a primary amino group.

6. A process as claimed in Claim 5 wherein:

(a) B is the succinimido, phthalimido, or maleimido group and the conversion of said group to amino comprises reaction with hydrazine or hydrolytic cleavage using a strong acid;

(b) B is the hexamethylenetetrammonium group and the conversion of said group to amino comprises treatment by heating with a strong acid; or

(c) B is $(C_1$—$C_4)$alkylcarboxyamino group and the conversion of said group to amino comprises hydrolysis with a strong mineral acid.

7. A process as claimed in claims 3, 4, 5 or 6 wherein X is fluorine, Y is hydrogen, R is 3,4-dimethoxyphenyl, and $R_1$ is hydrogen, and the product prepared is (*E*)-2-(3′,4′-dimethoxyphenyl)-3-fluoroallylamine.

8. A process for preparing a compound of the formula

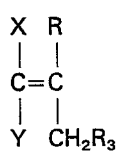 III

or

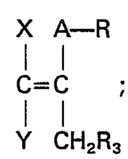 ; IV

wherein X, Y, R, and A are as defined in Claim 1; except that R cannot be mono-, di-, or tri-hydroxyphenyl; and when Y is fluorine, chlorine, or bromine, X cannot be hydrogen; and $R_3$ is hydroxy, which comprises reducing in manner known *per se* a compound of the formula:

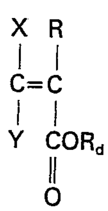

or

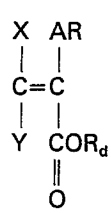

wherein X, Y, R, and A are as defined in Claim 1 and $R_d$ is hydrogen or $(C_1$—$C_4)$alkyl.

9. A process as claimed in Claim 8 wherein the reduction is carried out using diisobutylaluminum hydride.

10. A process as claimed in Claims 8 or 9 wherein X is fluorine, Y is hydrogen, and R is 3,4-dimethoxyphenyl, and the product prepared is (*E*)-2-(3′,4′-dimethoxyphenyl)-3-fluoroallyl alcohol.

**Patentansprüche für die Vertragsstaaten: BE CH DE FR IT LI LU NL SE**

1. Verbindung der Formel:

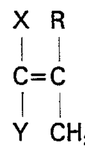 I oder 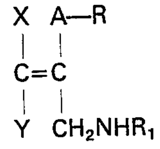 II

dadurch gekennzeichnet, daß:

R 3,4-Methylendioxyphenyl; Phenyl; durch ($C_1$—$C_8$)Alkyl, ($C_1$—$C_8$)Alkoxy, ($C_1$—$C_6$)Alkylcarbonyloxy, Hydroxy, Chlor, Brom, Iod, Fluor, Trifluormethyl, ($C_1$—$C_6$)Alkylcarbonyl, Benzoyl oder Phenyl monosubstituiertes, disubstituiertes oder trisubstituiertes Phenyl; 1-oder 2-Naphthyl; 1-, 2- oder 3-Indenyl; 1-, 2- oder 9-Fluorenyl; 2-Pyridinyl; 1-, 2- oder 3-Piperidinyl; 2-oder 3-Pyrrolyl; 2- oder 3-Thienyl; 2- oder 3-Furanyl; 2- oder 3-Indolyl; 2- oder 3-Thianaphthenyl; oder 2- oder 3-Benzofuranyl bedeutet;

$R_1$ Wasserstoff, ($C_1$—$C_8$)Alkyl, Benzyl oder Phenethyl bedeutet;

X und Y unabhängig voneinander Wasserstoff, Fluor, Chlor oder Brom bedeutet; und

A ein zweiwertiges Radikal der Formel:

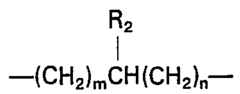

bedeutet, wobei $R_2$ Wasserstoff, Methyl oder Ethyl bedeutet, und m und n unabhängig voneinander eine ganze Zahl zwischen 0 und 4 bedeuten, vorausgesetzt, daß m + n nicht größer als 4 ist;

$—(CH_2)_p—D—(CH_2)_q—$ bedeutet, wobei D Sauerstoff oder Schwefel bedeutet, p eine ganze Zahl zwischen 2 und 4 und q eine ganze Zahl zwischen 0 und 2 bedeuten, vorausgesetzt, daß p + q nicht größer als 4 ist; oder

$—(CH_2)_rCH=CH(CH_2)_s—$ bedeutet, wobei r eine ganze Zahl zweischen 1 und 3 und s eine ganze Zahl zwischen 0 und 2 bedeuten, vorausgesetzt, daß r + s nicht größer als 3 ist;

oder ein nicht-toxisches, pharmazeutisch annehmbares Säureadditionssalz davon; vorausgesetzt, daß wenn sowohl X als auch Y in Formel I Wasserstoff bedeuten, R nicht Phenyl bedeuten kann.

2. Verbindung der Formel I nach Anspruch 1.

3. Verbindung der Formel II nach Anspruch 1.

4. Verbindung nach Anspruch 3, dadurch gekennzeichnet, daß A $—CH_2—$ bedeutet.

5. Verbindung nach einem der Ansprüche 1 bis 4, dadurch gekennzeichnet, daß $R_1$ Wasserstoff bedeutet.

6. Verbindung nach einem der Ansprüche 1 bis 4, dadurch gekennzeichnet, daß $R_1$ Methyl oder Ethyl bedeutet.

7. Verbindung nach einem der Ansprüche 1 bis 6, dadurch gekennzeichnet, daß X Fluor und Y Wasserstoff bedeuten.

8. Verbindung nach einem der Ansprüche 1 bis 7, dadurch gekennzeichnet, daß R 3,4-Methylendioxyphenyl; Phenyl; durch ($C_1$—$C_8$)Alkyl, ($C_1$—$C_8$)Alkoxy, ($C_1$—$C_6$)Alkylcarbonyloxy, Hydroxy, Chlor, Brom, Iod, Fluor, Trifluormethyl, ($C_1$—$C_6$)Alkylcarbonyl, Benzoyl oder Phenyl monosubstituiertes, disubstituiertes oder trisubstituiertes Phenyl; 1-Naphthyl oder 2-Naphthyl bedeutet.

9. Verbindung nach Anspruch 8, dadurch gekennzeichnet, daß R Phenyl, ($C_1$—$C_8$)Alkoxyphenyl, Di($C_1$—$C_8$)alkoxyphenyl, ($C_1$—$C_8$)Alkylphenyl, Hydroxyphenyl, Dihydroxphenyl, Chlorphenyl, Trifluormethylphenyl, 1-Naphthyl oder 2-Naphthyl bedeutet.

10. Verbindung nach Anspruch 9, dadurch gekennzeichnet, daß R Methoxphenyl, Ethoxyphenyl, Dimethoxyphenyl oder Di-ethoxyphenyl bedeutet.

11. Verbindung ausgewählt von:
(E)-2-Phenyl-3-fluoroallylamin,
(E)-2-(2'-Methoxyphenyl)-3-fluorallylamin,
(E)-2-(3'-Methoxyphenyl)-3-fluorallylamin,
(E)-2-(4'-Methoxyphenyl)-3-fluorallylamin,
(E)-2-(3',4'-Dimethoxyphenyl)-3-fluorallylamin,
(E)-N-Ethyl-2-(3'-methoxyphenyl)-3-fluorallylamin,
(E)-N-Ethyl-2-(3',4'-dimethoxyphenyl)-3-fluorallylamin,
(E)-2-(3'-Hydroxyphenyl)-3-fluorallylamin,
(E)-2-(3',4'-Dihydroxyphenyl)-3-fluorallylamin,
(E)-2-(3'-Trifluormethylphenyl)-3-fluorallylamin,
(E)-2-(4'-Methylphenyl)-3-fluorallylamin,
(E)-2-(4'-Chlorphenyl)-3-fluorallylamin,
(E)-2-Benzyl-3-fluorallylamin,

(E)-2-(2'-Methoxybenzyl)-3-fluorallylamin,
(E)-2-(Methoxybenzyl)-3-fluorallylamin,
(E)-2-(4'-Methoxybenzyl)-3-fluorallylamin,
(E)-2-(3',4'-Dimethoxybenzyl)-3-fluorallylamin,
(E)-2-(3'-Hydroxybenzyl)-3-fluorallylamin,
(E)-2-((3',4'-Dihydroxybenzyl)-3-fluorallylamin,
(E)-2-(2'-Naphthyl)-3-fluorallylamin, und
(E)-2-(1'-Naphthyl)-3-fluorallylamin,
oder ein pharmazeutisch annehmbares nicht-toxisches Säureadditionssalz davon.

12. (E)-2-(3',4'-Dimethoxyphenyl)-3-fluorallylamin oder ein pharmazeutisch annehmbares nicht-toxisches Säureadditionssalz davon.

13. Verbindung nach einem der vorangehenden Ansprüche zur Verwendung bei einem Verfahren zur Behandlung des menschlichen Körpers durch Therapie oder Diagnose, das am menschlichen oder tierischen Körper durchgeführt wird.

14. Verbindung nach einem der vorngehenden Ansprüche zur Verwendung bei der Inhibierung des Enzyms Monoamin-Oxidase im menschlichen Körper.

15. Verbinung nach einem der vorangehenden Ansprüche zur Verwendung bei der Behandlung des menschlichen Körpers bei Depression.

16. Pharmaceutische Zusammensetzungen, die eine Verbindung nach einem der vorangehenden Ansprüche in Mischung oder in anderer Weise in Assoziation mit einem pharmazeutisch annehmbaren Träger oder Verdünnungsmittel für diese enthalten.

17. Pharmazeutische Zusammensetzungen nach Anspruch 15 in Einheitsdosierungsform, welche 5 bis 100 mg der besagten Verbindung pro Dosis enthalten.

18. Verbindung der Formel:

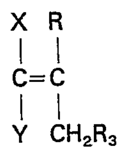 III oder 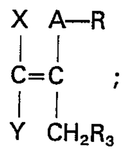 ; IV

dadurch gekennzeichnet, daß X, Y, R und A wie in Anspruch 1 definiert sind, außer daß R nicht Mono-, Di- oder Trihydroxyphenyl bedeuten kann; und daß, wenn Y Fluor, Chlor oder Brom bedeutet, X nicht Wasserstoff bedeuten kann und $R_3$ eine Hydroxy- oder eine Fluchgruppe bedeutet.

19. Verbindung nach Anspruch 18, dadurch gekennzeichnet, daß die Fluchtgruppe Chlor, Brom, Tosyloxy oder Mesyloxy ist.

20. Verbindung der Formel III nach einem der Ansprüche 18 und 19.

21. Verbindung der Formel IV nach einem der Ansprüche 18 und 19.

22. Verbindung nach Anspruch 21, dadurch gekennzeichnet, daß A $-CH_2-$ bedeutet.

23. Verbindung nach einem der Ansprüche 18 oder 20 bis 22, dadurch gekennzeichnet, daß X Fluor; Y Wasserstoff und $R_3$ Hydroxy bedeuten.

24. Verbindung nach Anspruch 23, dadurch gekennzeichnet, daß R Methoxyphenyl, Dimethoxyphenyl, Ethoxyphenyl oder Diethoxyphenyl bedeutet.

25. (E)-2-(3',4'-Dimethoxy)phenyl-3-fluorallylalkohol.

26. Verfahren zur Herstellung einer Verbindung nach Anspruch 1, dadurch gekennzeichnet, daß $R_1$ Wasserstoff bedeutet, welches die Behandlung einer Verbindung der Formel:

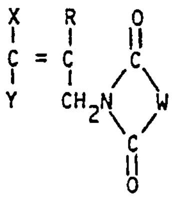 oder 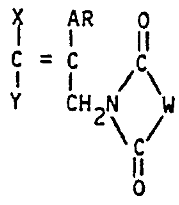

wobei X, Y, R und A wie in Anspruch 1 definiert sind und W

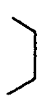 , 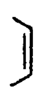 , oder 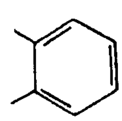

bedeutet, in an sich bekannter Weise beinhaltet, um die Succinimido-, Maleimido- oder Phthalimido-Gruppe in die primäre Aminogruppe umzusandeln.

27. Verfahren nach Anspruch 26, dadurch gekennzeichnet, daß W

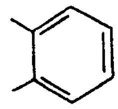

bedeutet, und die Behandlung die Reaktion mit Hydrazin oder hydrolytische Spaltung unter Verwendung einer starken Mineralsäure beinhaltet.

28. Verfahren zur Herstellung eine Verbindung nach Anspruch 1, dadurch gekennzeichnet, daß $R_1$ Wasserstoff bedeutet, welches die Behandlung eines Alkohols der Formel

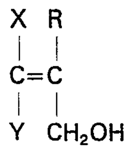 oder 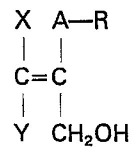

wobei, X, Y und R wie in Anspruch 1 definiert sind, vorausgesetzt, daß, wenn Y Fluor, Chlor oder Brom bedeutet, X nicht Wasserstoff bedeuten kann, in an sich bekannter Weise beinhaltet, um die Hydroxygruppe in die primäre Aminogruppe umzuwandeln.

29. Verfahren nach Anspruch 28, dadurch gekennzeichnet, daß der besagte Alkohol in an sich bekannter Weise in ein Derivat der Formel:

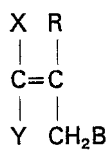 oder 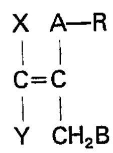

übergeführt wird, wobei X, Y und R wie in Anspruch 1 definiert sind, vorausgesetzt, daß, wenn Y Fluor, Chlor oder Brom bedeutet, X nicht Wasserstoff bedeuten kann, und B die Succinimido-, Phthalimido- oder Maleimido-, die Hexamethylentetrammoniumgruppe oder eine $(C_1—C_4)$Alkylcarboxyaminogruppe bedeutet; und daß das besagte Derivat anschließend in an sich bekannter Weise behandelt wird, um die Succinimido-, Phthalimido-, oder Maleimido-Gruppe, die Hexamethylentetrammoniumgruppe oder eine $(C_1—C_4)$Alkylcarboxyaminogruppe in eine primäre Aminogruppe umzuwandeln.

30. Verfahren nach Anspruch 29, dadurch gekennzeichnet, daß:

(a) B die Succinimido-, Phthalimido- oder Maleimido-Gruppe bedeutet, und die Umwandlung der besagten Gruppen in die Aminogruppe die Reaktion mit Hydrazin oder hydrolytische Spaltung under Verwendung einer starken Säure beinhaltet;

(b) B die Hexamethylentetrammoniumgruppe bedeutet, und die Umwandlung der besagten Gruppe in die Aminogruppe die Behandlung durch Erhitzen mit einer starken Säure beinhaltet; oder

c) B eine $(C_1—C_4)$Alkylcarboxyaminogruppe bedeutet, und die Umwandlung der besagten Gruppe in die Aminogruppe Hydrolyse mit einer starken Mineralsäure beinhaltet.

31. Verfahren nach einem der Ansprüche 27 bis 30, dadurch gekennzeichnet, daß X Fluor, Y Wasserstoff, R 3,4-Dimethoxyphenyl und $R_1$ Wasserstoff bedeuten, und das Produkt, das hergestellt wird, (E)-2-(3',4'-Dimethoxyphenyl)-3-fluorallylamin ist.

32. Verfahren zur Herstellung einer Verbindung nach Anspruch 18, dadurch gekennzeichnet, daß $R_3$ Hydroxy bedeutet, welches die Reduktion in an sich bekannter Weise oder Verbindung der Formel:

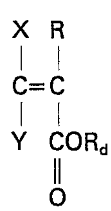 oder 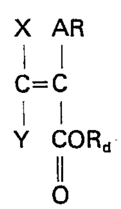

beinhaltet, wobei X, Y, R und A wie in Anspruch 1 definiert sind und $R_d$ Wasserstoff oder $(C_1—C_4)$Alkyl bedeutet.

33. Verfahren nach Anspruch 32, dadurch gekennzeichnet, daß die Reduktion unter Verwendung von Diisobutylaluminiumhydrid durchgeführt wird.

34. Verfahren nach einem der Ansprüche 32 und 33, dadurch gekennzeichnet, daß X Fluor, Y Wasserstoff und R 3,4-Dimethoxyphenyl bedeuten, und das hergestellte Produkt (E)-2-(3',4'-dimethoxyphenyl)-3-fluorallylalkohol ist.

**Patentansprüche für den Vertragsstaat: AT**

1. Verfahren zur Herstellung einer Verbindung der Formel

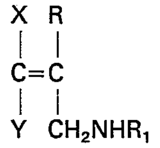 I oder 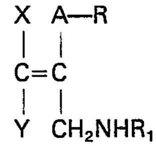 II

dadurch gekennzeichnet, daß:

R 3,4-Methylendioxyphenyl; Phenyl; durch ($C_1$—$C_8$)Alkyl, ($C_1$—$C_8$)Alkoxy, ($C_1$—$C_6$)Alkylcarbonyloxy, Hydroxy, Chlor, Brom, Iod, Fluor, Trifluormethyl, ($C_1$—$C_6$)Alkylcarbonyl, Benzoyl oder Phenyl monosubstituiertes, disubstituiertes oder trisubstituiertes Phenyl; 1-oder 2-Naphthyl; 1-, 2- oder 3-Indenyl; 1-, 2- oder 9-Fluorenyl; 2-Pyridinyl; 1-, 2- oder 3-Piperidinyl; 2-oder 3-Pyrrolyl; 2- oder 3-Thienyl; 2- oder 3-Furanyl; 2- oder 3-Indolyl; 2- oder 3-Thianaphthenyl; oder 2- oder 3-Benzofuranyl bedeutet;

$R_1$ Wasserstoff, ($C_1$—$C_8$)Alkyl, Benzyl oder Phenethyl bedeutet;

X und Y unabhängig voneinander Wasserstoff, Fluor, Chlor oder Brom bedeuten; und

A ein zweiwertiges Radikal der Formel:

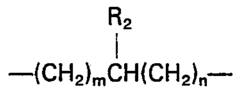

bedeutet, wobei $R_2$ Wasserstoff, Methyl oder Ethyl bedeutet, und m und n unabhängig eine ganze Zahl zwischen 0 und 4 bedeuten, vorausgesetzt, daß m + n nicht größer als 4 sein kann;

$—(CH_2)_p—D—(CH_2)_q—$ bedeutet, wobei D Sauerstoff oder Schwefel bedeutet, p eine ganze Zahl zwischen 2 und 4 und q eine ganze Zahl zwischen 0 und 2 bedeuten, vorausgesetzt, daß p + q nicht größer als 4 sein kann; oder

$—(CH_2)_rCH=CH(CH_2)_s—$ bedeutet, wobei r eine ganze Zahl zwischen 1 und 3 und s eine ganze Zahl zwischen 0 und 2 bedeuten, vorausgesetzt, daß r + s nicht größer sein kann als 3;

oder ein nicht-toxisches, pharmazeutisch annehmbares Säureadditionssalz davon; unter der Voraussetzung, daß, wenn sowohl X als auch Y Wasserstoff bedeutet, R nicht Phenyl bedeuten kann, welches die Behandlung einer Verbindung der Formel

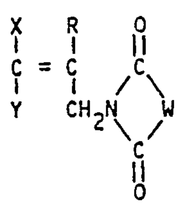 oder 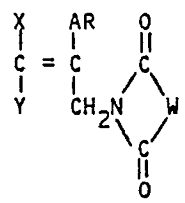

wobei X, Y, R und A wie oben definiert sind und W

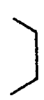, 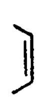, oder 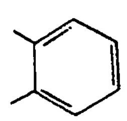

bedeutet, in an sich bekannter Weise beinhaltet, um die Succinimido-, Maleimido- oder Phthalimidogruppe in die primäre Aminogruppe umzuwandeln.

2. Verfahren nach Anspruch 1 zur Herstellung einer Verbindung, dadurch gekennzeichnet, daß $R_1$ Wasserstoff, Methyl oder Ethyl bedeutet, und R, X, Y und A wie in Anspruch 1 definiert sind.

3. Verfahren nach Anspruch 1, dadurch gekennzeichnet, daß W

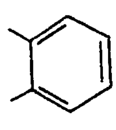

bedeutet, und die Behandlung die Reaktion mit Hydrazin oder hydroxyltische Spaltung unter Verwendung einer starken Mineralsäure beinhaltet.

4. Verfahren zur Herstellung einer Verbindung nach Anspruch 1, welches die Behandlung eines Alkohols der Formel:

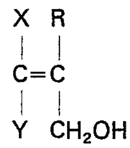

oder

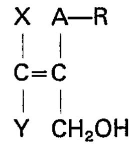

wobei, X, Y und R wie in Anspruch 1 definiert sind, vorausgesetzt, daß, wenn Y Fluor, Chlor oder Brom bedeutet, X nicht Wasserstoff bedeuten kann, in an sich bekannter Weise beinhaltet, um die Hydroxygruppe in die primäre Aminogruppe umzuwandeln.

5. Verfahren nach Anspruch 4, dadurch gekennzeichnet, daß der besagte Alkohol in an sich bekannter Weise in ein Derivat der Formel:

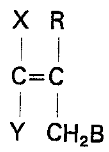

oder

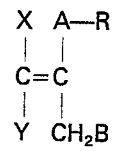

umgewandelt wird, wobei X, Y und R wie in Anspruch 1 definiert sind, vorausgesetzt, daß, wenn Y Fluor, Chlor oder Brom bedeutet, X nicht Wasserstoff bedeuten kann, und B die Succinimido-, Phthalimido- oder Maleimidogruppe, die Hexamethylentetrammoniumgruppe oder eine ($C_1$—$C_4$)Alkylcarboxyaminogruppe bedeutet; und das besagte Derivat anschließend in an sich bekannter Weise behandelt wird, um die Succinimido-, Phthalimido-, oder Maleimidogruppe, die Hexamethylentetrammoniumgruppe oder eine ($C_1$—$C_4$)Alkylcarboxyaminogruppe in eine primäre Aminogruppe umzuwandeln.

6. Verfahren nach Anspruch 5, dadurch gekennzeichnet, daß:

(a) B die Succinimido-, Phthalimido- oder Maleimido-Gruppe bedeutet, und die Umwandlung der besagten Gruppe in die Aminogruppe die Reaktion·mit Hydrazin oder hydrolytische Spaltung unter Verwendung einer starken Säure beinhaltet;

(b) B die Hexamethylentetrammoniumgruppe bedeutet, und die Umwandlung der besagten Gruppe in die Aminogruppe die Behandlung durch Erhitzen mit einer starken Säure beinhaltet, oder

(c) B eine ($C_1$—$C_4$)Alkylcarboxyaminogruppe bedeutet, und die Umwandlung der besagten Gruppe in die Aminogruppe Hydrolyse mit einer starken Mineralsäure beinhaltet.

7. Verfahren nach den Ansprüchen 3, 4, 5 oder 6, dadurch gekennzeichnet, daß X Fluor, Y Wasserstoff, R 3,4-Dimethoxyphenyl und $R_1$ Wasserstoff bedeuten, und das hergestellte Produkt (E)-2-(3′,4′-Dimethoxyphenyl)-3-fluorallylamin ist.

8. Verfahren zur Herstellung einer Verbindung der Formel

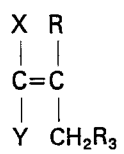 III

oder

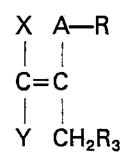 IV

dadurch gekennzeichnet, daß X, Y, R und A wie in Anspruch 1 definiert sind; außer, daß r nicht Mono-, Di- oder Trihydroxyphenyl bedeuten kann; und wenn Y Fluor, Chlor oder Brom bedeutet, X nicht Wasserstoff bedeuten kann; und $R_3$ Hydroxy bedeutet, welches die Reduktion in an sich bekannter Weise einer Verbindung der Formel

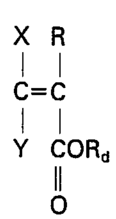

oder

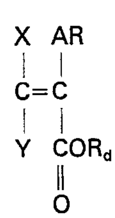

wobei X, Y, R und A wie in Anspruch 1 definiert sind und $R_d$ Wasserstoff oder ($C_1$—$C_4$)Alkyl bedeutet.

9. Verfahren nach Anspruch 8, dadurch gekennzeichnet, daß die Reduktion unter Verwendung von Diisobutylaluminiumhydrid durchgeführt wird.

10. Verfahren nach Anspruch 8 oder 9, dadurch gekennzeichnet, daß X Fluor, Y Wasserstoff und R 3,4-Dimethoxyphenyl bedeuten, und das hergestellte Produkt (E)-2-(3′,4′-Dimethoxyphenyl)-3-fluorallylalkohol ist.

**Revendications pour les Etats contractants: BE CH DE FR IT LI LU NL SE**

1. Un composé de formule:

$$\begin{array}{ll} X & R \\ | & | \\ C= & C \\ | & | \\ Y & CH_2NHR_1 \end{array} \quad I \qquad \text{ou} \qquad \begin{array}{ll} X & A—R \\ | & | \\ C= & C \\ | & | \\ Y & CH_2NHR_1 \end{array} \quad II$$

où:

R est un 3,4-méthylènedioxyphényle; un phényle; un phényle monosubstitué, disubstitué ou trisubstitué par un alkyle ($C_1$—$C_8$), un alcoxy ($C_1$—$C_8$), un alkyl($C_1$—$C_6$)carbonyloxy, un hydroxy, un chlore, un brome, un iode, un fluor, un trifluorométhyle, un alkyl($C_1$—$C_6$)carbonyle, un benzoyle ou un phényle; un 1- ou 2-naphtyle; un 1-, 2- ou 3-indényle; un 1-, 2- ou 9-fluorényle; un 2-pyridyle, un 1-, 2- ou 3-pipéridyle; un 2- ou 3-pyrrolyle; un 2- ou 3-thiényle; un 2- ou 3-furyle; un 2- ou 3-indolyle; un 2- ou 3-thianaphtényle; ou un 2- ou 3-benzofuryle;

$R_1$ est un hydrogène, un alkyle ($C_1$—$C_8$), un benzyle ou un phénéthyle; X et Y indépendamment sont un hydrogène, un fluor, un chlore ou un brome; et

A est un radical divalent de formule:

$$\begin{array}{c} R_2 \\ | \\ —(CH_2)_mCH(CH_2)_n— \end{array}$$

dans laquelle $R_2$ est un hydrogène, un méthyle ou un éthyle et m et n indépendamment sont un entier de 0 à 4 sous réserve que m + n ne peut pas être supérieur à 4;

$—(CH_2)_p—D—(CH_2)_q—$ dans laquelle D est un oxygène ou un soufre, p est un entier de 2 à 4 et q est un entier de 0 à 2, sour réserve que p + q ne peut pas être supérieur à 4; ou

$—(CH_2)_rCH{=}CH(CH_2)_s—$ dans laquelle r est un entier de 1 à 3 et s est un entier de 0 à 2, sous réserve que r + s ne peut pas être supérieur à 3;

ou un sel d'addition d'acide non toxique convenant en pharmacie correspondant; sous réserve que, lorsque chacun de X et Y dans la formule I est un hydrogène, R ne peut pas être un phényle.

2. Un composé de formule I comme revendiqué dans la revendication 1.

3. Un composé de formule II comme revendiqué dans la revendication 1.

4. Un composé comme revendiqué dans la revendication 3 où A est $—CH_2—$.

5. Un composé comme revendiqué dans l'une quelconque des revendications 1 à 4 où $R_1$ est un hydrogène.

6. Un composé comme revendiqué dans l'une quelconque des revendications 1 à 4 où $R_1$ est un méthyle ou un éthyle.

7. Un composé comme revendiqué dans l'une quelconque des revendications 1 à 6 où X est un fluor et Y est un hydrogène.

8. Un composé comme revendique dans l'une quelconque des revendications 1 à 7 où R est un 3,4-méthylènedioxyphényle; un phényle; un phényle monosubstitué, disubstitué ou trisubstitué par un alkyle ($C_1$—$C_8$), un alcoxy ($C_1$—$C_8$), un alkyl($C_1$—$C_6$)carbonyloxy, un hydroxy, un chlore, un brome, un iode, un fluor, un trifluorométhyle, un alkyl($C_1$—$C_6$)carbonyle, un benzoyle ou un phényle; un 1-naphtyle ou un 2-naphtyle.

9. Un composé comme revendiqué dans la revendication 8 où R est un phényle, un alcoxy($C_1$—$C_8$)phényle, un dialcoxy($C_1$—$C_8$)phényle, un alkyle($C_1$—$C_8$)phényle, un hydroxyphényle, un dihydroxyphényle, un chlorophényle, un trifluorométhylphényle, un 1-naphtyle ou un 2-naphtyle.

10. Un composé comme revendiqué dans la revendication 9 dans lequel R est un méthoxyphényle, un éthoxyphényle, un diméthoxyphényle ou un diéthoxyphényle.

11. Un composé choisi parmi:

la (E)-2-phényle-3-fluroro-allylamine,
la (E)-2-(2'-méthoxyphényle)-3-fluoro-allylamine,
la (E)-2-(3'-méthoxyphényle)-3-fluoro-allylamine,
la (E)-2-(4'-méthoxyphényl)-3-fluoro-allylamine,
la (E)-2-(3',4'-diméthoxyphényle)-3-fluoro-allylamine,
la (E)-N-éthyle-2-(3'-méthoxyphényl)-3-fluoro-allylamine,
la (E)-N-éthyl-2-(3',4'-diméthoxyphényl)-3-fluoro-allylamine,
la (E)-2-(3'-hydroxyphényl)-3-fluoro-allylamine,
la (E)-2-(3',4'-dihydroxyphényl)-3-fluoro-allylamine,
la (E)-2-(3'-trifluorométhylphényl)-3-fluoro-allylamine,
la (E)-2-(4'-méthylphényl)-3-fluoro-allylamine,

la (E)-2-(4'-chlorophényl)-3-fluoro-allylamine,
la (E)-2-benzyl-3-fluoro-allylamine,
la (E)-2-(2'-méthoxybenzyl)-3-fluoro-allylamine,
la (E)-2-(3'-méthoxybenzyl)-3-fluoro-allylamine,
la (E)-2-(4'-méthoxybenzyl)-3-fluoro-allylamine,
la (E)-2-(3',4'-diméthoxybenzyl)-3-fluoro-allylamine,
la (E)-2-(3'-hydroxybenzyl)-3-fluoro-allylamine,
la (E)-2-(3',4'-dihydroxybenzyl)-3-fluoro-allylamine,
la (E)-2-(2'-naphtyl)-3-fluoro-allylamine, et
la (E)-2-(1'-naphtyl)-3-fluoro-allylamine,

ou un sel d'addition d'acide non toxique convenant en pharmacie de ceux-ci.

12. (E)-2-(3',4'-diméthoxyphényle)-3-fluoro-allylamine ou un sel d'addition d'acide non toxique convenant en pharmacie de celle-ci.

13. Un composé comme revendiqué dans l'une quelconque des revendications précédentes pour l'emploi dans un procédé de traitement de l'organisme humain par thérapie ou de diagnostic pratiqué sur l'organisme d'un être humain ou d'un animal.

14. Un composé comme revendiqué dans l'une quelconque des revendications précédentes pour l'emploi dans l'inhibition dans l'organisme humain de l'enzyme mono-amine-oxydase.

15. Un composé comme revendiqué dans l'une quelconque des revendications précédentes pour l'emploi dans le traitement de la dépression dans l'organisme humain.

16. Compositions pharmaceutiques comprenant un composé comme revendiqué dans l'une quelconque des revendications précédentes mélangé ou associé d'autre façon à un support ou diluant convenant en pharmacie de celui-ci.

17. Compositions pharmaceutiques comme revendiqué dans la revendication 15 sous forme d'une dose unitaire d'administration contenant 5 à 100 dudit composé par dose.

18. Un composé de formule:

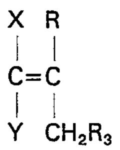 III ou 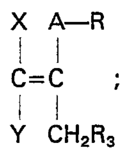 ; IV

où X, Y, R et A sont comme défini dans la revendication 1; si ce n'est que R ne peut pas être un mono-, di- ou tri-hydroxyphényle; et lorsque Y est un fluor, un chlore ou un brome, X ne peut pas être un hydrogène; et $R_3$ est un hydroxy ou un groupe labile.

19. Un composé comme revendique dans la revendication 18 dans lequel le groupe labile est chlore, brome, tosyloxy ou mésyloxy.

20. Un composé de formule III comme revendiqué dans l'une quelconque des revendications 18 et 19.

21. Un composé de formule IV comme revendiqué dans l'une quelconque des revendications 18 et 19.

22, Un composé comme revendiqué dans la revendication 21 où A est $-CH_2-$.

23. Un composé comme revendiqué dans l'lune quelconque des revendications 18 ou 20 à 22 où X est un fluor, Y est un hydrogène et $R_3$ est un hydroxy.

24. Un composé comme revendiqué dans la revendication 23 dans lequel R est un méthoxyphényle, un diméthoxyphényle, un éthoxyphényle ou un diéthoxyphényle.

25. Alcool (E)-2-(3',4'-diméthoxy)phényl-3-fluoro-allylique.

26. Un procédé de préparation d'un composé comme revendiqué dans la revendication 1 où $R_1$ est un hydrogène, qui comprend le traitement d'un composé de formule:

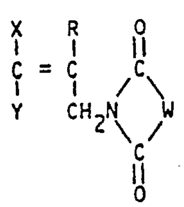 ou 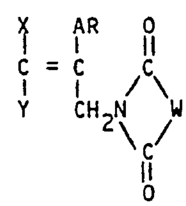

où X, Y, R et A sont comme défini dans la revendication 1 et W est

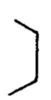 , 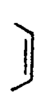 , ou 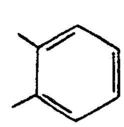

de façon connue en soi pour transformer le groupe succinimido, maléimido ou phtalimido en le groupe amino primaire.

27. Un procédé comme revendiqué dans la revendication 26 où W est

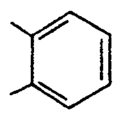

et le traitement comprend la réaction avec l'hydrazine ou un clivage hydrolytique avec un acide minéral fort.

28. Un procédé pour préparer un composé comme revendiqué dans la revendication 1 où $R_1$ est un hydrogène, qui comprend le traitement d'un alcool de formule:

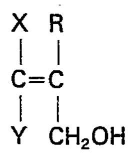

ou

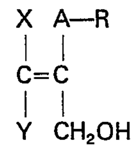

où X, Y, et R sont comme défini dans la revendication 1, sous réserve que lorsque Y est un fluor, un chlore ou un brome, X ne peut pas être un hydrogène, de façon connue en soi, pour transformer le groupe hydroxy en le groupe amino primaire.

29. Un procédé comme revendiqué dans la revendication 28 où ledit alcool est transformé de façon connue en soi en un dérivé de formule:

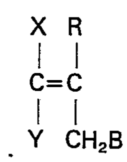

ou

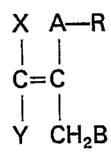

où X, Y et R sont comme défini dans la revendication 1, sous réserve que lorsque Y est un fluor, un chlore ou un brome, X ne peut pas être un hydrogène et B est un groupe succinimido, phtalimido ou maléimido, un groupe hexaméthylènetétrammonium ou un groupe alkyl($C_1$—$C_4$)carboxyamino; et ledit dérivé est ensuite traité de façon connue en soi pour transformer le groupe succinimido, phtalimido ou maléimido, le groupe hexaméthylènetétrammonium ou un groupe alkyl($C_1$—$C_4$)carboxyamino en un groupe amino primaire.

30. Un procédé comme revendiqué dans la revendication 29 où:

(a) B est le groupe succinimido, phtalimido ou maléimido et la conversion dudit groupe en un amino comprend la réaction avec l'hydrazine ou un clivage hydrolytique avec un acide fort;

(b) B est le groupe hexaméthylènetétrammonium et la conversion dudit groupe en un amino comprend le traitement par chauffage avec un acide fort; ou

(c) B est un groupe alkyl($C_1$—$C_4$)carboxyamino et la conversion dudit groupe en un amino comprend une hydrolyse avec un acide minéral fort.

31. Un procédé comme revendiqué dans l'une quelconque des revendications 27 à 30 où X est un fluor, Y est un hydrogène, R est un 3,4-diméthoxyphényle et $R_1$ est un hydrogène et le produit préparé est la (E)-2-(3',4'-diméthoxyphényl)-3-fluoro-allylamine.

32. Un procédé pour préparer un composé comme revendiqué dans la revendication 18 où $R_3$ est un hydroxy, qui comprend la réduction de façon connue en soi d'un composé de formule:

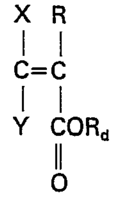

ou

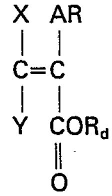

où X, Y, R et A sont comme défini dans la revendication 1 et $R_d$ est un hydrogène ou un alkyle ($C_1$—$C_4$).

33. Un procédé comme revendiqué dans la revendication 32 où la réduction est effectuée par emploi d'hydrure de diisobutylaluminium.

34. Un procédé comme revendiqué dans l'une quelconque des revendications 32 et 33 où X est un fluor, Y est un hydrogène et R est un 3,4-diméthoxyphényle et le produit préparé est l'alcool (E)-2-(3',4'-diméthoxyphényl)-3-fluoro-allylique.

**Revendications pour l'Etat contractant: AT**

1. Un procédé pour la préparation d'un composé de formule

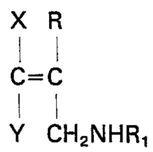 I ou 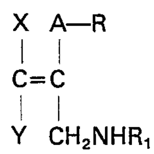 II

où

R est un 3,4-méthylènedioxyphényle; un phényle; un phényle monosubstitué, disubstitué ou trisubstitué par un alkyle ($C_1$—$C_8$), un alcoxy ($C_1$—$C_8$), un alkyl($C_1$—$C_6$)carbonyloxy, un hydroxy, un chlore, un brome, un iode, un fluor, un trifluorométhyle, un alkyl($C_1$—$C_6$)carbonyle, un benzoyle ou un phényle; un 1- ou un 2-naphtyle; un 1-, 2- ou 3-indényle; un 1-, 2- ou 9-fluorényle; un 2-pyridyle, un 1-, 2- ou 3-pipéridyle; un 2- ou 3-pyrrolyle; un 2- ou 3-thiényle; un 2- ou 3-furyle; un 2- ou 3-indolyle; un 2- ou 3-thianaphtényle; ou un 2- ou 3-benzofuryle;

$R_1$ est un hydrogène, un alkyle ($C_1$—$C_8$), un benzyle ou un phénéthyle; X et Y indépendamment sont un hydrogène, un fluor, un chlore ou un brome; et

A est un radical divalent de formule:

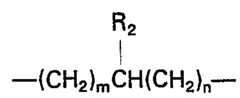

dans laquelle $R_2$ est un hydrogène, un méthyle ou un éthyle et m et n indépendamment sont un entier de 0 à 4 sous réserve que m + n ne peut pas être supérieur à 4;

$-(CH_2)_p-D-(CH_2)_q-$ où D est un oxygène ou un soufre, p est un entier de 2 à 4 et q est un entier de 0 à 2, sous réserve que p + q ne peut pas être supérieur à 4; ou $-(CH_2)_rCH=CH(CH_2)_s-$ où r est un entier de 1 à 3 et s est un entier de 0 à 2, sous réserve que r + s ne peut pas être supérieur à 3; ou un sel d'addition d'acide non toxique convenant en pharmacie correspondant; sous réserve que, lorsque chacun de X et Y est un hydrogène, R en peut pas être un phényle, qui comprend le traitement d'un composé de formule

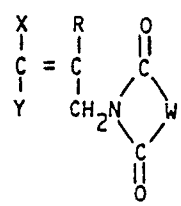 ou 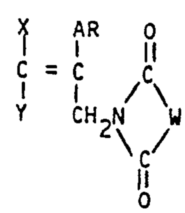

où X, Y, R est A sont comme défini ci-dessus et W est

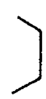, 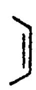, ou 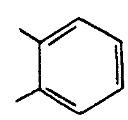

de façon connue en soi pour transformer le groupe succinimido, maléimido ou phtalimido en la groupe amino primaire.

2. Un procédé selon la revendication 1 pour la préparation d'un composé où $R_1$ est un hydrogène, un méthyle ou un éthyle et R, X, Y et A sont comme défini.

3. Un procédé comme revendiqué dans la revendication 1 où W est

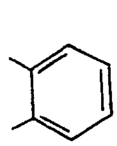

et le traitement comprend une réaction avec l'hydrazine ou un clivage hydrolytique avec un acide minéral fort.

4. Un procédé pour préparer un composé comme revendiqué dans la revendication 1 qui comprend le traitement d'un alcool de formule:

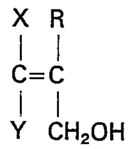 ou 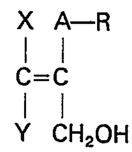

où X, Y, et R sont comme défini dans la revendication 1, sous réserve que lorsque Y est un fluor, un chlore ou un brome, X ne peut pas être un hydrogène, de façon connue en soi, pour transformer le groupe hydroxy en le groupe amino primaire.

5. Un procédé comme revendiqué dans la revendication 4 où ledit alcool est transformé de façon connue en soi en un dérivé de formule:

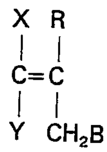 ou 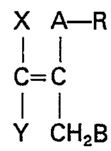

où X, Y et R sont comme défini dans la revendication 1, sous réserve que lorsque Y est un fluor, un chlore ou un brome, X ne peut pas être un hydrogène et B est un groupe succinimido, phtalimido ou maléimido, un groupe hexaméthylènetétrammonium ou un groupe alkyl($C_1$—$C_4$)carboxyamino; et ledit dérivé est ensuite traité de façon connue en soi pour transformer le groupe succinimido, phtalimido ou maléimido, le groupe hexaméthylènetétrammonium ou un groupe alkyl($C_1$—$C_4$)carboxyamino en un groupe amino primaire.

6. Un procédé comme revendiqué dans la revendication 5 où:

(a) B est le groupe succinimido, phtalimido ou maléimido et la conversion dudit groupe en un amino comprend la réaction avec l'hydrazine ou un clivage hydrolytique utilisant un acide fort;

(b) B est le groupe hexaméthylènetétrammonium et la conversion dudit groupe en un amino comprend le traitement par chauffage avec un acide fort; ou

(c) B est un groupe alkyl($C_1$—$C_4$)carboxyamino et la conversion dudit groupe en un amino comprend une hydrolyse avec un acide minéral fort.

7. Un procédé comme revendiqué dans les revendications 3, 4, 5 ou 6 où X est un flour, Y est un hydrogène, R est un 3,4-diméthoxyphényle et $R_1$ est un hydrogène et le produit préparé est l'(E)-2-(3',4'-diméthoxyphényl)-3-fluoro-allylamine.

8. Un procédé pour la préparation d'un composé de formule

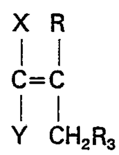 III ou 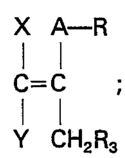 ; IV

où X, Y, R et A sont comme défini dans la revendication 1; si ce n'est que R ne peut pas être un mono-, di- ou tri-hydroxyphényle; et lorsque Y est un fluor, un chlore ou un brome, X ne peut pas être un hydrogène; et $R_3$ est un hydroxy, qui comprend la réduction de façon connue en soi d'un composé de formule:

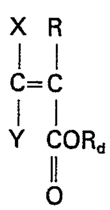 ou 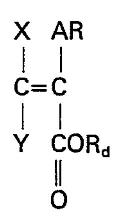

où X, Y, R et A sont comme défini dans la revendication 1 et $R_d$ est un hydrogène ou un alkyle ($C_1$—$C_4$).

9. Un procédé comme revendiqué dans la revendication 8 où la réduction est effectué par emploi d'hydrure de diisobutylaluminium.

10. Un procédé comme revendiqué dans les revendications 8 ou 9 où X est un fluor, Y est un hydrogène et R est un 3,4-diméthoxyphényle et le produit préparé est l'alcool (E)-2-(3',4'-diméthoxyphényl)-3-fluoro-allylique.

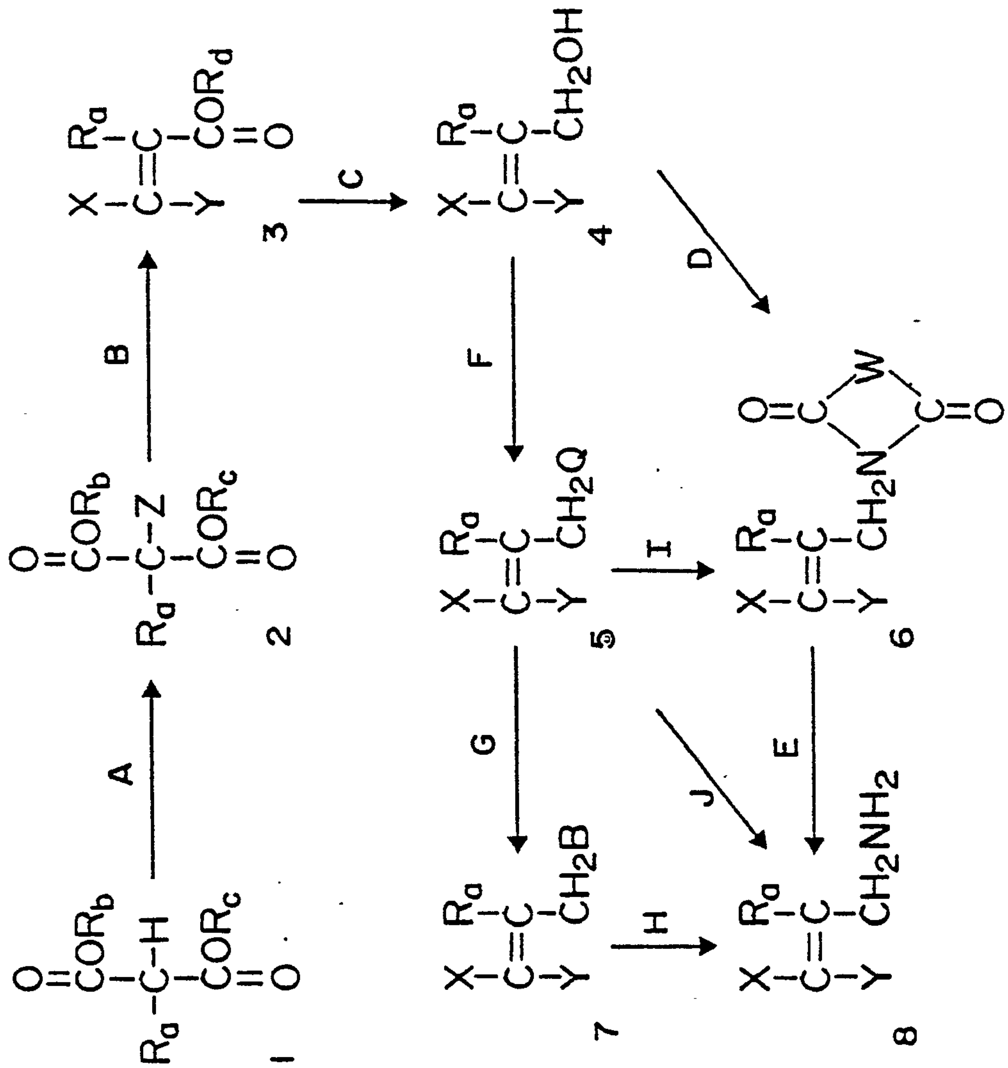


SCHEME I

Fig. I

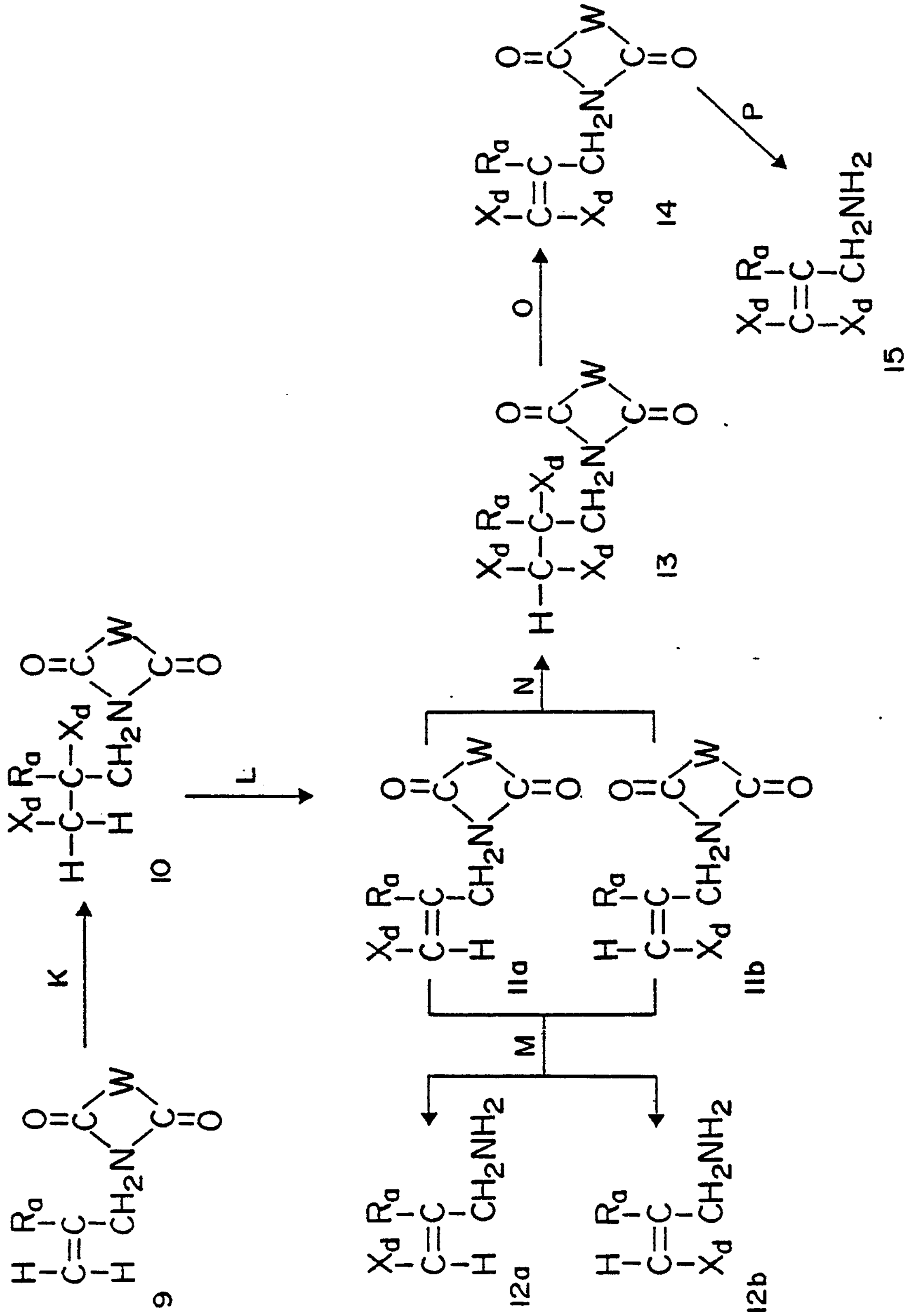


9, 10, 11a, 11b, 12a, 12b, 13, 14, 15

SCHEME II

Fig. 2